(19) Europäisches Patentamt / European Patent Office / Office européen des brevets

(11) **EP 2 198 890 B1**

(12) **EUROPEAN PATENT SPECIFICATION**

(45) Date of publication and mention of the grant of the patent:
**25.12.2013 Bulletin 2013/52**

(51) Int Cl.:
*A61K 47/48* (2006.01)  *C07K 19/00* (2006.01)
*C12Q 1/00* (2006.01)  *C12Q 1/68* (2006.01)
*A61K 38/00* (2006.01)  *C07K 1/00* (2006.01)
*C07H 21/04* (2006.01)  *G01N 33/53* (2006.01)

(21) Application number: **10155372.5**

(22) Date of filing: **06.11.2003**

(54) **Combinatorial libraries of monomer domains**

Kombinatorische Bibliotheken von Monomer-Domänen

Bibliothèques combinatoires de domaines monomères

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **06.11.2002 US 289660**

(43) Date of publication of application:
**23.06.2010 Bulletin 2010/25**

(62) Document number(s) of the earlier application(s) in accordance with Art. 76 EPC:
**03786612.6 / 1 587 843**

(73) Proprietor: **Amgen Mountain View Inc.**
**Thousand Oaks, CA 91320-1799 (US)**

(72) Inventors:
• **Kolkman, Joost A.**
  **9830 Sint Martens-Latem (BE)**
• **Stemmer, Willem P.C.**
  **Los Gatos, CA 95030 (US)**

(74) Representative: **Goodfellow, Hugh Robin**
**Carpmaels & Ransford LLP**
**One Southampton Row**
**London WC1B 5HA (GB)**

(56) References cited:
WO-A-02/088171    WO-A1-91/17271
WO-A1-99/60404

• MAMMEN M ET AL: "Polyvalent intercations in biological systems: implications for design and use of multivalent ligands and inhibitors" ANGEWANDTE CHEMIE. INTERNATIONAL EDITION, WILEY VCH VERLAG, WEINHEIM LNKD- DOI:10.1002/(SICI)1521-3773(19981102) 37:20 <2754::AID-ANIE2754>3.0.CO; 2-3, vol. 37, no. 20, 2 November 1998 (1998-11-02), pages 2754-2794, XP009113556 ISSN: 1433-7851 [retrieved on 1998-12-17]
• DALY N L ET AL: "Three-dimensional structure of a cysteine-rich repeat from the low-density lipoprotein receptor." PROCEEDINGS OF THE NATIONAL ACADEMY OF SCIENCES OF THE UNITED STATES OF AMERICA 3 JUL 1995 LNKD- PUBMED:7603991, vol. 92, no. 14, 3 July 1995 (1995-07-03), pages 6334-6338, XP002579730 ISSN: 0027-8424
• ESSER V ET AL: "Mutational Analysis of the Ligand Binding Domain of the Low Density Lipoprotein Receptor" JOURNAL OF BIOLOGICAL CHEMISTRY, AMERICAN SOCIETY FOR BIOCHEMISTRY AND MOLECULAR BIOLOGY, INC, US, vol. 263, no. 26, 15 September 1988 (1988-09-15), pages 13282-13290, XP003025627 ISSN: 0021-9258
• KRIEGER M ET AL: "STRUCTURES AND FUNCTIONS OF MULTILIGAND LIPOPROTEIN RECEPTORS: MACROPHAGE SCAVENGER RECEPTORS AND LDL RECEPTOR-RELATED PROTEIN (LRP)" ANNUAL REVIEW OF BIOCHEMISTRY, PALTO ALTO, CA, US, vol. 63, 1 January 1994 (1994-01-01), pages 601-637, XP002937333 ISSN: 0066-4154

**Description**

BACKGROUND OF THE INVENTION

Analysis of protein sequences and three-dimensional structures have revealed that many proteins are composed of a number of discrete monomer domains. The majority of discrete monomer domain proteins is extracellular or constitutes the extracellular parts of membrane-bound proteins. WO 02/088171 discloses combinatorial libraries of monomer domains.

An important characteristic of a discrete monomer domain is its ability to fold independently or with some limited assistance. Limited assistance can include assistance of a chaperonin(s) (e.g., a receptor-associated protein (RAP)).The presence of a metal ion(s) also offers limited assistance. The ability to fold independently prevents misfolding of the domain when it is inserted into a new protein environment. This characteristic has allowed discrete monomer domains to be evolutionarily mobile. As a result, discrete domains have spread during evolution and now occur in otherwise unrelated proteins. Some domains, including the fibronectin type III domains and the immunoglobin-like domain, occur in numerous proteins, while other domains are only found in a limited number of proteins.

Proteins that contain these domains are involved in a variety of processes, such as cellular transporters, cholesterol movement, signal transduction and signaling functions which are involved in development and neurotransmission. *See* Herz, Lipoprotein receptors: beacons to neutrons?, (2001) Trends in Neurosciences 24(4):193-195; Goldstein and Brown, The Cholesterol Quartet, (2001) Science 292: 1310-1312. The function of a discrete monomer domain is often specific but it also contributes to the overall activity of the protein or polypeptide. For example, the LDL-receptor class A domain (also referred to as a class A module, a complement type repeat or an A-domain) is involved in ligand binding while the gamma-carboxyglumatic acid (Gla) domain which is found in the vitamin-K-dependent blood coagulation proteins is involved in high-affinity binding to phospholipid membranes. Other discrete monomer domains include, e.g., the epidermal growth factor (EGF)-like domain in tissue-type plasminogen activator which mediates binding to liver cells and thereby regulates the clearance of this fibrinolytic enzyme from the circulation and the cytoplasmic tail of the LDL-receptor which is involved in receptor-mediated endocytosis.

Individual proteins can possess one or more discrete monomer domains. These proteins are often called mosaic proteins. For example, members of the LDL-receptor family contain four major structural domains: the cysteine rich A-domain repeats, epidermal growth factor precursor-like repeats, a transmembrane domain and a cytoplasmic domain. The LDL-receptor family includes members that: 1) are cell-surface receptors; 2) recognize extracellular ligands; and 3) internalize them for degradation by lysosomes. See Hussain et al., The Mammalian Low-Density Lipoprotein Receptor Family, (1999) Annu. Rev. Nutr. 19:141-72. For example, some members include very-low-density lipoprotein receptors (VLDL-R), apolipoprotein E receptor 2, LDLR-related protein (LRP) and megalin. Family members have the following characteristics: 1) cell-surface expression; 2) extracellular ligand binding consisting of A-domain repeats; 3) requirement of calcium for ligand binding; 4) recognition of receptor-associated protein and apolipoprotein (apo) E; 5) epidermal growth factor (EGF) precursor homology domain containing YWTD repeats (SEQ ID NO. 198); 6) single membrane-spanning region; and 7) receptor-mediated endocytosis of various ligands. *See* Hussain, *supra.* Yet, the members bind several structurally dissimilar ligands.

It is advantageous to develop methods for generating and optimizing the desired properties of these discrete monomer domains. However, the discrete monomer domains, while often being structurally conserved, are not conserved at the nucleotide or amino acid level, except for certain amino acids, e.g., the cysteine residues in the A-domain. Thus, existing nucleotide recombination methods fall short in generating and optimizing the desired properties of these discrete monomer domains.

The present invention addresses these and other problems.

BRIEF SUMMARY OF THE INVENTION

The invention provides a method for identifying a trimer that binds to a target molecule, the method comprising, providing a library of polypeptides, the polypeptides comprising LDL-receptor class A monomer domains, wherein the monomer domains consist of 30-100 amino acids comprising the following sequence:

$$C_aX_{3-14}C_bX_{3-7}C_cX_{4-16}C_dX_{1-2}C_eX_{8-23}C_f;$$

wherein C is cysteine, $X_{n-m}$ represents between n and m number of independently selected amino acids; and wherein $C_a$-$C_c$, $C_b$-$C_e$ and $C_d$-$C_f$ form disulfide bonds;
screening the library of polypeptides for affinity to a target molecule;
identifying at least one polypeptide comprising a first LDL-receptor class A monomer domain that specifically binds to a target molecule;

linking the first LDL-receptor class A monomer domain to a plurality of additional LDL-receptor class A monomer domains as defined above to form a library of LDL-receptor class A multimers, the multimers comprising the first monomer domain and one of the plurality of additional monomer domains;

screening the library of multimers for the ability to bind to the target molecule;

identifying a multimer that specifically binds to the target molecule;

linking the identified multimer to a plurality of different monomer domains to form a library of trimers, each trimer comprising the first monomer domain and the second monomer domain of the identified multimer and one of the plurality of different monomer domains;

screening the library of trimers for the ability to bind to the target molecule; and

identifying a trimer that specifically binds to the target molecule.

The present disclosure relates to methods for identifying monomers and multimers that bind to a target molecule. In some embodiments, the method comprises: providing a library of monomer domains; screening the library of monomer domains for affinity to a first target molecule; and identifying at least one monomer domain that binds to at least one target molecule. In some embodiments, the monomer domains each bind an ion

In some embodiments, the ion is selected from calcium or zinc. In some embodiments, the monomer domain is selected from the group consisting of an A domain, EGF domain, EF Hand, Cadherin domain, C-type lectin, C2 domain, Annexin, Gla-domain, Trombospondin type 3 domain and zinc finger.

In some embodiments, the method comprises providing a library of monomer domains; screening the library of monomer domains for affinity to a first target molecule; and identifying at least one monomer domain that binds to at least one target molecule. In some embodiments, the method further comprises linking the identified monomer domains to a second monomer domain to form a library of multimers, each multimer comprising at least two monomer domains; screening the library of multimers for the ability to bind to the first target molecule; and identifying a multimer that binds to the first target molecule.

Suitable monomer domains include those that are from 25 and 500 amino acids, 100 and 150 amino acids, or 25 and 50 amino acids in length.

In some embodiments, each monomer domain of the selected multimer binds to the same target molecule. In some embodiments, the selected multimer comprises at least three monomer domains. In some embodiments, the selected multimer comprises three to ten monomer domains. In some embodiments, the selected multimer comprises four monomer domains. In some embodiments, at least three monomer domains bind to the same target molecule.

In some embodiments, the monomer domain is an LDL receptor class A domain monomer comprising the following sequence:

$$C_aX_{3-15}C_bX_{3-15}C_cX_{6-7}C_d(D,N)X_4C_eX_{4-6}DEX_{2-8}C_f$$

wherein C is cysteine, $X_{n-m}$ represents between n and m number of independently selected amino acids, and (D,N) indicates that the position can be either D or N; and wherein $C_a$-$C_c$, $C_b$-$C_c$ and $C_d$-$C_f$ form disulfide bonds.

In some embodiments, the monomer domain is an LDL receptor class A domain monomer comprising the following sequence:

$$CaX_{6-7}C_bX_{4-5}C_cX_6C_dX_5C_eX_{8-10}C_f$$

wherein X is defined as follows:

In some embodiments, the LDL receptor class A domain monomers each comprise SEQ ID NO:201.

In some embodiments, the monomer domain is an EGF domain monomer comprising the following sequence:

$$C_aX_{3-14}C_bX_{3-7}C_cX_{4-16}C_dX_{1-2}C_eX_{8-23}C_f$$

wherein C is cysteine, $X_{n-m}$ represents between n and m number of independently selected amino acids; and wherein $C_a$-$C_c$, $C_b$-$C_c$ and $C_d$-$C_f$ form disulfide bonds.

In some embodiments, the monomer domain is an EGF domain monomer comprising the following sequence:

$$C_aX_{4-6}C_bX_{3-5}C_cX_{8-9}C_dX_1C_eX_{8-12}C_f$$

wherein X is defined as follows:

In some embodiments, the method further comprises screening the library of monomer domains for affinity to a second target molecule; identifying a monomer domain that binds to a second target molecule; linking at least one monomer domain with affinity for the first target molecule with at least one monomer domain with affinity for the second target molecule, thereby forming a multimer with affinity for the first and the second target molecule.

In some embodiments, the methods comprise identifying a multimer with an improved avidity for the target compared to the avidity of a monomer domain alone for the same target molecule. In some embodiments, the avidity of the multimer is at least two times the avidity of a monomer domain alone.

In some embodiments, the screening of the library of monomer domains and the identifying of monomer domains occurs simultaneously. In some embodiments, the screening of the library of multimers and the identifying of multimers occurs simultaneously.

In some embodiments, the polypeptide domain is selected from the group consisting of an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an Immunoglobulin-like domain, a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain; a Laminin-type EGF-like domain, and a C2 domain

In some embodiments, the methods comprise a further step of mutating at least one monomer domain, thereby providing a library comprising mutated monomer domains. In some embodiments, the mutating step comprises recombining a plurality of polynucleotide fragments of at least one polynucleotide encoding a monomer domain. In some embodiments, the mutating step comprises directed evolution. In some embodiments the mutating step comprises combining different loop sequences. In some embodiments, the mutating step comprises site-directed mutagenesis. In some embodiments , the mutating step comprises site-directed recombination to create crossovers that result in the generation of sequences that are identical to human sequences.

In some embodiments, the methods further comprise: screening the library of monomer domains for affinity to a second target molecule; identifying a monomer domain that binds to a second target molecule; linking at least one monomer domain with affinity for the first target molecule with at least one monomer domain with affinity for the second target molecule, thereby forming a library of multimers; screening the library of multimers for the ability to bind to the first and second target molecule; and identifying a multimer that binds to the first and second target molecule, thereby identifying a multimer that specifically binds a first and a second target molecule.

The present disclosure also provides methods of producing a polypeptide comprising the monomer domains and multimers identified according to the methods described above. In some embodiments, the monomer domains or multimers are produced by recombinant gene expression.

Certain methods of the present disclosure further comprise: providing a second library of monomer domains; screening the second library of monomer domains for affinity to at least a second target molecule; identifying a second monomer domain that binds to the second target molecule; linking the identified monomer domains that bind to the first target molecule or the second target molecule, thereby forming a library of multimers; screening the library of multimers for the ability to bind to the first and second target molecule; and identifying a multimer that binds to the first and second target molecules.

In some embodiments, the target molecule is selected from the group consisting of a viral antigen, a bacterial antigen, a fungal antigen, an enzyme, a cell surface protein, an enzyme inhibitor, a reporter molecule, a serum protein, and a receptor. In some embodiments, the viral antigen is a polypeptide required for viral replication. In some embodiments, the first and at least second target molecules are different components of the same viral replication system. In some embodiments, the selected multimer binds to at least two serotypes of the same virus.

In some embodiments, the library of monemr domains is expressed as a phage display, ribosome display, polysome display, or cell surface display. In some embodiments, the library of monomer domains is presented on a microarray.

In some embodiments, the monomer domains are linked by a polypeptide linker. In some embodiments, the polypeptide linker is a linker naturally-associated with the monomer domain. In some embodiments, the polypeptide linker is a variant of a linker naturally-associated with the monomer domain. In some embodiments, the linking step comprises linking the monomer domains with a variety of linkers of different lengths and composition.

In some embodiments, the domains form a secondary structure by the formation of disulfide bonds. In some embodiments, the multimers comprise an A domain connected to a monomer domain by a polypeptide linker. In some embodiments, the linker is from 1-20 amino acids inclusive. In some embodiments, the linker is made up of 5-7 amino acids. In some embodiments, the linker is 6 amino acids in length. In some embodiments, the linker comprises the following sequence, $A_1A_2A_3A_4A_5A_6$ (SEQ ID NO. 244), wherein $A_1$ is selected from the amino acids A, P, T, Q, E and K; $A_2$ and $A_3$ are any amino acid except C, F, Y, W, or M; $A_4$ is selected from the amino acids S, G and R; As is selected from the amino acids H, P, and R; $A_6$ is the amino acid, T. In some embodiments, the linker comprises a naturally-occurring sequence between the C-terminal cysteine of a first A domain and the N-terminal cysteine of a second A domain.

In some embodiments, the multimers comprise a C2 domain connected to a monomer domain by a polypeptide linker. In some embodiments, each C2 monomer domain differs from the corresponding wild-type C2 monomer domain in that at least one amino acid residue constituting part of the loop regions has been substituted with another amino acid residue; at least one amino acid residue constituting part of the loop regions has been deleted and/or at least one amino acid residue has been inserted in at least one of the loop regions. In some embodiments, the C2 domain comprises loop regions 1, 2, and 3 and the amino acid sequences outside of the loop regions 1, 2 and 3 are identical for all C2 monomer domains present in the polypeptide multimer. In some of these embodiments, the linker is between 1-20 amino acid residues in length. In some embodiments, the linker is between 10-12 amino acid residues in length. In some embodiments, the linker is 11 amino acid residues in length.

The present disclosure also provides polypeptides comprising the multimers or monomer domains selected as described above.

The present disclosure also provides polynucleotides encoding the multimers or monomer domains selected as described above.

The present disclosure also provides libraries of multimers or monomer domains formed as described above.

The present disclosure also provides methods for identifying a multimer that binds to at least one target molecule, comprising the steps of: providing a library of multimers, wherein each multimer comprises at least two monomer domains and wherein each monomer domain exhibits a binding specificity for a target molecule; and screening the library of multimers for target molecule-binding multimers. In some embodiments, the methods further comprise identifying target molecule-binding multimers having an avidity for the target molecule that is greater than the avidity of a single monomer domain for the target molecule. In some embodiments, one or more of the multimers comprises a monomer domain that specifically binds to a second target molecule.

Alternative methods for identifying a multimer that binds to a target molecule include methods comprising providing a library of monomer domains and/or immuno domains; screening the library of monomer domains and/or immuno domain for affinity to a first target molecule; identifying at least one monomer domain and/or immuno domain that binds to at least one target molecule; linking the identified monomer domain and/or immuno domain to a library of monomer domains and/or immuno domains to form a library of multimers, each multimer comprising at least two monomer domains, immuno domains or combinations thereof; screening the library of multimers for the ability to bind to the first target molecule; and identifying a multimer that binds to the first target molecule.

In some embodiments, the monomer domains each bind an ion. In some embodiments, the ion is selected from the group consisting of calcium and zinc. In some embodiments, the monomer domains are selected from the group consisting of an A domain, EGF domain, EF Hand, Cadherin domain, C-type lectin, C2 domain, Annexin, Gla-domain, Trombospondin type 3 domain and zinc finger.

The present disclosure also provides libraries of multimers. In some embodiments, each multimer comprises at least two monomer domains connected by a linker; each monomer domain exhibits a binding specificity for a target molecule; and each monomer domain is a non-naturally occurring monomer domain. In some embodiments, each multimer comprises at least two monomer domains connected by a linker; and each monomer domain binds an ion.

In some embodiments, the ion is selected from calcium and zinc. In some embodiments, the polypeptide domains are selected from the group consisting of consisting of an A domain, EGF domain, EF Hand, Cadherin domain, C-type lectin, C2 domain, Annexin, Gla-domain, Trombospondin type 3 domain and zinc finger.

In some embodiments, the linker comprises at least 3 amino acid residues. In some embodiments, the linker comprises at least 6 amino acid residues. In some embodiments, the linker comprises at least 10 amino acid residues.

The present disclosure also provides polypeptides comprising at least two monomer domains separated by a heterologous linker sequence. In some embodiments, each monomer domain specifically binds to a target molecule; and each monomer domain is a non-naturally occurring protein monomer domain. In some embodiments, each monomer domain binds an ion.

In some embodiments, the ion is selected from calcium and zinc. In some embodiments, the monomer domain is selected from the group consisting of an A domain, EGF domain, EF Hand, Cadherin domain, C-type lectin, C2 domain, Annexin, Gla-domain, Trombospondin type 3 domain and zinc finger.

In some embodiments, polypeptides comprise a first monomer domain that binds a first target molecule and a second monomer domain that binds a second target molecule. In some embodiments, the polypeptides comprise two monomer domains, each monomer domain having a binding specificity that is specific for a different site on the same target molecule. In some embodiments, the polypeptides further comprise a monomer domain having a binding specificity for a second target molecule.

In some embodiments, the monomer domain is an LDL receptor class A domain monomer comprising the following sequence:

$$C_aX_{3-15}C_bX_{3-15}C_cX_{6-7}C_d(D,N)X_4C_cX_{4-6}DEX_{2-8}C_f$$

wherein C is cysteine, $X_{n-m}$ represents between n and m number of independently selected amino acids, and (D,N) indicates that the position can be either D or N; and wherein $C_a$-$C_c$, $C_b$-$C_e$ and $C_d$-$C_f$ form disulfide bonds.

In some embodiments, the monomer domain is an LDL receptor class A domain monomer comprising the following sequence:

$$CaX_{6-7}C_bX_{4-5}C_cX_6C_dX_5C_cX_{8-10}C_f$$

wherein X is defined as follows:

In some embodiments, the LDL receptor class A domain monomers each comprise SEQ ID NO:201.

In some embodiments, the monomer domain is an EGF domain monomer comprising the following sequence:

$$CaX_{3-14}C_bX_{3-7}C_cX_{4-16}C_dX_{1-2}C_eX_{8-23}C_f$$

wherein C is cysteine, $X_{n-m}$ represents between n and m number of independently selected amino acids; and wherein $C_a$-$C_c$, $C_b$-$C_e$ and $C_d$-$C_f$ form disulfide bonds.

In some embodiments, the monomer domain is an EGF domain monomer comprising the following sequence:

$$C_aX_{4-6}C_bX_{3-5}C_cX_{8-9}C_dX_1 C_cX_{8-12}C_f$$

wherein X is defined as follows:

In some embodiments, the monomer domains of a library, multimer or polypeptide are typically about 40% identical to each other, usually about 50% identical, sometimes about 60% identical, and frequently at least 70% identical.

The disclosure also provides polynucleotides encoding the above-described polypeptides.

The present disclosure also provides multimers of immuno-domains having binding specificity for a target molecule, as well as methods for generating and screening libraries of such multimers for binding to a desired target molecule. More specifically, the present disclosure provides a method for identifying a multimer that binds to a target molecule, the method comprising, providing a library of immuno-domains; screening the library of immuno-domains for affinity to a first target molecule; identifying one or more (e.g., two or more) immuno-domains that bind to at least one target molecule; linking the identified monomer domain to form a library of multimers, each multimer comprising at least three immuno-domains (e.g., four or more, five or more, six or more, etc.); screening the library of multimers for the ability to bind to the first target molecule; and identifying a multimer that binds to the first target molecule. Libraries of multimers of at least two immuno-domains that are minibodies, single domain antibodies, Fabs, or combinations thereof are also employed in the practice of the present disclosure. Such libraries can be readily screened for multimers that bind to desired target molecules in accordance with the methods described herein.

The present disclosure further provides methods of identifying hetero-immuno multimers that binds to a target molecule. In some embodiments, the methods comprise, providing a library of immuno-domains; screening the library of immuno-domains for affinity to a first target molecule; providing a library of monomer domains; screening the library of monomer domains for affinity to a first target molecule; identifying at least one immuno-domain that binds to at least one target molecule; identifying at least one monomer domain that binds to at least one target molecule; linking the identified immuno-domain with the identified monomer domains to form a library ofmultimers, each multimer comprising at least two domains; screening the library of multimers for the ability to bind to the first target molecule; and identifying a multimer that binds to the first target molecule.

The present disclosure also provides methods for identifying an LDL-receptor class A monomer domain that binds to a target molecule. In some embodiments, the method comprises providing a library of LDL-receptor class A monomer domains; screening the library of LDL-receptor class A monomer domains for affinity to a target molecule; and identifying an LDL-receptor class A monomer domain that binds to the target molecule.

In some embodiments, the method comprises linking each member of a library of LDL-receptor class A monomers to the identified monomer domain to form a library of multimers; screening the library ofmultimers for affinity to the target molecule; and identifying a multimer that binds to the target. In some embodiments, the multimer binds to the target with greater affinity than the monomer. In some embodiments, the method further comprises expressing the library using a display format selected from the group consisting of a phage display, a ribosome display, a polysome display, or a cell surface display.

In some embodiments, the method further comprises a step of mutating at least one monomer domain, thereby providing a library comprising mutated LDL-receptor class A monomer domains. In some embodiments, the mutating step comprises directed evolution. In some embodiments, the mutating step comprises site-directed mutagenesis.

The present disclosure also provides method of producing a polypeptide comprising the multimer identified in a method comprising providing a library of LDL-receptor class A monomer domains; screening the library of LDL-receptor class A monomer domains for affinity to a target molecule; and identifying an LDL-receptor class A monomer domain that binds to the target molecule. In some embodiments, the multimer is produced by recombinant gene expression.

The present disclosure also provides methods for generating a library of chimeric LDL receptor A-domain monomers derived from human LDL receptor A-domains. In some embodiments, the methods comprise providing loop sequences corresponding to at least one loop from each of two different naturally occurring variants of a human LDL receptor A-domains, wherein the loop sequences are polynucleotide or polypeptide sequences; covalently combining loop sequences to generate a library of chimeric monomer domain sequences, each chimeric sequence encoding a chimeric LDL receptor A-domain monomer having at least two loops; expressing the library of chimeric LDL receptor A-domain monomers using a display format selected from the group consisting of phage display, ribosome display, polysome display, and cell surface display; screening the expressed library of chimeric LDL receptor A-domain monomers for binding to a target molecule; and identifying a chimeric LDL receptor A-domain monomer that binds to the target molecule.

In some embodiments, the methods further comprise linking the identified chimeric LDL receptor A-domain monomer domain to each member of the library of chimeric LDL receptor A-domain monomers to form a library of multimers; screening the library of multimers for the ability to bind to the first target molecule with an increased affinity; and identifying a multimer of chimeric LDL receptor A-domain monomers that binds to the first target molecule with an increased affinity.

The present disclosure also provides methods of making chimeric LDL receptor A-domain monomer identified in a method comprising providing loop sequences corresponding to at least one loop from each of two different naturally occurring variants of a human LDL receptor A-domains, wherein the loop sequences are polynucleotide or polypeptide sequences; covalently combining loop sequences to generate a library of chimeric monomer domain sequences, each chimeric sequence encoding a chimeric LDL receptor A-domain monomer having at least two loops; expressing the library of chimeric LDL receptor A-domain monomers using a display format selected from the group consisting of phage display, ribosome display, polysome display, and cell surface display; screening the expressed library of chimeric LDL receptor A-domain monomers for binding to a target molecule; and identifying a chimeric LDL receptor A-domain monomer that binds to the target molecule. In some embodiments, the chimeric LDL receptor A-domain monomer is produced by recombinant gene expression.

The present disclosure also provides non-naturally-occurring polypeptides comprising an LDL receptor class A domain monomer, wherein the monomer comprises the following sequence:

$$C_aX_{3\text{-}15}C_bX_{3\text{-}15}C_cX_{6\text{-}7}C_d(D,N)C_4C_eX_{4\text{-}6}DEX_{2\text{-}8}C_f$$

wherein C is cysteine, $X_{n\text{-}m}$ represents between n and m number of independently selected amino acids, and (D,N) indicates that the position can be either D or N; and wherein $C_a\text{-}C_c$, $C_b\text{-}C_e$ and $C_d\text{-}C_f$ form disulfide bonds.

In some embodiments, the monomer domain is an LDL receptor class A domain monomer comprising the following sequence:

$$CaX_{6\text{-}7}C_bX_{4\text{-}5}C_cX_6C_dX_5C_eX_{8\text{-}10}C_f$$

wherein X is defined as follows:

In some embodiments, the polypeptide is 65 or fewer amino acids long. In some embodiments, the monomer is fused to a heterologous amino acid sequence. In some embodiments, the monomer binds to a target molecule. In some embodiments, the heterologous amino acid sequence is selected from an affinity peptide, a heterologous LDL receptor class A domain, a heterologous EGF domain, and a purification tag

The present disclosure also provides non-naturally-occurring polypeptides comprising an EGF domain monomer, wherein the EGF domain monomer comprises the following sequence:

$$C_a X_{3-14} C_b X_{3-7} C_c X_{4-16} C_d X_{1-2} C_e X_{8-23} C_f$$

wherein C is cysteine, $X_{n-m}$ represents between n and m number of independently selected amino acids; and wherein $C_a$-$C_c$, $C_b$-$C_c$ and $C_d$-$C_f$ form disulfide bonds.

In some embodiments, the monomer domain is an EGF domain monomer comprising the following sequence:

$$C_aX_{4-6}C_bX_{3-5}C_cX_{8-9}C_dX_1C_eX_{8-12}C_f$$

wherein X is defined as follows:

In some embodiments, the EGF domain monomer is fused to a heterologous amino acid sequence. In some embodiments, the monomer binds to a target molecule. In some embodiments, the polypeptide is 45 or fewer amino acids long. In some embodiments, the heterologous amino acid sequence is selected from an affinity peptide (e.g., SKVILF), a heterologous LDL receptor class A domain, a heterologous EGF domain, a purification tag, an enzyme (e.g., horseradish peroxidase or alkaline phosphatase), and a reporter protein (e.g., green fluorescent protein or luciferase).

The present disclosure provides methods for screening a library of monomer domains or multimers comprising monomer domains for binding affinity to multiple ligands. In some embodiments, the method comprises contacting a library of monomer domains or multimers of monomer domains to multiple ligands; and selecting monomer domains or multimers that bind to at least one of the ligands.

In some embodiments, the methods comprise (i.) contacting a library of monomer domains to multiple ligands; (ii.) selecting monomer domains that bind to at least one of the ligands; (iii.) linking the selected monomer domains to a library of monomer domains to form a library of multimers, each comprising a selected monomer domain and a second monomer domain; (iv.) contacting the library of multimers to the multiple ligands to form a plurality of complexes, each complex comprising a multimer and a ligand; and (v.) selecting at least one complex.

In some embodiments, the method further comprises linking the multimers of the selected complexes to a library of monomer domains or multimers to form a second library of multimers, each comprising a selected multimer and at least a third monomer domain; contacting the second library of multimers to the multiple ligands to form a plurality of second complexes; and selecting at least one second complex.

In some embodiments, the identity of the ligand and the multimer is determined. In some embodiments, a library of monomer domains is contacted to multiple ligands. In some embodiments, a library of multimers is contacted to multiple ligands.

In some embodiments, the multiple ligands are in a mixture. In some embodiments, the multiple ligands are in an array. In some embodiments, the multiple ligands are in or on a cell or tissue. In some embodiments, the multiple ligands are immobilized on a solid support.

In some embodiments, the ligands are polypeptides. In some embodiments, the polypeptides are expressed on the surface of phage. In some embodiments, the monomer domain or multimer library is expressed on the surface of phage.

In some embodiments, the monomer domain is a LDL receptor type A monomer domain. In some embodiments, the monomer domain is an EGF monomer domain.

In some embodiments, the library of multimers is expressed on the surface of phage to form library-expressing phage and the ligands are expressed on the surface of phage to form ligand-expressing phage, and the method comprises contacting library-expressing phage to the ligand-expressing phage to form ligand-expressing phage/library-expressing phage pairs; removing ligand-expressing phage that do not bind to library-expressing or removing library-expressing phage that do not bind to ligand-expressing phage; and selecting the ligand-expressing phage/library-expressing phage pairs. In some embodiments, the methods further comprise isolating polynucleotides from the phage pairs and amplifying the polynucleotides to produce a polynucleotide hybrid comprising polynucleotides from the ligand-expressing phage and the library-expressing phage.

In some embodiments, the methods comprise isolating polynucleotide hybrids from a plurality of phage pairs, thereby forming a mixture of polynucleotide hybrids. In some embodiments, the methods comprise contacting the mixture of hybrid polynucleotides to a cDNA library under conditions to allow for polynucleotide hybridization, thereby hybridizing a hybrid polynucleotide to a cDNA in the cDNA library; and determining the nucleotide sequence of the hybridized hybrid polynucleotide, thereby identifying a monomer domain that specifically binds to the polypeptide encoded by the cDNA. In some embodiments, the monomer domain library is expressed on the surface of phage to form library-expressing phage and the ligands are expressed on the surface of phage to form ligand-expressing phage, and the selected complexes comprise a library-expressing phage bound to a ligand-expressing phage and the method comprises: dividing the selected monomer domains or multimers into a first and a second portion, linking the monomer domains or multimers of the first portion to a solid surface and contacting a phage-displayed ligand library to the monomer domains or multimers of the first portion to identify target ligand phage that binds to a monomer domain or multimer of the first portion; infecting phage displaying the monomer domains or multimers of the second portion into bacteria to express the phage; and contacting the target ligand phage to the expressed phage to form phage pairs comprised of a target ligand phage and a phage displaying a monomer domain or multimer.

In some embodiments, the methods further comprise isolating a polynucleotide from each phage of the phage pair, thereby identifying a multimer or monomer domain that binds to the ligand in the phage pair. In some embodiments, the methods further comprise amplifying the polynucleotides to produce a polynucleotide hybrid comprising polynucleotides from the target ligand phage and the library phage.

In some embodiments, the methods comprise isolating and amplifying polynucleotide hybrids from a plurality of phage pairs, thereby forming a mixture of polynucleotide hybrids. In some embodiments, the methods comprise contacting the mixture of hybrid polynucleotides to a cDNA library under conditions to allow for hybridization, thereby hybridizing a hybrid polynucleotide to a cDNA in the cDNA library; and determining the nucleotide sequence of the associated hybrid polynucleotide, thereby identifying a monomer domain that specifically binds to the ligand encoded by the cDNA associated cDNA.

The present disclosure also provides methods of increasing the serum half-life of a molecule in blood. In some embodiments, the methods comprise administering to a subject the molecule bound to a multimer, wherein the multimer comprises a first monomer domain that specifically binds to a blood component, and a second monomer domain that specifically binds to the molecule. In some embodiments, the blood component is selected from the group consisting of human serum albumin, a red blood cell and IgG. In some embodiments, the first and second domains are LDL receptor class A domains.

DEFINITIONS

Unless otherwise indicated, the following definitions supplant those in the art.

The term "monomer domain" or "monomer" is used interchangeably herein refer to a discrete region found in a protein or polypeptide. A monomer domain forms a native three-dimensional structure in solution in the absence of flanking native amino acid sequences. Monomer domains of the invention will specifically bind to a target molecule. For example, a polypeptide that forms a three-dimensional structure that binds to a target molecule is a monomer domain. As used herein, the term "monomer domain" does not encompass the complementarity determining region (CDR) of an antibody.

The term "monomer domain variant" refers to a domain resulting from human-manipulation of a monomer domain sequence. Examples of man-manipulated changes include, e.g., random mutagenesis, site-specific mutagenesis, shuffling, directed evolution, oligo-directed forced crossover events, direct gene synthesis incorporation of mutation, etc. The term "monomer domain variant" does not embrace a mutagenized complementarity determining region (CDR) of an antibody.

The term "loop" refers to that portion of a monomer domain that is typically exposed to the environment by the assembly of the scaffold structure of the monomer domain protein, and which is involved in target binding. The present disclosure provides three types of loops that are identified by specific features, such as, potential for disulfide bonding, bridging between secondary protein structures, and molecular dynamics (i.e., flexibility). The three types of loop sequences are a cysteine-defined loop sequence, a structure-defined loop sequence, and a B-factor-defined loop sequence.

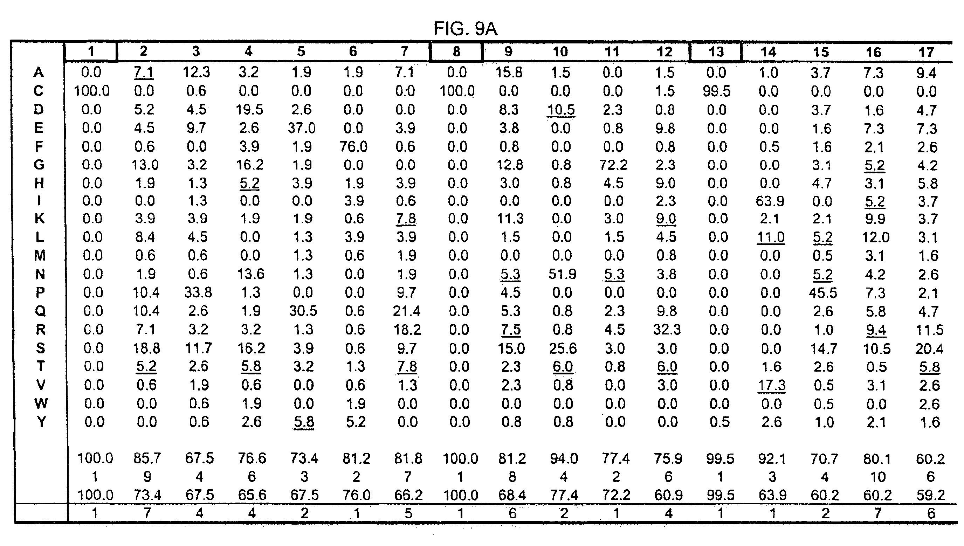

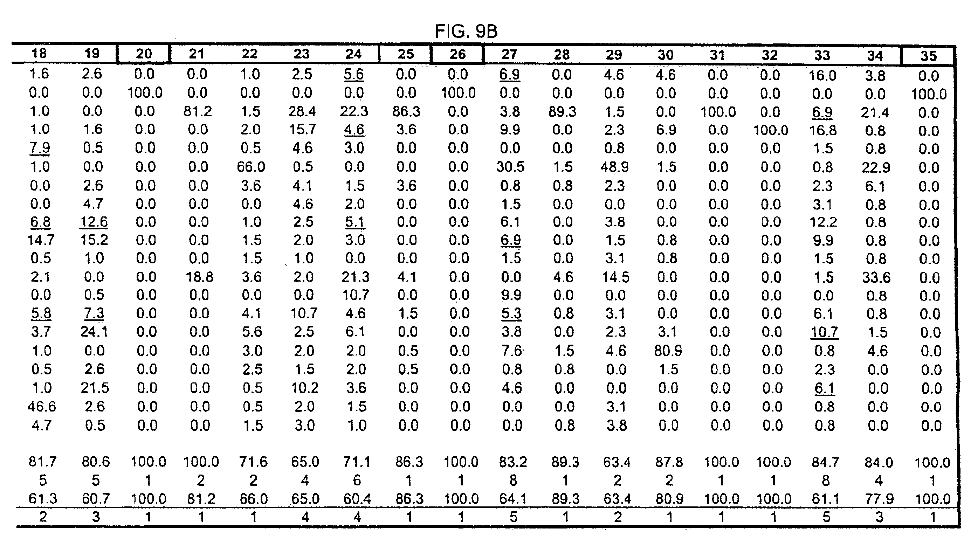

As used herein, the term "cysteine-defined loop sequence" refers to a subsequence of a naturally occurring monomer domain-encoding sequence that is bound at each end by a cysteine residue that is conserved with respect to at least one other naturally occurring monomer domain of the same family. Cysteine-defined loop sequences are identified by multiple sequence alignment of the naturally occurring monomer domains, followed by sequence analysis to identify conserved cysteine residues. The sequence between each consecutive pair of conserved cysteine residues is a cysteine-defined loop sequence. The cysteine-defined loop sequence does not include the cysteine residues adjacent to each terminus. Monomer domains having cysteine-defined loop sequences include the LDL receptor A-domains, EGF-like domains, sushi domains, Fibronectin type 1 domains, and the like. Thus, for example, in the case of LDL receptor A-domains represented by the consensus sequence, $CX_6CX_4CX_6CX_5CX_8C$ (see also Figure 9), wherein $X_6$, $X_4$, $X_5$, and $X_8$ each represent a cysteine-defined loop sequence.

As used herein, the term "structure-defined loop sequence" refers to a subsequence of a monomer-domain encoding sequence that is bound at each end to subsequences that each form a secondary structure. Secondary structures for proteins with known three dimensional structures are identified in accordance with the algorithm STRIDE for assigning protein secondary structure as described in Frishman, D. and Argos, P. (1995) "Knowledge-based secondary structure assignment," Proteins, 23(4):566-79 (see also //hgmp-mrc.ac.uk/Registered/Option/stride.html at the World Wide Web). Secondary structures for proteins with unknown or uncharacterized three dimensional structures are identified in accordance with the algorithm described in Jones, D.T. (1999), "Protein secondary structure prediction based on position-specific scoring matrices," J. Mol. Biol., 292:195-202 (see also McGuffin, L.J., Bryson, K., Jones, D.T. (2000) "The PSIPRED protein structure prediction server," Bioinformatics, 16:404-405, and //bioinf.cs.ucl.ac.uk/psipred/ at the World Wide Web). Secondary structures include, for example, pleated sheets, helices, and the like. Examples of monomer domains having structure-defined loop sequences are the C2 domains, Ig domains, Factor 5/8 C domains, Fibronectin type 3 domains, and the like.

The term "B-factor-defined loop sequence" refers to a subsequence of at least three amino acid residues of a monomer-domain encoding sequence in which the B-factors for the alpha carbons in the B-factor-defined loop are among the 25% highest alpha carbon B factors in the entire monomer domain. Typically the average alpha-carbon B-factor for the subsequence is at least about 65. As used herein, the term "B-factor" (or "temperature factor" or "Debye-Waller factor") is derived from X-ray scattering data. The B-factor is a factor that can be applied to the X-ray scattering term for each atom, or for groups of atoms, that describes the degree to which electron density is spread out B-factors employed in the practice of the present invention may be either isotropic or anisotropic. The term "average alpha-carbon B-factor" refers to:

$$(\sum_{i=1}^{n} \text{B-factor}_{C\alpha i}) / n$$

where n corresponds to the number of residues in the loop, and is at least 3, and B-factor$_{C\alpha i}$ is the B-factor for the alpha carbon of amino acid residue i of the loop.

The term "multimer" is used herein to indicate a polypeptide comprising at least two monomer domains and/or immuno-domains (e.g., at least two monomer domains, at least two immuno-domains, or at least one monomer domain and at least one immuno-domain). The separate monomer domains and/or immuno-domains in a multimer can be joined together by a linker. A multimer is also known as a combinatorial mosaic protein or a recombinant mosaic protein.

The term "family" and "family class" are used interchangably to indicate proteins that are grouped together based on similarities in their amino acid sequences. These similar sequences are generally conserved because they are important for the function of the protein and/or the maintenance of the three dimensional structure of the protein. Examples of such families include the LDL Receptor A-domain family, the EGF-like family, and the like.

The term "ligand," also referred to herein as a "target molecule," encompasses a wide variety of substances and molecules, which range from simple molecules to complex targets. Target molecules can be proteins, nucleic acids, lipids, carbohydrates or any other molecule capable of recognition by a polypeptide domain. For example, a target molecule can include a chemical compound (i.e., non-biological compound such as, e.g., an organic molecule, an inorganic molecule, or a molecule having both organic and inorganic atoms, but excluding polynucleotides and proteins), a mixture of chemical compounds, an array of spatially localized compounds, a biological macromolecule, a bacteriophage peptide display library, a polysome peptide display library, an extract made from a biological materials such as bacteria, plants, fungi, or animal (e.g., mammalian) cells or tissue, a protein, a toxin, a peptide hormone, a cell, a virus, or the like. Other target molecules include, e.g., a whole cell, a whole tissue, a mixture of related or unrelated proteins, a mixture of viruses or bacterial strains or the like. Target molecules can also be defined by inclusion in screening assays described herein or by enhancing or inhibiting a specific protein interaction (i.e., an agent that selectively inhibits a binding interaction between two predetermined polypeptides).

As used herein, the term "immuno-domains" refers to protein binding domains that contain at least one complementarity determining region (CDR) of an antibody. Immuno-domains can be naturally occurring immunological domains (i.e. isolated from nature) or can be non-naturally occurring immunological domains that have been altered by human-manipulation (e.g., via mutagenesis methods, such as, for example, random mutagenesis, site-specific mutagenesis, recombinantion, and the like, as well as by directed evolution methods, such as, for example, recursive error-prone PCR, recursive recombination, and the like.). Different types of immuno-domains that are suitable for use in the practice of the present invention include a minibody, a single-domain antibody, a single chain variable fragment (ScFv), and a Fab fragment.

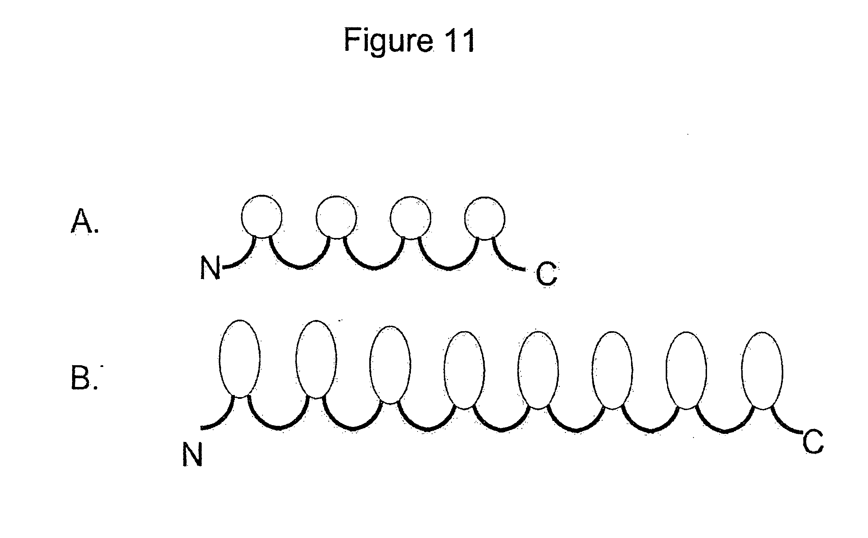

The term "minibody" refers herein to a polypeptide that encodes only 2 complementarity determining regions (CDRs) of a naturally or non-naturally (e.g., mutagenizcd) occurring heavy chain variable domain or light chain variable domain, or combination thereof. An example of a minibody is described by Pessi et al., A designed metal-binding protein with a novel fold, (1993) Nature 362:367-369. A multimer of minibodies is schematically illustrated in Figure 11A. The circles depict minibodies, and the solid lines depict the linker moieties joining the immuno-domains to each other.

As used herein, the term "single-domain antibody" refers to the heavy chain variable domain ("$V_H$") of an antibody, i.e., a heavy chain variable domain without a light chain variable domain. Exemplary single-domain antibodies employed in the practice of the present invention include, for example, the Camelid heavy chain variable domain (about 118 to 136 amino acid residues) as described in Hamers-Casterman, C. et al., Naturally occurring antibodies devoid of light chains (1993) Nature 363:446-448, and Dumoulin, et al., Single-domain antibody fragments with high conformational stability (2002) Protein Science 11:500-515. A multimer of single-domain antibodies is depicted in Figure 11B. The ellipses represent the single-domain antibodies, and the solid lines depict the linker moieties joining the single-domain antibodies to each other.

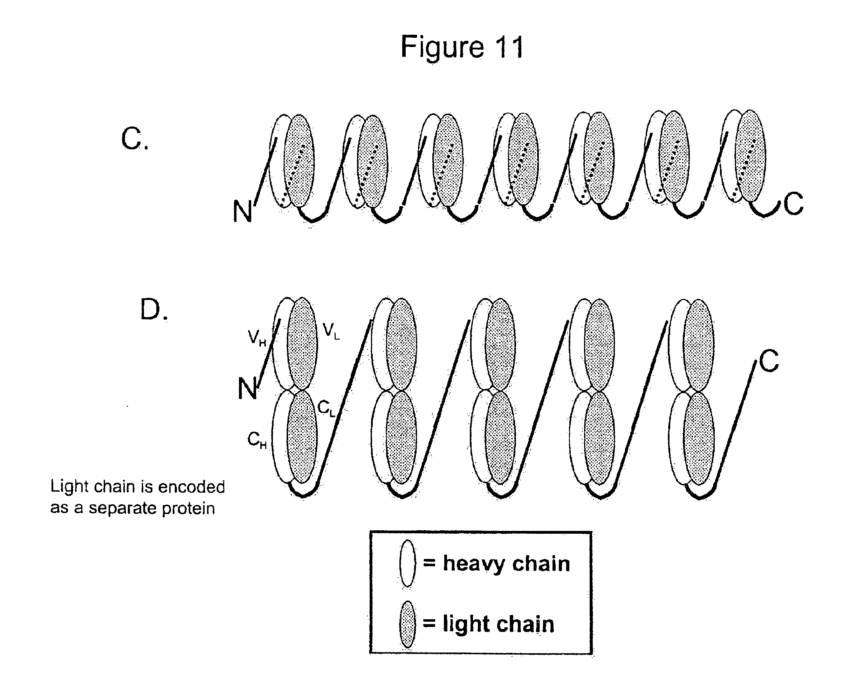

The terms "single chain variable fragment" or "ScFv" are used interchangeably herein to refer to antibody heavy and light chain variable domains that are joined by a peptide linker having at least 12 amino acid residues. Single chain variable fragments contemplated for use in the practice of the present invention include those described in Bird, et al., Single-chain antigen-bindingproteins (1988) Science 242(4877):423-426 and Huston et al., Protein engineering of antibody binding sites: recovery of specific activity in an anti-digoxin single-chain Fv analogue produced in Escherichia coli (1988) Proc Natl Acad Sci U S A 85(16):5879-83. A multimer of single chain variable fragments is illustrated in Figure 11C. The dotted lines represent the peptide linker joining the heavy and light chain variable domains to each other. The solid lines depict the linker moieties joining the heavy chain variable domains to each other.

As used herein, the term "Fab fragment" refers to an immuno-domain that has two protein chains, one of which is a light chain consisting of two light chain domains ($V_L$ variable domain and $C_L$ constant domain) and a heavy chain consisting of two heavy domains (i.e., a $V_H$ variable and a $C_H$ constant domain). Fab fragments employed in the practice of the present invention include those that have an interchain disulfide bond at the C-terminus of each heavy and light component, as well as those that do not have such a C-terminal disulfide bond. Each fragment is about 47 kD. Fab fragments are described by Pluckthun and Skerra, Expression of functional antibody Fv and Fab fragments in Escherichia col (1989) Methods Enzymol 178:497-515. A multimer ofFab fragments is depicted in Figure 11D. The white ellipses represent the heavy chain component of the Fab fragment, the filled ellipses represent the light chain component of the Fab.

The term "linker" is used herein to indicate a moiety or group of moieties that joins or connects two or more discrete separate monomer domains. The linker allows the discrete separate monomer domains to remain separate when joined together in a multimer. The linker moiety is typically a substantially linear moiety. Suitable linkers include polypeptides, polynucleic acids, peptide nucleic acids and the like. Suitable linkers also include optionally substituted alkylene moieties that have one or more oxygen atoms incorporated in the carbon backbone. Typically, the molecular weight of the linker is less than about 2000 daltons. More typically, the molecular weight of the linker is less than about 1500 daltons and usually is less than about 1000 daltons. The linker can be small enough to allow the discrete separate monomer domains to cooperate, e.g., where each of the discrete separate monomer domains in a multimer binds to the same target molecule via separate binding sites. Exemplary linkers include a polynucleotide encoding a polypeptide, or a polypeptide of amino acids or other non-naturally occurring moieties. The linker can be a portion of a native sequence, a variant thereof, or a synthetic sequence. Linkers can comprise, e.g., naturally occurring, non-naturally occurring amino acids, or a combination of both.

The term "separate" is used herein to indicate a property of a moiety that is independent and remains independent even when complexed with other moieties, including for example, other monomer domains. A monomer domain is a separate domain in a protein because it has an independent property that can be recognized and separated from the protein. For instance, the ligand binding ability of the A-domain in the LDLR is an independent property. Other examples of separate include the separate monomer domains in a multimer that remain separate independent domains even when complexed or joined together in the multimer by a linker. Another example of a separate property is the separate binding sites in a multimer for a ligand.

As used herein, "directed evolution" refers to a process by which polynucleotide variants are generated, expressed, and screened for an activity (e.g., a polypeptide with binding activity) in a recursive process. One or more candidates in the screen are selected and the process is then repeated using polynucleotides that encode the selected candidates to generate new variants. Directed evolution involves at least two rounds of variation generation and can include 3, 4, 5, 10, 20 or more rounds of variation generation and selection. Variation can be generated by any method known to those of skill in the art, including, e.g., by error-prone PCR, gene shuffling, chemical mutagenesis and the like.

The term "shuffling" is used herein to indicate recombination between non-identical sequences. In some embodiments, shuffling can include crossover via homologous recombination or via non-homologous recombination, such as via cre/lox and/or flp/frt systems. Shuffling can be carried out by employing a variety of different formats, including for example, *in vitro* and *in vivo* shuffling formats, in silico shuffling formats, shuffling formats that utilize either double-stranded or single-stranded templates, primer based shuffling formats, nucleic acid fragmentation-based shuffling formats, and oligonucleotide-mediated shuffling formats, all of which are based on recombination events between non-identical sequences and are described in more detail or referenced herein below, as well as other similar recombination-based formats. The term "random" as used herein refers to a polynucleotide sequence or an amino acid sequence composed of two or more amino acids and constructed by a stochastic or random process. The random polynucleotide sequence or amino acid sequence can include framework or scaffolding motifs, which can comprise invariant sequences.

The term "pseudorandom" as used herein refers to a set of sequences, polynucleotide or polypeptide, that have limited variability, so that the degree of residue variability at some positions is limited, but any pseudorandom position is allowed at least some degree of residue variation.

The terms "polypeptide," "peptide," and "protein" are used herein interchangeably to refer to an amino acid sequence of two or more amino acids.

'Conservative amino acid substitution" refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The phrase "nucleic acid sequence" refers to a single or double-stranded polymer of deoxyribonucleotide or ribonucleotide bases read from the 5' to the 3' end. It includes chromosomal DNA, self-replicating plasmids and DNA or RNA that performs a primarily structural role.

The term "encoding" refers to a polynucleotide sequence encoding one or more amino acids. The term does not require a start or stop codon. An amino acid sequence can be encoded in any one of six different reading frames provided by a polynucleotide sequence.

The term "promoter" refers to regions or sequence located upstream and/or downstream from the start of transcription that are involved in recognition and binding of RNA polymerase and other proteins to initiate transcription.

A "vector" refers to a polynucleotide, which when independent of the host chromosome, is capable of replication in a host organism. Examples of vectors include plasmids. Vectors typically have an origin of replication. Vectors can comprise, e.g., transcription and translation terminators, transcription and translation initiation sequences, and promoters useful for regulation of the expression of the particular nucleic acid.

The term "recombinant" when used with reference, *e.g.*, to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under-expressed or not expressed at all.

The phrase "specifically (or selectively) binds" to a polypeptide, when referring to a monomer or multimer, refers to a binding reaction that can be determinative of the presence of the polypeptide in a heterogeneous population of proteins and other biologics. Thus, under standard conditions or assays used in antibody binding assays, the specified monomer or multimer binds to a particular target molecule above background (e.g., 2X, 5X, 10X or more above background) and does not bind in a significant amount to other molecules present in the sample.

The terms "identical" or percent "identity," in the context of two or more nucleic acids or polypeptide sequences, refer to two or more sequences or subsequences that are the same. "Substantially identical" refers to two or more nucleic acids or polypeptide sequences having a specified percentage of amino acid residues or nucleotides that are the same (*i.e.*, 60% identity, optionally 65%, 70%, 75%, 80%, 85%, 90%, or 95% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity or substantial identity exists over a region that is at least about 50 nucleotides in length, or more preferably over a region that is 100 to 500 or 1000 or more nucleotides or amino acids in length.

A polynucleotide or amino acid sequence is "heterologous to" a second sequence if the two sequences are not linked in the same manner as found in naturally-occurring sequences. For example, a promoter operably linked to a heterologous coding sequence refers to a coding sequence which is different from any naturally-occurring allelic variants. The term "heterologous linker," when used in reference to a multimer, indicates  that the multimer comprises a linker and a monomer that are not found in the same relationship to each other in nature (*e.g.*, they form a fusion protein).

BRIEF DESCRIPTION OF THE DRAWINGS

Figure 1 schematically illustrates the type, number and order of monomer domains found in members of the LDL-receptor family. These monomer domains include β-Propeller domains, EGF-like domains and LDL receptor class A-domains. The members shown include low-density lipoprotein receptor (LDLR), ApoE Receptor 2 (ApoER2), very-low-density lipoprotein receptor (VLDLR), LDLR-related protein 2 (LRP2) and LDLR-related protein1 (LRP1).

Figure 2 schematically illustrates the alignment of partial amino acid sequence from a variety of the LDL-receptor class A-domains (SEQ ID NOS: 103, 100, 65, 117, 128, 21, 29, 39, 30, 77, 58, 50, and 14, respectively in order of appearance) that include two human LRP1 sequences, two human LRP2 sequences, two human LDLR sequences, two human LDVR sequences, one human LRP3 sequence, one human MAT sequence, a human CO6 sequence, and a human SORL sequence, to demonstrate the conserved cysteines.

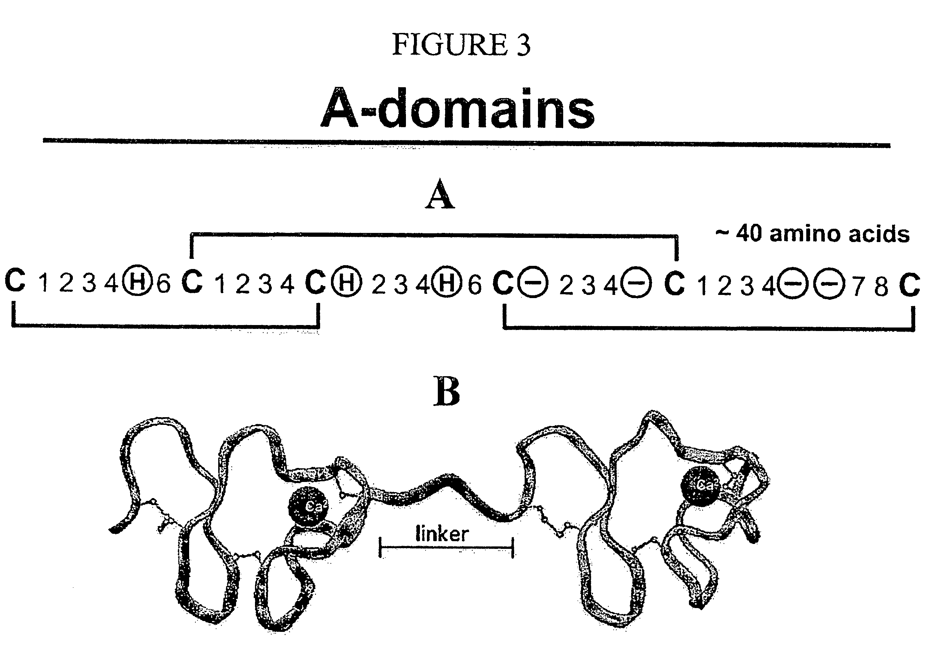

Figure 3, panel A schematically illustrates an example of an A-domain. Panel A schematically illustrates conserved amino acids in an A-domain of about 40 amino acids long. The conserved cysteine residues are indicated by C, and the negatively charged amino acids are indicated by a circle with a minus ("-") sign. Circles with an "H" indicate hydrophobic residues. Panel B schematically illustrates two folded A-domains connected via a linker. Panel B also indicates two calcium binding sites, dark circles with $Ca^{+2}$, and three disulfide bonds within each folded A-domain for a total of 6 disulfide bonds.

Figure 4 indicates some of the ligands recognized by the LDL-receptor family, which include inhibitors, proteases, protease complexes, vitamin-carrier complexes, proteins involved in lipoprotein metabolism, non-human ligands, antibiotics, viruses, and others.

Figure 5 schematically illustrates a general scheme for identifying monomer domains that bind to a ligand, isolating the selected monomer domains, creating multimers of the selected monomer domains by joining the selected monomer domains in various combinations and screening the multimers to identify multimers comprising more than one monomer that binds to a ligand.

Figure 6 is a schematic representation of another selection strategy (guided selection). A monomer domain with appropriate binding properties is identified from a library of monomer domains. The identified monomer domain is then linked to monomer domains from another library of monomer domains to form a library of multimers. The multimer library is screened to identify a pair of monomer domains that bind simultaneously to the target. This process can then be repeated until the optimal binding properties are obtained in the multimer.

Figure 7 shows the multimerization process of monomer domains. The target-binding monomer hits are amplified from a vector. This mixture of target-binding monomer domains and/or immuno-domains is then cleaved and mixed with an optimal combination of linker and stopper oligonucleotides. The multimers that are generated are then cloned into a suitable vector for the second selection step for identification of target-binding multimers.

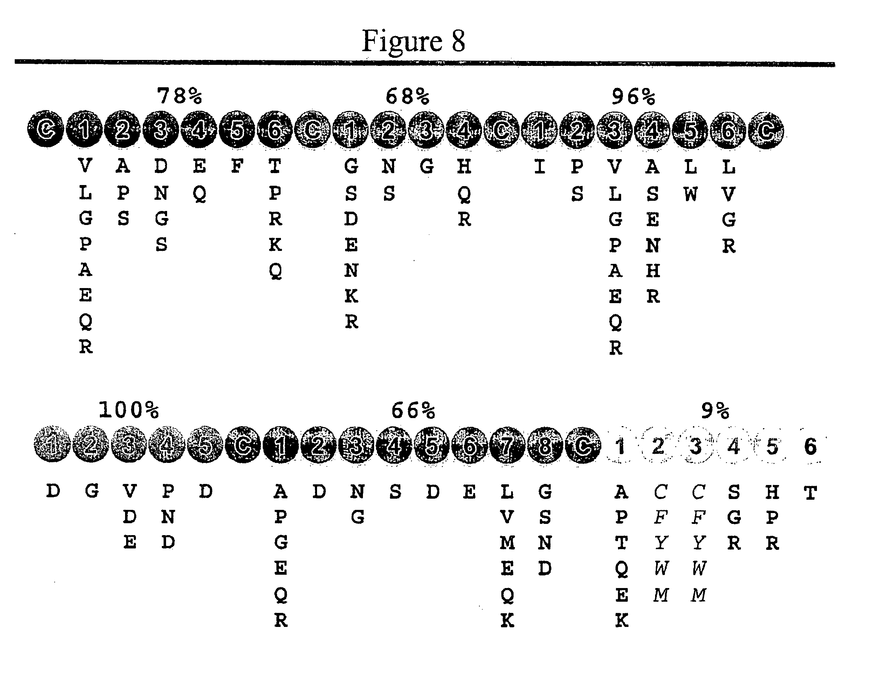

Figure 8 depicts common amino acids in each position of the A domain. The percentages above the amino acid positions refer to the percentage of naturally-occurring A domains with the inter-cysteine spacing displayed. Potential amino acid residues in bold depicted under each amino acid position represent common residues at that position. The final six amino acids, depicted as lighter-colored circles, represent linker sequences. The two columns of italicized amino acid residues at positions 2 and 3 of the linker represent amino acid residues that do not occur at that position. Any other amino acid (e.g., A, D, E, G, H, I, K, L, N, P, Q, R, S, T, and V) may be included at these positions.

Figures 9A and 9B display the frequency of occurrence of amino acid residues in naturally-occurring A domains for A domains with the following spacing between cysteines: $CX_6CX_4CX_6CX_5CX_8C$ (SEQ ID NO: 199).

Figure 10 depicts an alignment of A domains (SEQ ID NO: 1-197). At the top and the bottom of the figure, small letters (a-q) indicate conserved residues. The predominant amino acids at these positions and the frequency with which they were observed in native A domains is illustrated at the bottom of the figure.

Figure 11 depicts possible multimer conformations comprised of immuno-domains. Figure 11A illustrates a multimer of minibodies. Figure 11B illustrates a multimer of single-domain antibodies. Figure 11C illustrates an immuno-domain multimer of scfvs. Figure 11D illustrates a multimer of Fab fragments.

Figure 12 depicts linkage of domains via partial linkers.

Figures 13A, B and C illustrate exemplary multimer ring formations.

Figure 14 illustrates various multimer conformations of heavy and light chains of Fvs.

Figure 15 is a graphical representation of the regions of sequence identity between the sequences of two different selected clones and known human sequences from a database. The horizontal bars indicate areas of sequence identity between the sequence of the selected clone and the human sequence and the numbers indicate the exact amino acid numbers that define the region of identity. The vertical arrow depicts an acceptable crossover sequence.

Figure 16 illustrates cell killing induced by CD20-specific A domain monomers.

Figure 17 illustrates binding of A domain monomer-expressing phage to recombinant TPO-R abd TF1 cells.

Figure 18 illustrates TF1 cell proliferation in response to TPO-R-specific A domain monomers and multimers.

Figure 19 illustrates IgE-specific A domain monomer and multimer-expressing phage binding to (a) IgE directly immobilized on a plate, or (b) IgE immobilized on the plate by binding to an immobilized antibody to IgE's CE2 domain, or (c) IgE immobilized on the plate by binding to an immobilized antibody to IgE's CE3 domain, or (d) immobilized by binding to immobilized IgE receptor R1.

Figure 20 illustrates ELISA binding data of selected A-domain monomers that bind to CD28.

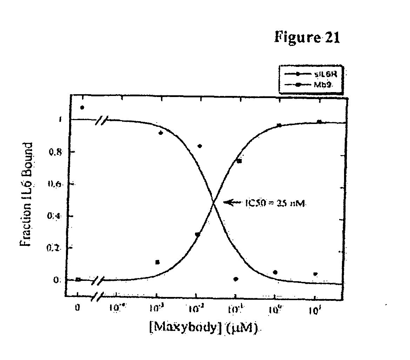

Figure 21 illustrates results from a competition ELISA experiment where soluble IL6 receptor and monomer Mb9 are competed against immobilized IL6.

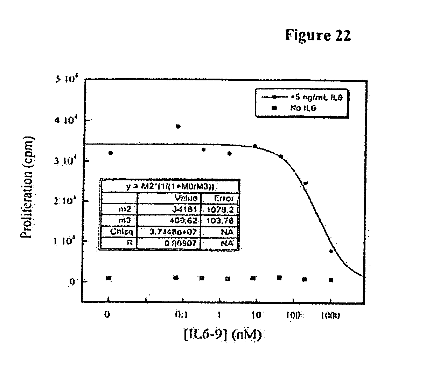

Figure 22 illustrates cell proliferation inhibition of by a IL6-specific monomer.

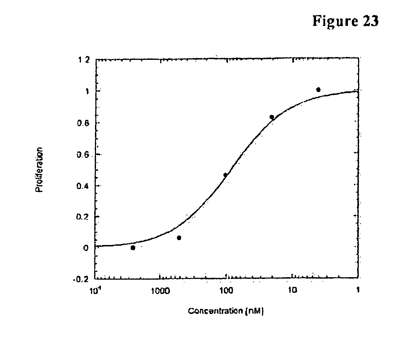

Figure 23 illustrates the effect of an IL6-specific monomer on isolated peripheral blood lymphocytes (PBMC).

Figure 24 illustrates screening a library of monomer domains against multiple ligands displayed on a cell.

Figure 25 illustrates identification of monomers that were selected to bind to one of a plurality of ligands.

Figure 26 illustrates an embodiment for identifying polynucleotides encoding ligands and monomer domains.

Figure 27 illustrates monomer domain and multimer embodiments for increased avidity. While the figure illustrates specific gene products and binding affinities, it is appreciated that these are merely examples and that other binding targets can be used with the same or similar conformations.

Figure 28 illustrates monomer domain and multimer embodiments for increased avidity. While the figure illustrates specific gene products and binding affinities, it is appreciated that these are merely examples and that other binding targets can be used with the same or similar conformations.

DETAILED DESCRIPTION OF THE INVENTION

The invention provides an enhanced approach for selecting and optimizing properties of discrete monomer domains and/or immuno-domains to create multimers. In particular, this disclosure describes methods, compositions and kits for identifying discrete monomer domains and/or immuno-domains that bind to a desired ligand or mixture of ligands and creating multimers (also known as combinatorial mosaic proteins or recombinant mosaic proteins) that comprise two or more monomer domains and/or immuno-domains that are joined via a linker. The multimers can be screened to identify those that have an improved phenotype such as improved avidity or affinity or altered specificity for the ligand or the mixture of ligands, compared to the discrete monomer domain.

### 1. Discrete Monomer Domains

Monomer domains can be polypeptide chains of any size. In some embodiments, monomer domains have about 25 to about 500, about 30 to about 200, about 30 to about 100, about 90 to about 200, about 30 to about 250, about 30 to about 60, about 9 to about 150, about 100 to about 150, about 25 to about 50, or about 30 to about 150 amino acids. Similarly, a monomer domain of the present invention can comprise, e.g., from about 30 to about 200 amino acids; from about 25 to about 180 amino acids; from about 40 to about 150 amino acids; from about 50 to about 130 amino acids; or from about 75 to about 125 amino acids. Monomer domains and immuno-domains can typically maintain a stable conformation in solution. Sometimes, monomer domains and immuno-domains can fold independently into a stable conformation. In one embodiment, the stable conformation is stabilized by metal ions. The stable conformation can optionally contain disulfide bonds (e.g., at least one, two, or three or more disulfide bonds). The disulfide bonds can optionally be formed between two cysteine residues. In some embodiments, monomer domains, or monomer domain variants, are substantially identical to the sequences exemplified (e.g., A, C2) or otherwise referenced herein.

Publications describing monomer domains and mosaic proteins and references cited within include the following: Hegyi, H and Bork, P., On the classification and evolution of protein modules, (1997) J. Protein Chem., 16(5):545-551; Baron et al., Protein modules (1991) Trends Biochem. Sci., 16(1):13-7; Ponting et al., Evolution of domain families, (2000), Adv. Protein Chem., 54:185-244; Doolittle, The multiplicity of domains in proteins, (1995) Annu. Rev. Biochem 64:287-314; Doolitte and Bork, Evolutionarily mobile modules in proteins (1993) Scientific American, 269 (4):50-6; and Bork, Shuffled domains in extracellular proteins (1991), FEBS letters 286(1-2):47-54. Monomer domains of the present invention also include those domains found in Pfam database and the SMART database. *See* Schultz, et al., SMART: a web-based tool for the study of genetically mobile domains, (2000) Nucleic Acid Res. 28(1):231-34.

Monomer domains that are particularly suitable for use in the practice of the present invention are (1)β sandwich domains; (2) β-barrel domains; or (3) cysteine-rich domains comprising disulfide bonds. Cysteine-rich domains employed in the practice of the present invention typically do not form an α helix, a β sheet, or a β-barrel structure. Typically, the disulfide bonds promote folding of the domain into a three-dimensional structure. Usually, cysteine-rich domains have at least two disulfide bonds, more typically at least three disulfide bonds.

Domains can have any number of characteristics. For example, in some embodiments, the domains have low or no immunogenicity in an animal (e.g., a human). Domains can have a small size. In some embodiments, the domains are small enough to penetrate skin or other tissues. Domains can have a range of *in vivo* half-lives or stabilities.

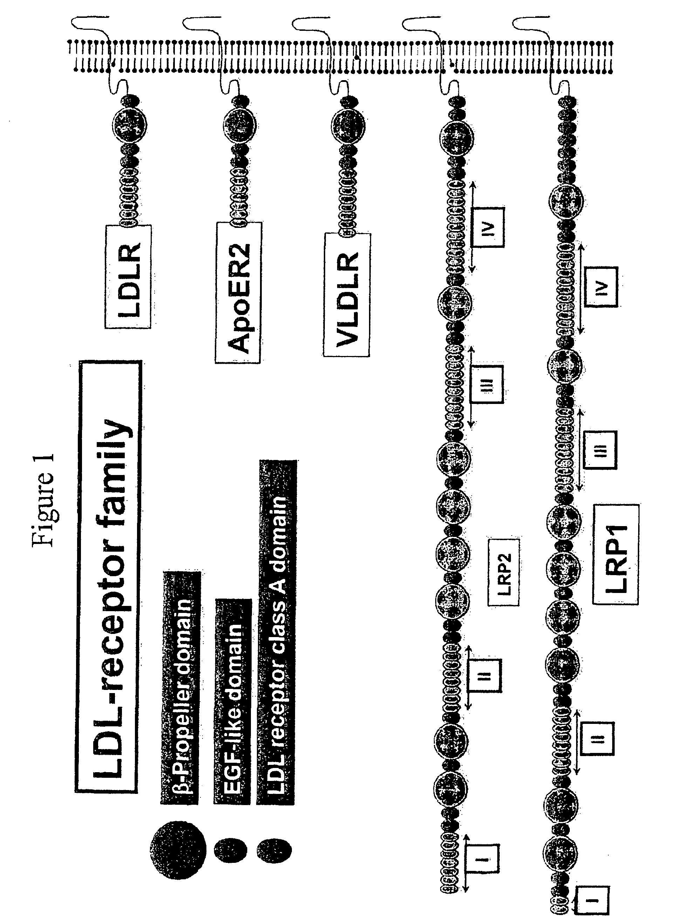

Illustrative monomer domains suitable for use in the practice of the present invention include, e.g., an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an Immunoglobulin-like domain, a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain, a Laminin-type EGF-like domain, a C2 domain, and other such domains known to those of ordinary skill in the art, as well as derivatives and/or variants thereof. In some embodiments, the monomer domain is not the C2 domain. Figure 1 schematically diagrams various kinds of monomer domains found in molecules in the LDL-rcceptor family.

In some embodiments, suitable monomer domains (e.g. domains with the ability to fold independently or with some limited assistance) can be selected from the families of protein domains that contain β-sandwich or β-barrel three dimensional structures as defined by such computational sequence analysis tools as Simple Modular Architecture Research Tool (SMART), *see* Shultz et.al., SMART: a web-based tool for the study of genetically mobile domains, (2000) Nucleic Acids Research 28(1):231-234) or CATH (*see* Pearl et.al., Assigning genomic sequences to CATH, (2000) Nucleic Acids Research 28(1):277-282).

In another embodiment, monomer domains of the present invention include domains other than a fibronectin type III domain, an anticalin domain and a Ig-like domain from CTLA-4. Some aspects of these domains are described in WO01/64942 entitled "Protein scaffolds for antibody mimics and other binding proteins" by Lipovsek et al., published on September 7, 2001, WO99/16873 entitled "Anticalins" by Beste et al., published April 8, 1999 and WO 00/60070 entitled "A polypeptide structure for use as a scaffold" by Desmet, et al., published on October 12, 2000.

As described *supra,* monomer domains are optionally cysteine rich. Suitable cysteine rich monomer domains include, e.g., the LDL receptor class A domain ("A-domain") or the EGF-like domain. The monomer domains can also have a cluster of negatively charged residues. Optionally, the monomer domains contain a repeated sequence, such as YWTD (SEQ ID NO: 198) as found in the β-Propeller domain.

Other features of monomer domains include the ability to bind ligands (e.g., as in the LDL receptor class A domain, or the CUB domain (complement Clr/Cls, Uegf, and bone morphogenic protein-1 domain)), the ability to participate in endocytosis or internalization (e.g., as in the cytoplasmic tail of the LDL receptor or the cytoplasmic tail of Megalin), the ability to bind an ion (e.g., $Ca^{2+}$ binding by the LDL receptor A-domain), and/or the ability to be involved in cell adhesion (e.g., as in the EGF-like domain). Other monomer domains that bind ions to maintain their secondary structure include, e.g., A domain, EGF domain, EF Hand (e.g., such as those found in present in calmodulin and troponin C), Cadherin domain, C-type lectin, C2 domain, Annexin, Gla-domain, Trombospondin type 3 domain, all of which bind calcium, and and zinc fingers (e.g., C2H2 type C3HC4 type (RING finger), Integrase Zinc binding domain, PHD finger, GATA zinc finger, FYVE zinc finger, B-box zinc finger), which bind zinc. Without intending to limit the invention, it is believed that ion-binding provides stability of secondary structure while providing sufficient flexibility to allow for numerous binding conformations depending on primary sequence.

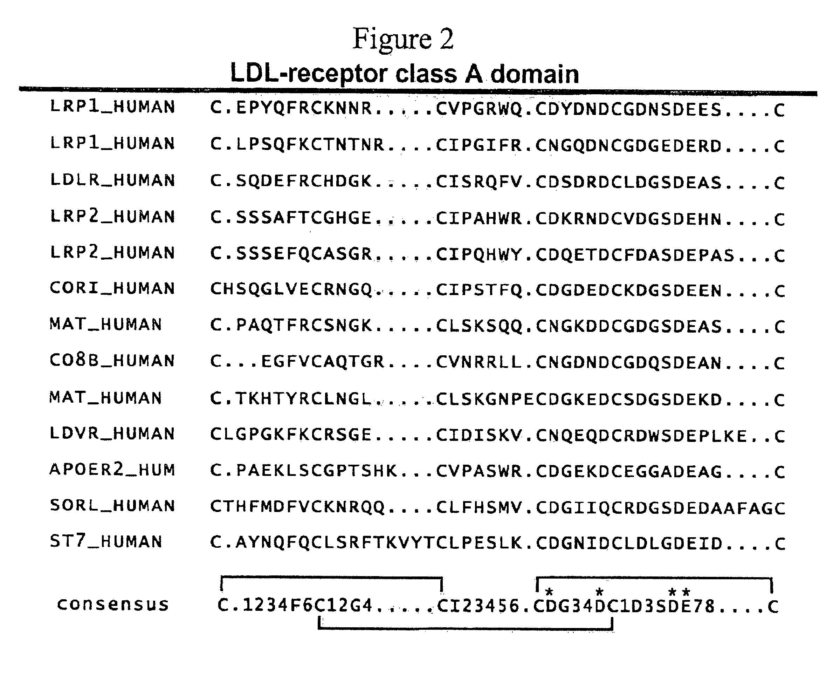

Characteristics of a monomer domain include the ability to fold independently and the ability to form a stable structure. Thus, the structure of the monomer domain is often conserved, although the polynucleotide sequence encoding the monomer need not be conserved. For example, the A-domain structure is conserved among the members of the A-domain family, while the A-domain nucleic acid sequence is not. Thus, for example, a monomer domain is classified as an A-domain by its cysteine residues and its affinity for calcium, not necessarily by its nucleic acid sequence. *See*, Figure 2.

Specifically, the A-domains (sometimes called "complement-type repeats") contain about 30-50 or 30-65 amino acids. In some embodiments, the domains comprise about 35-45 amino acids and in some cases about 40 amino acids. Within the 30-50 amino acids, there are about 6 cysteine residues. Of the six cysteines, disulfide bonds typically are found between the following cysteines: C1 and C3, C2 and C5, C4 and C6. The A domain constitutes a ligand binding moiety. The cysteine residues of the domain are disulfide linked to form a compact, stable, functionally independent moiety. *See,* Figure 3. Clusters of these repeats make up a ligand binding domain, and differential clustering can impart specificity with respect to the ligand binding.

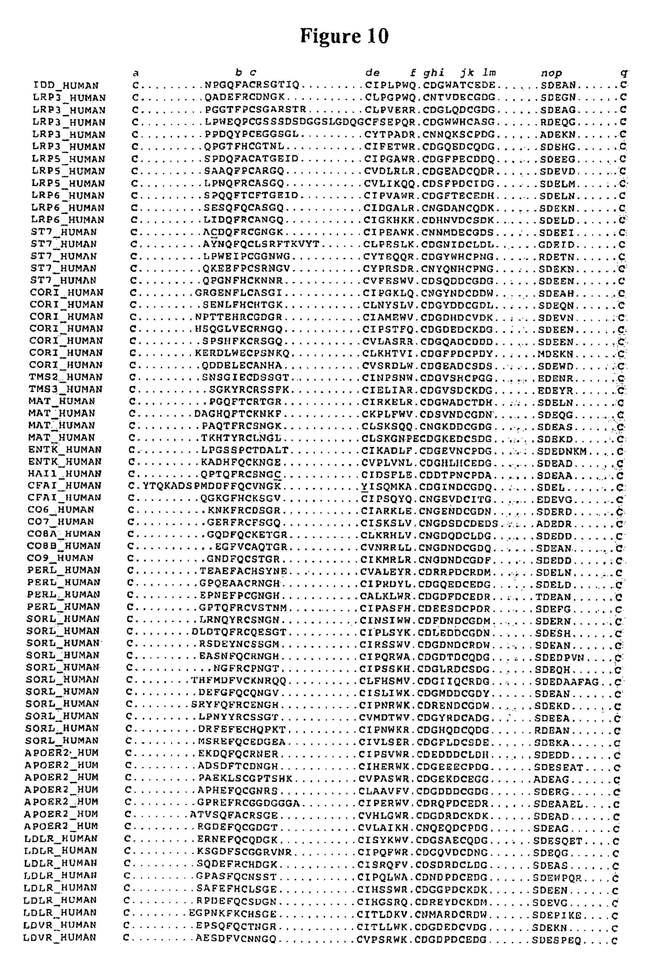

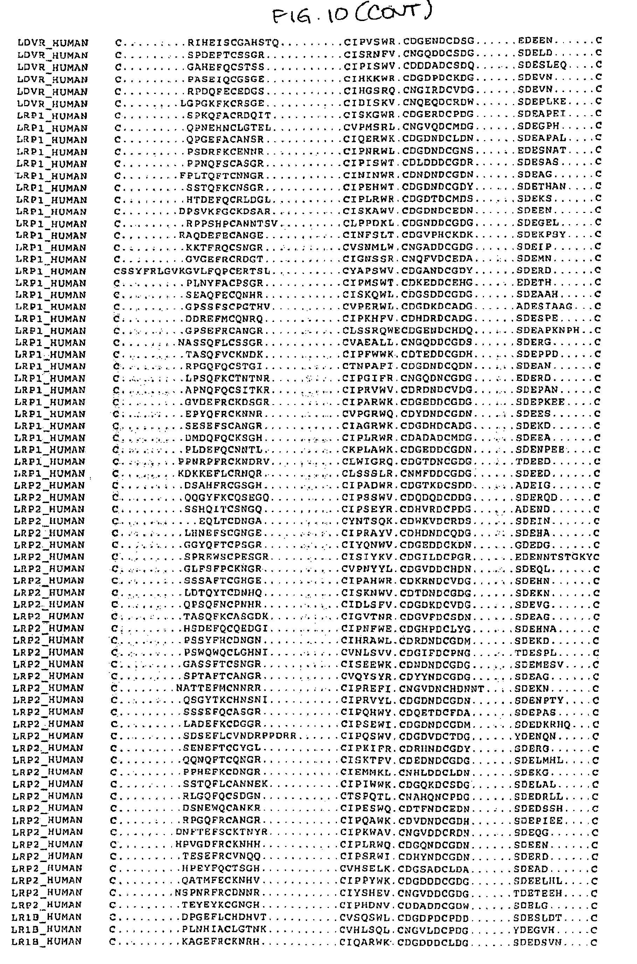

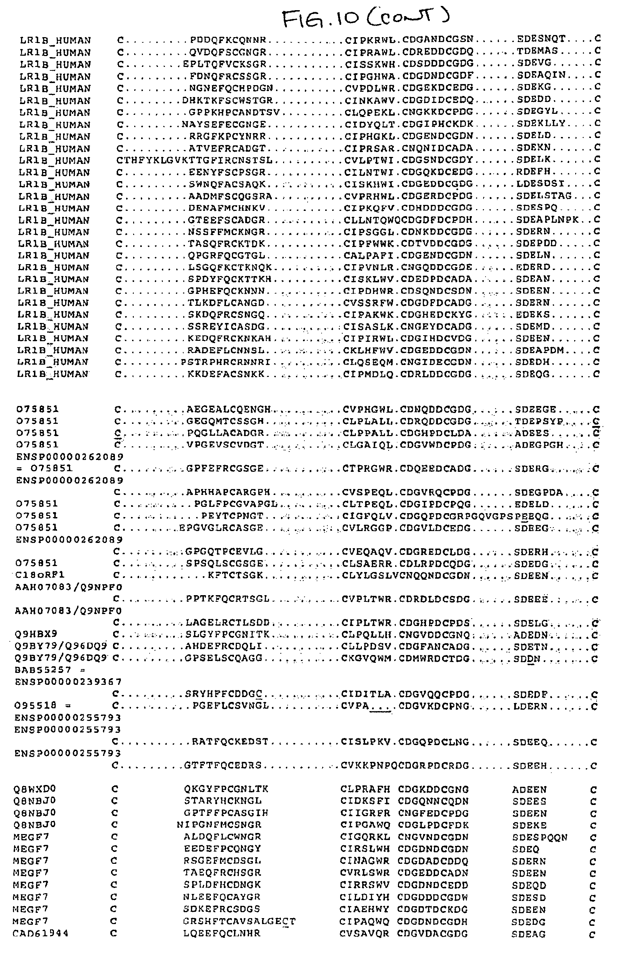

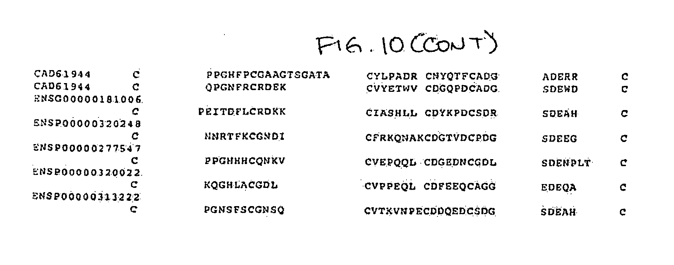

Exemplary A domain sequences and consensus sequences are depicted in Figures 2, 3 and 8. Figure 9 displays location and occurrence of residues in A domains with the following spacing between cysteines. In addition, Figure 10 depicts a number of A domains and provides a listing of conserved amino acids. One typical consensus sequence useful to identify A domains is the following: C-[VILMA]-$X_{(5)}$-C-[DNH]-$X_{(3)}$-[DENQHT]-C-$X_{(3,4)}$-[STADE]- [DEH]-[DE]-$X_{(1,5)}$-C (SEQ ID NO: 200), where the residues in brackets indicate possible residues at one position. "$X_{(\#)}$" indicates number of residues. These residues can be any amino acid residue. Parentheticals containing two numbers refers to the range of amino acids that can occupy that position (e.g., "[DE]-$X_{(1,5)}$-C" means that the amino acids DE are followed by 1, 2, 3, 4, or 5 residues, followed by C). This consensus sequence only represents the portion of the A domain beginning at the third cysteine. A second consensus is as follows: C-$X_{(3-15)}$-C-$X_{(4-15)}$-C-$X_{(6-7)}$-C-[N,D]-$X_{(3)}$-[D,E,N,Q,H,S,T]-C-$X_{(4-6)}$-D-E-$X_{(2-8)}$-C (SEQ ID NO: 201). The second consensus predicts amino acid residues spanning all six cysteine residues. In some embodiments, A domain variants comprise sequences substantially identical to any of the above-described sequences.

Additional exemplary A domains include the following sequence:

$$C_a X_{3-15} C_b X_{3-15} C_c X_{6-7} C_d (D,N) X_4 C_e X_{4-6} DE X_{2-8} C_f$$

wherein C is cysteine, $X_{n-m}$ represents between n and m number of independently selected amino acids, and (D,N) indicates that the position can be either D or N; and wherein $C_a$-$C_c$, $C_b$-$C_e$ and $C_d$-$C_f$ form disulfide bonds.

In some embodiments, the monomer domain is an LDL receptor class A domain monomer comprising the following sequence:

$$CaX_{6-7} C_b X_{4-5} C_c X_6 C_d X_5 C_e X_{8-10} C_f$$

wherein X is defined as follows:

The table above indicates alternative amino acid residues at each position of the LDL receptor class A monomer domain. For example, there can be either 6 or 7 amino acids between cysteine C1 and cysteine C2. The upper left box of the table indicates alternative amino acid residues at each position if there are 6 amino acids between C1 and C2. The bottom left box in the table indicates alternative amino acid residues if there are seven amino acids between C1 and C2. In all cases, the amino acid for one position (e.g., X1) is selected independently of the amino acids selected for remaining positions (e.g., X2, X3, etc.)

To date, at least 190 human A-domains are identified based on cDNA sequences. *See, e.g.,* Figure 10. Exemplary proteins containing A-domains include, e.g., complement components (e.g., C6, C7, C8, C9, and Factor I), serine proteases (e.g., enteropeptidase, matriptase, and corin), transmembrane proteins (e.g., ST7, LRP3, LRP5 and LRP6) and endocytic receptors (e.g., Sortilin-related receptor, LDL-receptor, VLDLR, LRP1, LRP2, and ApoER2). A domains and A domain variants can be readily employed in the practice of the present invention as monomer domains and variants thereof. Further description of A domains can be found in the following publications and references cited therein: Howell and Hertz, The LDL receptor gene family: signaling functions during development, (2001) Current Opinion in Neurobiology 11:74-81; Herz (2001), *supra*; Krieger, The "best" of cholesterols, the "worst " of cholesterols: A tale of two receptors, (1998) PNAS 95: 4077-4080; Goldstein and Brown, The Cholesterol Quartet, (2001) Science, 292: 1310-1312; and, Moestrup and Verroust, Megalin-and Cubilin-Mediated Endocytosis of Protein-Bound Vitamins, Lipids, and Hormones in Polarized Epithelia, (2001) Ann. Rev. Nutr. 21:407-28.

Another exemplary monomer domain suitable for use in the practice of the present invention is the C2 domain. C2 monomer domains are polypeptides containing a compact β-sandwich composed of two, four-stranded β-sheets, where loops at the "top" of the domain and loops at the "bottom" of the domain connect the eight β-strands. C2 monomer domains may be divided into two subclasses, namely C2 monomer domains with topology I (synaptotagmin-like topology) and topology II (cytosolic phospholipase A2-like topology), respectively. C2 monomer domains with topology I contains three loops at the "top" of the molecule (all of which are $Ca^{2+}$ binding loops), whereas C2 monomer domains with topology II contain four loops at the "top" of the molecule (out of which only three are $Ca^{2+}$ binding loops). The structure of C2 monomer domains have been reviewed by Rizo and Südhof, J. Biol. Chem: 273;15879-15882 (1998) and by Cho, J. Biol. Chem. 276;32407-32410 (2001). The terms "loop region 1", "loop region 2" and "loop region 3" refer to the $Ca^{2+}$ binding loop regions located at the "top" of the molecule. This nomenclature, which is used to distinguish the three $Ca^{2+}$ binding loops located at the "top" of the molecule from the non-$Ca^{2+}$ binding loops (mainly located at the "bottom" of the molecule) is widely used and recognized in the literature. *See* Rizo and Südhof, J. Biol. Chem. 273;15879-15882 (1998). Loop regions 1, 2, and 3 represent target binding regions and thus can be varied to modulate binding specificity and affinity. The remaining portions of the C2 domain can be maintained without alteration if desired. Some exemplary C2 domains are substantially identical to the following sequence (SEQ ID NO: 202):

| Tyr 1 | Ser | His | Lys | Phe 5 | Thr | Val | Val | Val | Leu 10 | Arg | Ala | Thr | Lys | Val 15 |
|---|---|---|---|---|---|---|---|---|---|---|---|---|---|---|
| Thr | Lys | Gly | Ala | Phe 20 | Gly | Asp | Met | Leu | Asp 25 | Thr | Pro | Asp | Pro | Tyr 30 |
| Val | Glu | Leu | Phe | Ile 35 | Ser | Thr | Thr | Pro | Asp 40 | Ser | Arg | Lys | Arg | Thr 45 |
| Arg | His | Phe | Asn | Asn 50 | Asp | Ile | Asn | Pro | Val 55 | Trp | Asn | Glu | Thr | Phe 60 |
| Glu | Phe | Ile | Leu | Asp 65 | Pro | Asn | Gln | Glu | Asn 70 | Val | Leu | Glu | Ile | Thr 75 |
| Leu | Met | Asp | Ala | Asn 80 | Tyr | Val | Met | Asp | Glu 85 | Thr | Leu | Gly | Thr | Ala 90 |
| Thr | Phe | Thr | Val | Ser 95 | Ser | Met | Lys | Val | Gly 100 | Glu | Lys | Lys | Glu | Val 105 |
| Pro | Phe | Ile | Phe | Asn 110 | Gln | Val | Thr | Glu | Met 115 | Val | Leu | Glu | Met | Ser 120 |
| Leu | Glu | Val 123. | | | | | | | | | | | | |

Residues 1-16, 29-48, 54-77 and 86-123 constitute positions located outside loop regions 1, 2 and 3 and residues 17-28, 49-53 and 78-85 constitute the loop regions 1, 2 and 3, respectively.

Other examples of monomer domains can be found in the protein Cubilin, which contains EGF-type repeats and CUB domains. The CUB domains are involved in ligand binding, e.g., some ligands include intrinsic factor (IF)-vitamin B12, receptor associated protein (RAP), Apo A-I, Transferrin, Albumin, Ig light chains and calcium. *See,* Moestrup and Verroust, *supra.*

Megalin also contains multiple monomer domains. Specifically, mcgalin possesses LDL-receptor type A-domain, EGF-type repeat, a transmembrane segment and a cytoplasmic tail. Megalin binds a diverse set of ligands, e.g., ApoB, ApoE, ApoJ, clusterin, ApopH/Beta2-glycoprotein-I, PTH, Transthyretin, Thyroglobulin, Insulin, Aminoglycosides, Polymyxin B, Aprotinin, Trichosanthin, PAI-1, PAI-1-urokinase, PAI-1-tPA, Pro-urokinase, Lipoprotein lipase, alpha-Amylase, Albumin, RAP, Ig light chains, calcium, C1q, Lactoferrin, beta2-microglobulin, EGF, Prolactin, Lysozyme, Cytochrome c, PAP-1, Odorant-binding protein, seminal vesicle secretory protein II. *See,* Moestrup & Verroust, *supra.*

Exemplary EGF monomer domains include the sequence:

$$C_aX_{3\text{-}14}C_bX_{3\text{-}7}C_cX_{4\text{-}16}C_dX_{1\text{-}2}C_eX_{8\text{-}23}C_f$$

wherein C is cysteine, $X_{n\text{-}m}$ represents between n and m number of independently selected amino acids; and wherein $C_a$-$C_c$, $C_b$-$C_e$ and $C_d$-$C_f$ form disulfide bonds.

In some embodiments, the monomer domain is an EGF domain monomer comprising the following sequence:

$$C_aX_{4-6}C_bX_{3-5}C_cX_{8-9}C_dX_1C_eX_{8-12}C_f$$

wherein X is defined as follows:

EGF monomer domains are sometimes between 25-45 amino acids and typically 30-39 amino acids.

Descriptions of some exemplary monomer domains can be found in the following publications and the references cited therein: Yamazaki et al., Elements of neutral Adhesion Molecules and a Yeast Vacuolar Protein Sorting Receptor are Present in a Novel Mammalian Low Density Lipoprotein Receptor Family Member, (1996) Journal of Biological Chemistry 271(40) 24761-24768; Nakayama et al., Identification of High-Molecular-Weight Proteins with Multiple EGF-like Motifs by Motif-Trap Screening, (1998) Genomics 51:27-34; Liu et al, Genomic Organization of New Candidate Tumor Suppressor Gene, LRPIB, (2000) Genomics 69:271-274; Liu et al., The Putative Tumor Suppressor LRP1B, a Novel Member of the Low Density Lipoprotein (LDL) Receptor Family, Exhibits Both Overlapping and Distinct Properties with the LDL Receptor-related Protein, (2001) Journal of Biological Chemistry 276(31):28889-28896; Ishii et al, cDNA of a New Low-Density Lipoprotein Receptor-Related Protein and Mapping of its Gene (LRP3) to Chromosome Bands 19q12-q13.2, (1998) Genomics 51:132-135; Orlando et al, Identification of the second cluster of ligand-binding repeats in megalin as a site for receptor-ligand interactions, (1997) PNAS USA 94:2368-2373; Jeon and Shipley, Vesicle-reconstituted Low Density Lipoprotein Receptor, (2000) Journal of Biological Chemistry 275(39):30458-30464; Simmons et al., Human Low Density Lipoprotein Receptor Fragment, (1997) Journal of Biological Chemistry 272(41):25531-25536; Fass et al., Molecular Basis of familial hypercholesterolaemia from structure of LDL receptor module, (1997) Nature 388:691-93; Daly et al., Three-dimensional structure of a cysteine-rich repeat from the low-density lipoprotein receptor, (1995) PNAS USA 92:6334-6338; North and Blacklow, Structural Independence of Ligand-Binding Modules Five and Six of the LDL Receptor, (1999) Biochemistry 38:3926-3935; North and Blacklow, Solution Structure of the Sixth LDL-A module of the LDL Receptor, (2000) Biochemistry 39:25640-2571; North and Blacklow, Evidence that Familial Hypercholeslerolemia Mutations of the LDL Receptor Cause Limited Local Misfolding in an LDL-A Module Pair, (2000) Biochemistry 39:13127-13135; Beglova et al., Backbone Dynamics of a Module Pair from the Ligand-Binding Domain of the LDL Receptor, (2001) Biochemistry 40:2808-2815; Bieri et al., Folding, Calcium binding, and Structural Characterization of a Concatemer of the Fist and Second Ligand-Binding Modules of the Low-Density Lipoprotein Receptor, (1998) Biochemistry 37:10994-11002; Jeon et al., Implications for familial hypercholesterolemia from the structure of the LDL receptor YWTD-EGF domain pair, (2001) Nature Structural Biology 8(6):499-504; Kurniawan et al., NMR structure of a concatemer of the first and second ligand-binding modules of the human low-density lipoprotein receptor, (2000) Protein Science 9:1282-1293; Esser et al., Mutational Analysis of the Ligand Binding Domain of the Low Density Lipoprotein Receptor, (1988) Journal of Biological Chemistry 263(26):13282-13290; Russell et al., Different Combinations of Cysteine-rich Repeats Mediate Binding of Low Density Lipoprotein Receptor to Two Different Proteins, (1989) Journal of Biological Chemistry 264(36):21682-21688; Davis et al., Acid-dependent ligand dissociation and recycling of LDL receptor meditated by growth factor homology region, (1987) Nature 326:760-765; Rong et al., Conversion of a human low-density lipoprotein receptor ligand binding repeat to a virus receptor: Identification of residues important for ligand specificity, (1998) PNAS USA 95:8467-8472; Agnello et al., Hepatitis C virus and other Flaviviridae viruses enter cells via low density lipoprotein receptor; (1999) PNAS 96(22):12766-1277 1; Esser and Russell, Transport-deficient Mutations in the Low Density lipoprotein receptor, (1988) Journal of Biological Chemistry 263(26):13276-1328 1; Davis et al., The Low Density Lipoprotein Receptor, (1987) Journal of Biological Chemistry 262(9):4075-4082; and, Peacock et al., Human Low Density Lipoprotein Receptor Expressed in Xenopus Oocytes, (1988) Journal of Biological Chemistry 263(16): 7838-7845.

Other publications that describe the VLDLR, ApoER2 and LRP1 proteins and their monomer domains include the following as well as the references cited therein: Savonen et al., The Carboxyl-terminal Domain of Receptor-associated Protein Facilitates Proper Folding and Trafficking of the Very Low Density Lipoprotein Receptor by Interaction with the Three Amino-terminal Ligand-binding Repeats of the Receptor, (1999) Journal of Biological Chemistry 274(36): 25877-25882; Hewat et al., The cellular receptor to human rhinovirus 2 binds around the 5-fold axis and not in the canyon: a structural view, (2000) EMBO Journal 19(23):6317-6325; Okun et al., VLDL Receptor Fragments of Different Lengths Bind to Human Rhinovirus HRV2 with Different Stoichiometry, (2001) Journal of Biological Chemistry 276(2): 1057-1062; Rettenberger et al., Ligand Binding Properties of the Very Low Density Lipoprotein Receptor, (1999) Journal of biological Chemistry 274(13):8973-8980; Mikhailenko et al., Functional Domains of the very low density lipoprotein receptor: molecular analysis of ligand binding and acid-dependent ligand dissociation mechanisms, (1999) Journal of Cell Science 112:3269-3281; Brandes et al., Alternative Splicing in the Ligand Binding Domain of Mouse ApoE Receptor-2 Produces Receptor Variants Binding Reelin but not alpa2-macroglobulin, (2001) Journal of Biological Chemistry 276 (25):22160-22169; Kim et al., Exon/Intron Organization, Chromosome Localization, Alternative Splicing, and Transcription Units of the Human Apolipoprotein E Receptor 2 Gene, (1997) Journal of Biological Chemistry 272(13):8498-8504; Obermoeller-McCormick et al., Dissection of receptor folding and ligand-binding property with functional minireceptors of LDL receptor-related protein, (2001) Journal of Cell Science 114(5):899-908; Hom et al., Molecular Analysis of Ligand Binding of the Second Cluster of Complement-type Repeats of the Low Density Lipoprotein Receptor-related Protein, (1997) Journal of Biological Chemistry 272(21):13608-13613; Neels et al., The Second and Fourth Cluster of Class A Cysteine-rich Repeats of the Low Density Lipoprotein Receptor-related Protein Share Ligand-binding Properties, (1999) Journal of Biological Chemistry 274(44):31305-31311; Obermoeller et al., Differential Functions of the Triplicated Repeats Suggest Two Independent Roles for the Receptor-Associated Protein as a Molecular Chaperone, (1997) Journal of Biological Chemistry 272(16):10761-10768; Andersen et al., Identification of the Minimal Functional Unit in the Low Density Liporerein Receptor-related Protein for Binding the Receptor-associated Protein (RAP), (2000) Journal of Biological Chemistry 275(28):21017-21024; Andersen et al., Specific Binding of alpha-Macroglobulin to Complement-Type Repeat CR4 of the Low-Density Lipoprotein Receptor-Related Protein, (2000) Biochemistry 39:10627-10633; Vash et al., Three Complement-Type Repeats of the Low-Density Lipoprotein Receptor-Related Protein Define a Common Binding Site for RAP, PAI-1, and Lactoferrin, (1998) Blood 92(9):3277-3285; Dolmer et al., NMR Solution Structure of Complement-like Repeat CR3 from the L ow Density Lipoprotein Receptor-related Protein, (2000) Journal of Biological Chemistry 275(5):3264-3269; Huang et al., NMR Solution Structure of Complement-like Repeat CR8 from the Low Density Lipoprotein Receptor-related Protein, (1999) Journal of Biological Chemistry 274(20):14130-14136; and Liu et al., Uptake of HIV-1 Tat protein mediated by low-density lipoprotein receptor-related protein disrupts the neuronal metabolic balance of the receptor ligands, (2000) Nature Medicine 6(12):1380-1387.

Other references regarding monomer domains also include the following publications and references cited therein: FitzGerald et al, Pseudomonas Exotoxin-mediated Selection Yields Cells with Altered Expression of Low-Density Lipoprotein Receptor-related Protein, (1995) Journal of Cell Biology, 129: 1533-41; Willnow and Herz, Genetic deficiency in low density lipoprotein receptor-related protein confers cellular resistance to Pseudomonas exotoxin A, (1994) Journal of Cell Science, 107:719-726; Trommsdorf et al., Interaction of Cytosolic Adaptor Proteins with Neuronal Apolipoprotein E Receptors and the Amyloid Precursor Protein, (1998) Journal of Biological Chemistry, 273(5): 33556-33560; Stockinger et al., The Low Density Lipoprotein Receptor Gene Family, (1998) Journal of Biological Chemistry, 273(48): 32213-32221; Obermoeller et al., Ca+2 and Receptor-associated Protein are independently required for proper folding and disulfide bond formation of the low density lipoprotein receptor-related protein, (1998) Journal of Biological Chemistry, 273(35): 22374-22381; Sato et al., 39-kDa receptor-associated protein (RAP) facilitates secretion and ligand binding of extracellular region of very-low-density-lipoprotein receptor: implications for a distinct pathway from low-density-lipoprotein receptor, (1999) Biochem. J., 341:377-383; Avromoglu et al, Functional Expression of the Chicken Low Density Lipoprotein Receptor-related Protein in a mutant Chinese Hamster Ovary Cell Line Restores Toxicity of Pseudomonas Exotoxin A and Degradation of alpha2-Macroglobulin, (1998) Journal of Biological Chemistry, 273(11) 6057-6065; Kingsley and Krieger, Receptor-mediated endocytosis of low density lipoprotein: Somatic cell mutants define multiple genes required for expression of surface-receptor activity, (1984) PNAS USA, 81:5454-5458; Li et al, Differential Functions of Members of the Low Density Lipoprotein Receptor Family Suggests by their distinct endocystosis rates, (2001) Journal of Biological Chemistry 276(21):18000-18006; and, Springer, An Extracellular beta-Propeller Module Predicted in Lipoprotein and Scavenger Receptors, Tyrosine Kinases, Epidermal Growth Factor Precursor, and Extracellular Matrix Components, (1998) J. Mol. Biol. 283:837-862.

Polynucleotides (also referred to as nucleic acids) encoding the monomer domains are typically employed to make monomer domains via expression. Nucleic acids that encode monomer domains can be derived from a variety of different sources. Libraries of monomer domains can be prepared by expressing a plurality of different nucleic acids encoding naturally occurring monomer domains, altered monomer domains (i.e., monomer domain variants), or a combinations thereof.

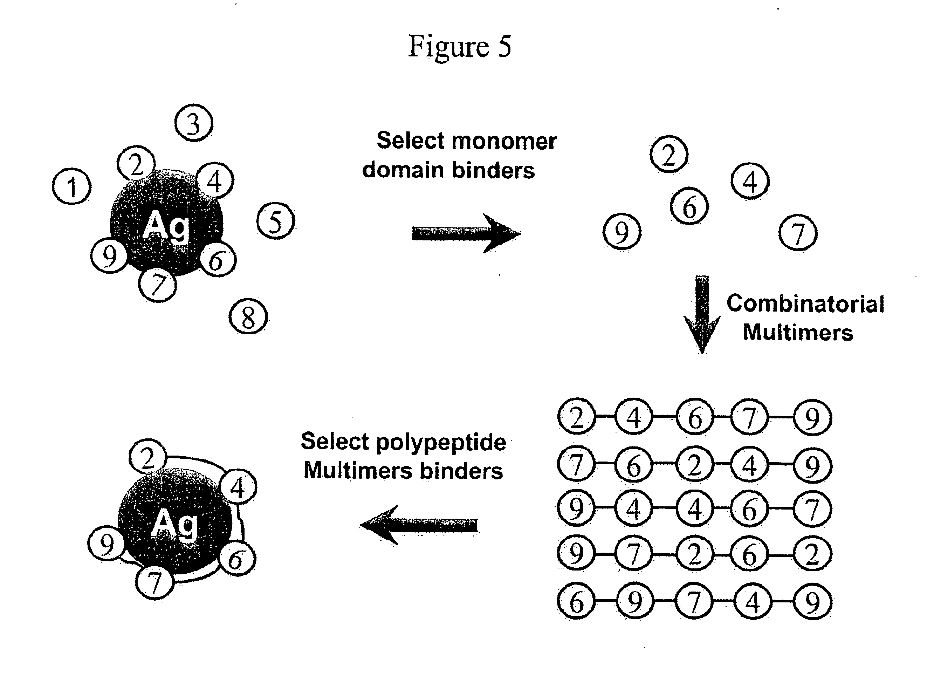

The disclosure provides methods of identifying monomer domains that bind to a selected or desired ligand or mixture of ligands. In some embodiments, monomer domains and/or immuno-domains are identified or selected for a desired property (e.g., binding affinity) and then the monomer domains and/or immuno-domains are formed into multimers. See, e.g., Figure 5. For those embodiments, any method resulting in selection of domains with a desired property (e.g., a specific binding property) can be used. For example, the methods can comprise providing a plurality of different nucleic acids, each nucleic acid encoding a monomer domain; translating the plurality of different nucleic acids, thereby providing a plurality of different monomer domains; screening the plurality of different monomer domains for binding of the desired ligand or a mixture of ligands; and, identifying members of the plurality of different monomer domains that bind the desired ligand or mixture of ligands.

As mentioned above, monomer domains can be naturally-occurring or altered (non-natural variants). The term "naturally occurring" is used herein to indicate that an object can be found in nature. For example, natural monomer domains can include human monomer domains or optionally, domains derived from different species or sources, e.g., mammals, primates, rodents, fish, birds, reptiles, plants, etc. The natural occurring monomer domains can be obtained by a number of methods, e.g., by PCR amplification of genomic DNA or cDNA.

Monomer domains of the present invention can be naturally-occurring domains or non-naturally occurring variants. Libraries of monomer domains employed in the practice of the present invention may contain naturally-occurring monomer domain, non-naturally occurring monomer domain variants, or a combination thereof.

Monomer domain variants can include ancestral domains, chimeric domains, randomized domains, mutated domains, and the like. For example, ancestral domains can be based on phylogenetic analysis. Chimeric domains are domains in which one or more regions are replaced by corresponding regions from other domains of the same family. For example, chimeric domains can be constructed by combining loop sequences from multiple related domains of the same family to form novel domains with potentially lowered immunogenicity. Those of skill in the art will recognized the immunologic benefit of constructing modified binding domain monomers by combining loop regions from various related domains of the same family rather than creating random amino acid sequences. For example, by constructing variant domains by combining loop sequences or even multiple loop sequences that occur naturally in human LDL receptor class A-domains, the resulting domains may contain novel binding properties but may not contain any immunogenic protein sequences because all of the exposed loops are of human origin. The combining of loop amino acid sequences in endogenous context can be applied to all of the monomer constructs of the invention. Thus the present disclosure provides a method for generating a library of chimeric monomer domains derived from human proteins, the method comprising: providing loop sequences corresponding to at least one loop from each of at least two different naturally occurring variants of a human protein, wherein the loop sequences are polynucleotide or polypeptide sequences; and covalently combining loop sequences to generate a library of at least two different chimeric sequences, wherein each chimeric sequence encodes a chimeric monomer domain having at least two loops. Typically, the chimeric domain has at least four loops, and usually at least six loops. As described above, the present invention provides three types of loops that are identified by specific features, such as, potential for disulfide bonding, bridging between secondary protein structures, and molecular dynamics (i.e., flexibility). The three types of loop sequences are a cysteine-defined loop sequence, a structure-defined loop sequence, and a B-factor-defined loop sequence.

Randomized domains are domains in which one or more regions are randomized. The randomization can be based on full randomization, or optionally, partial randomization based on natural distribution of sequence diversity.

The non-natural monomer domains or altered monomer domains can be produced by a number of methods. Any method of mutagenesis, such as site-directed mutagenesis and random mutagenesis (e.g., chemical mutagenesis) can be used to produce variants. In some embodiments, error-prone PCR is employed to create variants. Additional methods include aligning a plurality of naturally occurring monomer domains by aligning conserved amino acids in the plurality of naturally occurring monomer domains; and, designing the non-naturally occurring monomer domain by maintaining the conserved amino acids and inserting, deleting or altering amino acids around the conserved amino acids to generate the non-naturally occurring monomer domain. In one embodiment, the conserved amino acids comprise cysteines. In another embodiment, the inserting step uses random amino acids, or optionally, the inserting step uses portions of the naturally occurring monomer domains. The portions could ideally encode loops from domains from the same family. Amino acids are inserted or exchanged using synthetic oligonucleotides, or by shuffling, or by restriction enzyme based recombination. Human chimeric domains of the present invention are useful for therapeutic applications where minimal immunogenicity is desired. The present invention discloses methods for generating libraries of human chimeric domains. Human chimeric monomer domain libraries can be constructed by combining loop sequences from different variants of a human monomer domain, as described above. The loop sequences that are combined may be sequence-defined loops, structure-defined loops, B-factor-defined loops, or a combination of any two or more thereof.

Alternatively, a human chimeric domain library can be generated by modifying naturally occurring human monomer domains at the amino acid level, as compared to the loop level. To minimize the potential for immunogenicity, only those residues that naturally occur in protein sequences from the same family of human monomer domains are utilized to create the chimeric sequences. This can be achieved by providing a sequence alignment of at least two human monomer domains from the same family of monomer domains, identifying amino acid residues in corresponding positions in the human monomer domain sequences that differ between the human monomer domains, generating two or more human chimeric monomer domains, wherein each human chimeric monomer domain sequence consists of amino acid residues that correspond in type and position to residues from two or more human monomer domains from the same family of monomer domains. Libraries of human chimeric monomer domains can be employed to identify human chimeric monomer domains that bind to a target of interest by: screening the library of human chimeric monomer domains for binding to a target molecule, and identifying a human chimeric monomer domain that binds to the target molecule. Suitable naturally occurring human monomer domain sequences employed in the initial sequence alignment step include those corresponding to any of the naturally occurring monomer domains described herein.

Human chimeric domain libraries of the present invention (whether generated by varying loops or single amino acid residues) can be prepared by methods known to those having ordinary skill in the art. Methods particularly suitable for generating these libraries are split-pool format and trinucleotide synthesis format as described in WO01/23401.

In addition to the invention methods for generating human chimeric domain libraries, the present invention also provides a method for screening a protein for potential immunogenicity by:

        providing a candidate protein sequence;
        comparing the candidate protein sequence to a database of human protein sequences;
        identifying portions of the candidate protein sequence that correspond to portions of human protein sequences from the database; and
        determining the extent of correspondence between the candidate protein sequence and the human protein sequences from the database.

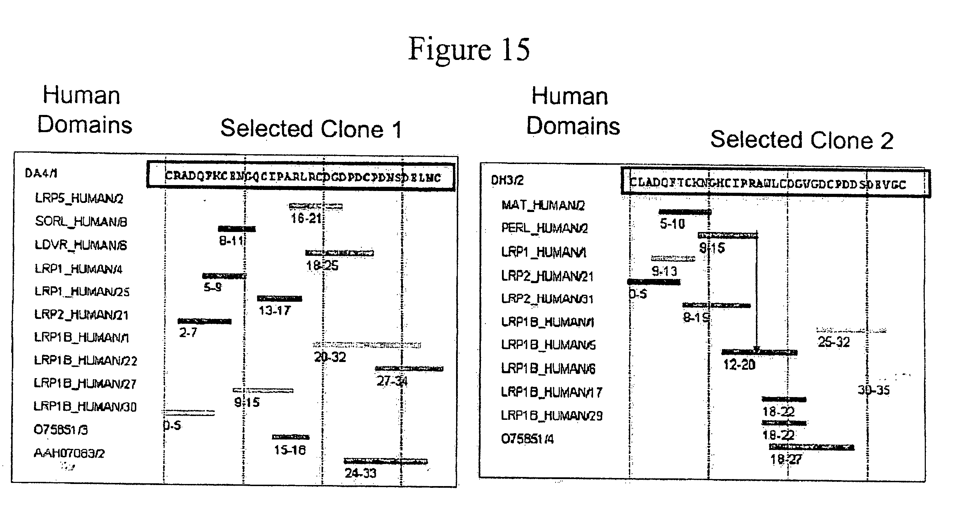

In general, the greater the extent of correspondence between the candidate protein sequence and one or more of the human protein sequences from the database, the lower the potential for immunogenicity is predicted as compared to a candidate protein having little correspondence with any of the human protein sequences from the database. A database of human protein sequences that is suitable for use in the practice of the invention method for screening candidate proteins can be found at ncbi.nlm.nih.gov/blast/Blast.cgi at the World Wide Web (in addition, the following web site can be used to search short, nearly exact matches: cbi.nlm.nih.gov/blast/Blast.cgi?CMD =Web&LAYOUT=TwoWindows&AUTO_FORMAT=Semiauto&ALIGNMENTS=50&ALI        GNMENT_VIEW=Pairwise&CLIENTweb&DATABASE=nr&DDESCRIPTIONS=100&EN        TREZ_QUERY=(none)&EXPECT=1000&FORMAT_OBJECT=Alignment&FORMAT_TY        PE=HTML&NCBI_GI=on&PAGE=Nucleotides&PROGRAM=blastn&SERVICE=plain&S ET_DEFAULTS.x=29&SET_DEFAULTS.y=6&SHOW_OVERVIEW=on&WORD_SIZE= 7&END_OF_HTTPGET=Yes&SHOW_LINKOUT=yes at the World Wide Web). The method is particularly useful in determining whether a crossover sequence in a chimeric protein, such as, for example, a chimeric monomer domain, is likely to cause an immunogenic event. If the crossover sequence corresponds to a portion of a sequence found in the database of human protein sequences, it is believed that the crossover sequence is less likely to cause an immunogenic event. An example of the comparison step is depicted in Figure 15, which shows a comparison of two candidate protein sequences to human sequences from a database. The horizontal lines indicate where the human protein sequences from the database are identical to the candidate protein sequence.

Human chimeric domain libraries prepared in accordance to the methods of the present invention can be screened for potential immunogenicity, in addition to binding affinity. Furthermore, information pertaining to portions of human protein sequences from the database can be used to design a protein library of human-like chimeric proteins. Such library can be generated by using information pertaining to "crossover sequences" that exist in naturally occurring human proteins. The term "crossover sequence" refers herein to a sequence that is found in its entirety in at least one naturally occurring human protein, in which portions of the sequence are found in two or more naturally occurring proteins. Thus, recombination of the latter two or more naturally occurring proteins would generate a chimeric protein in which the chimeric portion of the sequence actually corresponds to a sequence found in another naturally occurring protein. The crossover sequence contains a chimeric junction of two consecutive amino acid residue positions in which the first amino acid position is occupied by an amino acid residue identical in type and position found in a first and second naturally occurring human protein sequence, but not a third naturally occurring human protein sequence. The second amino acid position is occupied by an amino acid residue identical in type and position found in a second and third naturally occurring human protein sequence, but not the first naturally occurring human protein sequence. In other words, the "second" naturally occurring human protein sequence corresponds to the naturally occurring human protein in which the crossover sequence appears in its entirety, as described above.

In accordance with the present invention, a library of human-like chimeric proteins is generated by: identifying human protein sequences from a database that correspond to proteins from the same family of proteins; aligning the human protein sequences from the same family of proteins to a reference protein sequence; identifying a set of subsequences derived from different human protein sequences of the same family, wherein each subsequence shares a region of identity with at least one other subsequence derived from a different naturally occurring human protein sequence; identifying a chimeric junction from a first, a second, and a third subsequence, wherein each subsequence is derived from a different naturally occurring human protein sequence, and wherein the chimeric junction comprises two consecutive amino acid residue positions in which the first amino acid position is occupied by an amino acid residue common to the first and second naturally occurring human protein sequence, but not the third naturally occurring human protein sequence, and the second amino acid position is occupied by an amino acid residue common to the second and third naturally occurring human protein sequence, and generating human-like chimeric protein molecules each corresponding in sequence to two or more subsequences from the set of subsequences, and each comprising one of more of the identified chimeric junctions.

Thus, for example, if the first naturally occurring human protein sequence is, A-B-C, and the second is, B-C-D-E, and the third is, D-E-F, then the chimeric junction is C-D. Alternatively, if the first naturally occurring human protein sequence is D-E-F-G, and the second is B-C-D-E-F, and the third is A-B-C-D, then the chimeric junction is D-E. Human-like chimeric protein molecules can be generated in a variety of ways. For example, oligonucleotides comprising sequences encoding the chimeric junctions can be recombined with oligonucleotides corresponding in sequence to two or more subsequences from the above-described set of subsequences to generate a human-like chimeric protein, and libraries thereof. The reference sequence used to align the naturally occurring human proteins is a sequence from the same family of naturally occurring human proteins, or a chimera or other variant of proteins in the family.

Nucleic acids encoding fragments of naturally-occurring monomer domains and/or immuno-domains can also be mixed and/or recombined (e.g., by using chemically or enzymatically-produced fragments) to generate full-length, modified monomer domains and/or immuno-domains. The fragments and the monomer domain can also be recombined by manipulating nucleic acids encoding domains or fragments thereof. For example, ligating a nucleic acid construct encoding fragments of the monomer domain can be used to generate an altered monomer domain.

Altered monomer domains can also be generated by providing a collection of synthetic oligonucleotides (e.g., overlapping oligonucleotides) encoding conserved, random, pseudorandom, or a defined sequence of peptide sequences that are then inserted by ligation into a predetermined site in a polynucleotide encoding a monomer domain. Similarly, the sequence diversity of one or more monomer domains can be expanded by mutating the monomer domain(s) with site-directed mutagenesis, random mutation, pseudorandom mutation, defined kernal mutation, codon-based mutation, and the like. The resultant nucleic acid molecules can be propagated in a host for cloning and amplification. In some embodiments, the nucleic acids are shuffled.

The present invention also provides a method for recombining a plurality of nucleic acids encoding monomer domains and screening the resulting library for monomer domains that bind to the desired ligand or mixture of ligands or the like. Selected monomer domain nucleic acids can also be back-crossed by shuffling with polynucleotide sequences encoding neutral sequences (i.e., having insubstantial functional effect on binding), such as for example, by back-crossing with a wild-type or naturally-occurring sequence substantially identical to a selected sequence to produce native-like functional monomer domains. Generally, during back-crossing, subsequent selection is applied to retain the property, e.g., binding to the ligand.

In some embodiments, the monomer library is prepared by shuffling. In such a case, monomer domains are isolated and shuffled to combinatorially recombine the nucleic acid sequences that encode the monomer domains (recombination can occur between or within monomer domains, or both). The first step involves identifying a monomer domain having the desired property, e.g., affinity for a certain ligand. While maintaining the conserved amino acids during the recombination, the nucleic acid sequences encoding the monomer domains can be recombined, or recombined and joined into multimers.

Selection of monomer domains and/or immuno-domains from a library of domains can be accomplished by a variety of procedures. For example, one method of identifying monomer domains and/or immuno-domains which have a desired property involves translating a plurality of nucleic acids, where each nucleic acid encodes a monomer domain and/or immuno-domain, screening the polypeptides encoded by the plurality of nucleic acids, and identifying those monomer domains and/or immuno-domains that, e.g., bind to a desired ligand or mixture of ligands, thereby producing a selected monomer domain and/or immuno-domain. The monomer domains and/or immuno-domains expressed by each of the nucleic acids can be tested for their ability to bind to the ligand by methods known in the art (i.e. panning, affinity chromatography, FACS analysis).

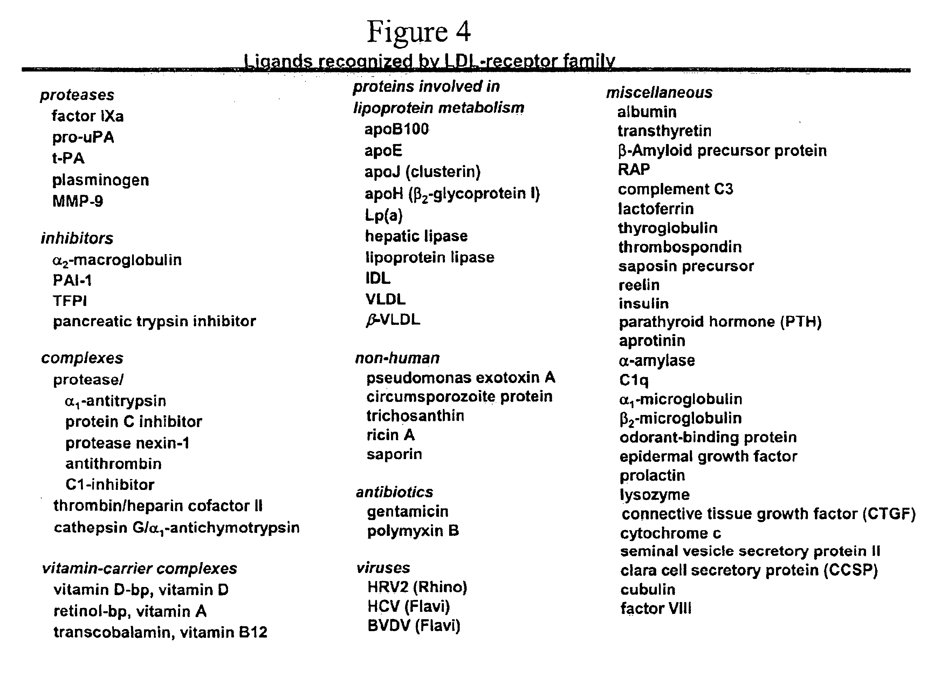

As mentioned above, selection of monomer domains and/or immuno-domains can be based on binding to a ligand such as a target protein or other target molecule (e.g., lipid, carbohydrate, nucleic acid and the like). Other molecules can optionally be included in the methods along with the target, e.g., ions such as $Ca^{+2}$. The ligand can be a known ligand, e.g., a ligand known to bind one of the plurality of monomer domains, or e.g., the desired ligand can be an unknown monomer domain ligand. *See, e.g.*, Figure 4, which illustrates some of the ligands that bind to the A-domain. Other selections of monomer domains and/or immuno-domains can be based, e.g., on inhibiting or enhancing a specific function of a target protein or an activity. Target protein activity can include, e.g., endocytosis or internalization, induction of second messenger system, up-regulation or down-regulation of a gene, binding to an extracellular matrix, release of a molecule(s), or a change in conformation. In this case, the ligand docs not need to be known. The selection can also include using high-throughput assays.

When a monomer domain and/or immuno-domain is selected based on its ability to bind to a ligand, the selection basis can include selection based on a slow dissociation rate, which is usually predictive of high affinity. The valency of the ligand can also be varied to control the average binding affinity of selected monomer domains and/or immuno-domains. The ligand can be bound to a surface or substrate at varying densities, such as by including a competitor compound, by dilution, or by other method known to those in the art. High density (valency) of predetermined ligand can be used to enrich for monomer domains that have relatively low affinity, whereas a low density (valency) can preferentially enrich for higher affinity monomer domains.

A variety of reporting display vectors or systems can be used to express nucleic acids encoding the monomer domains immuno-domains and/or multimers of the present invention and to test for a desired activity. For example, a phage display system is a system in which monomer domains are expressed as fusion proteins on the phage surface (Pharmacia, Milwaukee Wis.). Phage display can involve the presentation of a polypeptide sequence encoding monomer domains and/or immuno-domains on the surface of a filamentous bacteriophage, typically as a fusion with a bacteriophage coat protein.

Generally in these methods, each phage particle or cell serves as an individual library member displaying a single species of displayed polypeptide in addition to the natural phage or cell protein sequences. The plurality of nucleic acids are cloned into the phage DNA at a site which results in the transcription of a fusion protein, a portion of which is encoded by the plurality of the nucleic acids. The phage containing a nucleic acid molecule undergoes replication and transcription in the cell. The leader sequence of the fusion protein directs the transport of the fusion protein to the tip of the phage particle. Thus, the fusion protein that is partially encoded by the nucleic acid is displayed on the phage particle for detection and selection by the methods described above and below. For example, the phage library can be incubated with a predetermined (desired) ligand, so that phage particles which present a fusion protein sequence that binds to the ligand can be differentially partitioned from those that do not present polypeptide sequences that bind to the predetermined ligand. For example, the separation can be provided by immobilizing the predetermined ligand. The phage particles (i.e., library members) which are bound to the immobilized ligand are then recovered and replicated to amplify the selected phage subpopulation for a subsequent round of affinity enrichment and phage replication. After several rounds of affinity enrichment and phage replication, the phage library members that are thus selected are isolated and the nucleotide sequence encoding the displayed polypeptide sequence is determined, thereby identifying the sequence(s) of polypeptides that bind to the predetermined ligand. Such methods are further described in PCT patent publication Nos. 91/17271, 91/18980, and 91/19818 and 93/08278.

Examples of other display systems include ribosome displays, a nucleotide-linked display (*see, e.g.,* U.S. Patent Nos. 6,281,344; 6,194,550, 6,207,446, 6,214,553, and 6,258,558), polysome display, cell surface displays and the like. The cell surface displays include a variety of cells, e.g., E. coli, yeast and/or mammalian cells. When a cell is used as a display, the nucleic acids, e.g., obtained by PCR amplification followed by digestion, are introduced into the cell and translated. Optionally, polypeptides encoding the monomer domains or the multimers of the present invention can be introduced, e.g., by injection, into the cell.

The invention also includes compositions that are produced by methods of the the present invention. For example, the present invention includes monomer domains selected or identified from a library and/or libraries comprising monomer domains produced by the methods of the present invention.

The present invention also provides libraries of monomer domains, immuno-domains and libraries of nucleic acids that encode monomer domains and/or immuno-domains. The libraries can include, e.g., about 100, 250, 500 or more nucleic acids encoding monomer domains and/or immuno-domains, or the library can include, e.g., about 100, 250, 500 or more polypeptides that encode monomer domains and/or immuno-domains. Libraries can include monomer domains containing the same cysteine frame, e.g., A-domains or EGF-like domains.

In some embodiments, variants are generated by recombining two or more different sequences from the same family of monomer domains and/or immuno-domains (e.g., the LDL receptor class A domain). Alternatively, two or more different monomer domains and/or immuno-domains from different families can be combined to form a multimer. In some embodiments, the multimers are formed from monomers or monomer variants of at least one of the following family classes: an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an Immunoglobulin-like domain, a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain, a Laminin-type EGF-like domain, a C2 domain and derivatives thereof. In another embodiment, the monomer domain and the different monomer domain can include one or more domains found in the Pfam database and/or the SMART database. Libraries produced by the methods above, one or more cell(s) comprising one or more members of the library, and one or more displays comprising one or more members of the library are also included in the present invention.

Optionally, a data set of nucleic acid character strings encoding monomer domains can be generated e.g., by mixing a first character string encoding a monomer domain, with one or more character string encoding a different monomer domain, thereby producing a data set of nucleic acids character strings encoding monomer domains, including those described herein. In another embodiment, the monomer domain and the different monomer domain can include one or more domains found in the Pfam database and/or the SMART database. The methods can further comprise inserting the first character string encoding the monomer domain and the one or more second character string encoding the different monomer domain in a computer and generating a multimer character string(s) or library(s), thereof in the computer.

The libraries can be screened for a desired property such as binding of a desired ligand or mixture of ligands. For example, members of the library of monomer domains can be displayed and prescreened for binding to a known or unknown ligand or a mixture of ligands. The monomer domain sequences can then be mutagenized(e.g., recombined, chemically altered, etc.) or otherwise altered and the new monomer domains can be screened again for binding to the ligand or the mixture of ligands with an improved affinity. The selected monomer domains can be combined or joined to form multimers, which can then be screened for an improved affinity or avidity or altered specificity for the ligand or the mixture of ligands. Altered specificity can mean that the specificity is broadened, e.g., binding of multiple related viruses, or optionally, altered specificity can mean that the specificity is narrowed, e.g., binding within a specific region of a ligand. Those of skill in the art will recognize that there are a number of methods available to calculate avidity. *See, e.g.,* Mammen et al., Angew Chem Int. Ed. 37:2754-2794 (1998); Muller et al., Anal. Biochem. 261:149-158 (1998).

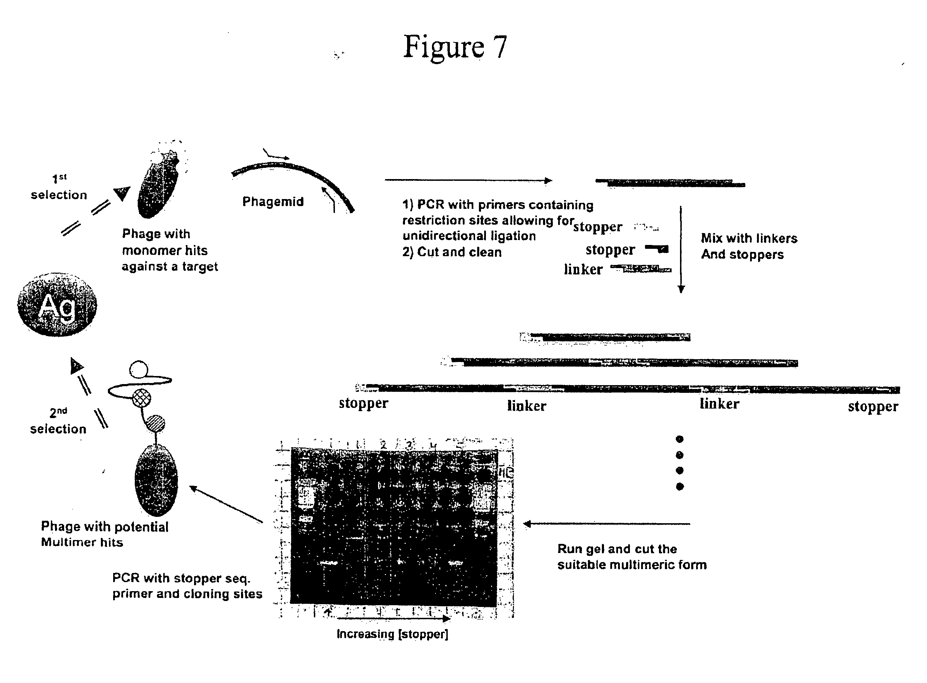

Those of skill in the art will recognize that the steps of generating variation and screening for a desired property can be repeated (i.e., performed recursively) to optimize results. For example, in a phage display library or other like format, a first screening of a library can be performed at relatively lower stringency, thereby selected as many particles associated with a target molecule as possible. The selected particles can then be isolated and the polynucleotides encoding the monomer or multimer can be isolated from the particles. Additional variations can then be generated from these sequences and subsequently screened at higher affinity. Figure 7 illustrates a generic cycle of selection and generation of variation.

Compositions of nucleic acids and polypeptides are included in the present invention. For example, the present disclosure provides a plurality of different nucleic acids wherein each nucleic acid encodes at least one monomer domain or immuno-domain. In some embodiments, at least one monomer domain is selected from the group consisting of: an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an Immunoglobulin-like domain, a C-type lectin domain, a MAM domain, a von Willebrand factor type A domain, a Somatomcdin B domain, a WAP-type four disulfide core domain, a F5/8 type C domain, a Hemopexin domain, an SH2 domain, an SH3 domain, a Laminin-type EGF-like domain, a C2 domain and variants of one or more thereof. Suitable monomer domains also include those listed in the Pfam database and/or the SMART database.

The present invention also provides recombinant nucleic acids encoding one or more polypeptides comprising a plurality of monomer domains and/or immuno-domains, which monomer domains are altered in order or sequence as compared to a naturally occuring polypeptide. For example, the naturally occuring polypeptide can be selected from the group consisting of: an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an Immunoglobulin-like domain, a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain, a Laminin-type EGF-like domain, a C2 domain and variants of one or more thereof. In another embodiment, the naturally occuring polypeptide encodes a monomer domain found in the Pfam database and/or the SMART database.

All the compositions of the present invention, including the compositions produced by the methods of the present invention, e.g., monomer domains and/or immuno-domains, as well as multimers and libraries thereof can be optionally bound to a matrix of an affinity material. Examples of affinity material include beads, a column, a solid support, a microarray, other pools of reagent-supports, and the like.

### 2. Multimers (also called Recombinant Mosaic Proteins or Combinatorial Mosaic Proteins)

Methods for generating multimers are a feature of the present invention. Multimers comprise at least two monomer domains and/or immuno-domains. For example, multimers of the invention can comprise from 2 to about 10 monomer domains and/or immuno-domains, from 2 and about 8 monomer domains and/or immuno-domains, from about 3 and about 10 monomer domains and/or immuno-domains, about 7 monomer domains and/or immuno-domains, about 6 monomer domains and/or immuno-domains, about 5 monomer domains and/or immuno-domains, or about 4 monomer domains and/or immuno-domains. In some embodiments, the multimer comprises at least 3 monomer domains and/or immuno-domains. Typically, the monomer domains have been pre-selected for binding to the target molecule of interest.

In some embodiments, each monomer domain specifically binds to one target molecule. In some of these embodiments, each monomer binds to a different position (analogous to an epitope) on a target molecule. Multiple monomer domains and/or immuno-domains that bind to the same target molecule results in an avidity effect resulting in improved avidity of the multimer for the target molecule compared to each individual monomer. In some embodiments, the multimer has an avidity of at least about 1.5, 2, 3, 4, 5, 10, 20, 50 or 100 times the avidity of a monomer domain alone.

In another embodiment, the multimer comprises monomer domains with specificities for different target molecules. For example, multimers of such diverse monomer domains can specifically bind different components of a viral replication system or different serotypes of a virus. In some embodiments, at least one monomer domain binds to a toxin and at least one monomer domain binds to a cell surface molecule, thereby acting as a mechanism to target the toxin. In some embodiments, at least two monomer domains and/or immuno-domains of the multimer bind to different target molecules in a target cell or tissue. Similarly, therapeutic molecules can be targeted to the cell or tissue by binding a therapeutic agent to a monomer of the multimer that also contains other monomer domains and/or immuno-domains having cell or tissue binding specificity.

Multimers can comprise a variety of combinations of monomer domains. For example, in a single multimer, the selected monomer domains can be the same or identical, optionally, different or non-identical. In addition, the selected monomer domains can comprise various different monomer domains from the same monomer domain family, or various monomer domains from different domain families, or optionally, a combination of both.

Multimers that are generated in the practice of the present invention may be any of the following:

(1) A homo-multimer (a multimer of the same domain, i.e., A1-A1-A1-A1);

(2) A hetero-multimer of different domains of the same domain class, e.g., A1-A2-A3-A4. For example, hetero-multimer include multimers where A1, A2, A3 and A4 are different non-naturally occurring variants of a particular LDL-receptor class A domains, or where some of A1, A2, A3, and A4 are naturally-occurring variants of a LDL-receptor class A domain (*see, e.g.,* Figure 10).

(3) A hetero-multimer of domains from different monomer domain classes, e.g., A1-B2-A2-B1. For example, where A1 and A2 are two different monomer domains (either naturally occurring or non-naturally-occurring) from LDL-receptor class A, and B1 and B2 are two different monomer domains (either naturally occurring or non-naturally occurring) from class EGF-like domain).

Multimer libraries employed in the practice of the present invention may contain homo-multimers, hetero-multimers of different monomer domains (natural or non-natural) of the same monomer class, or hetero-multimers of monomer domains (natural or non-natural) from different monomer classes, or combinations thereof. Exemplary heteromultimers comprising immuno-domains include dimers of, e.g., minibodies, single domain antibodies and Fabs, wherein the dimers are linked by a covalent linker. Other exemplary multimers include, e.g., trimers and higher level (e.g., tetramers) multimers of minibodies, single domain antibodies and Fabs. Yet more exemplary multimers include, e.g., dimers, trimers and higher level multimers of single chain antibody fragments, wherein the single chain antibodies are not link covalently.

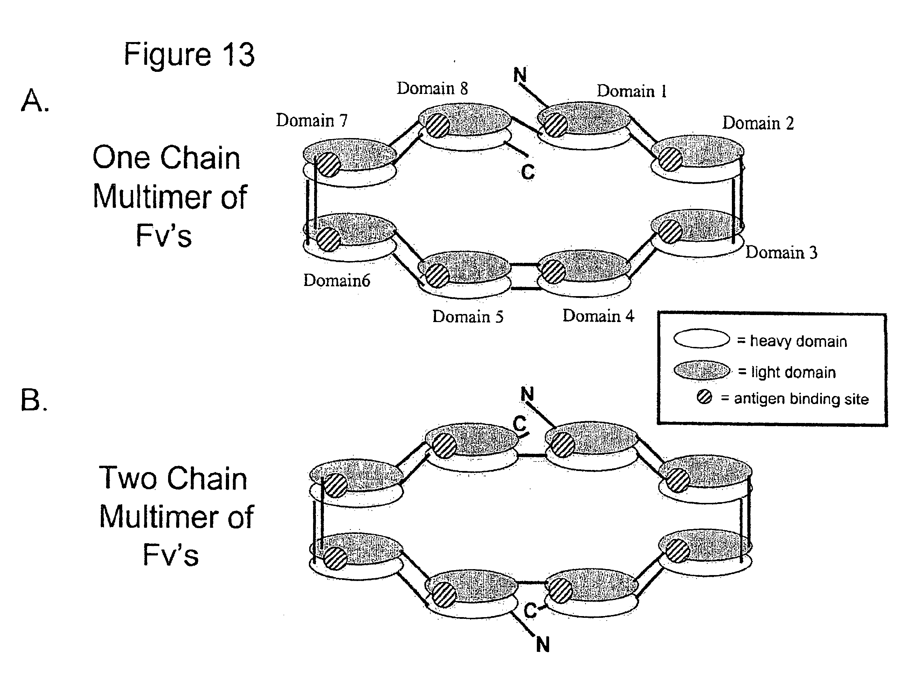

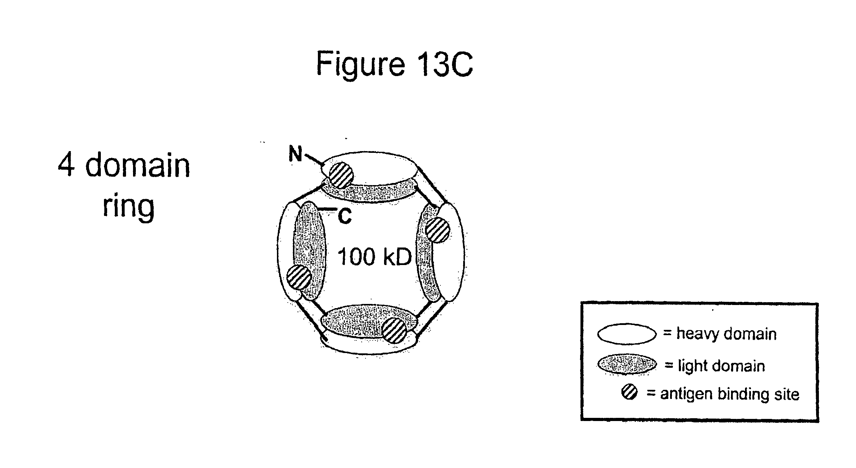

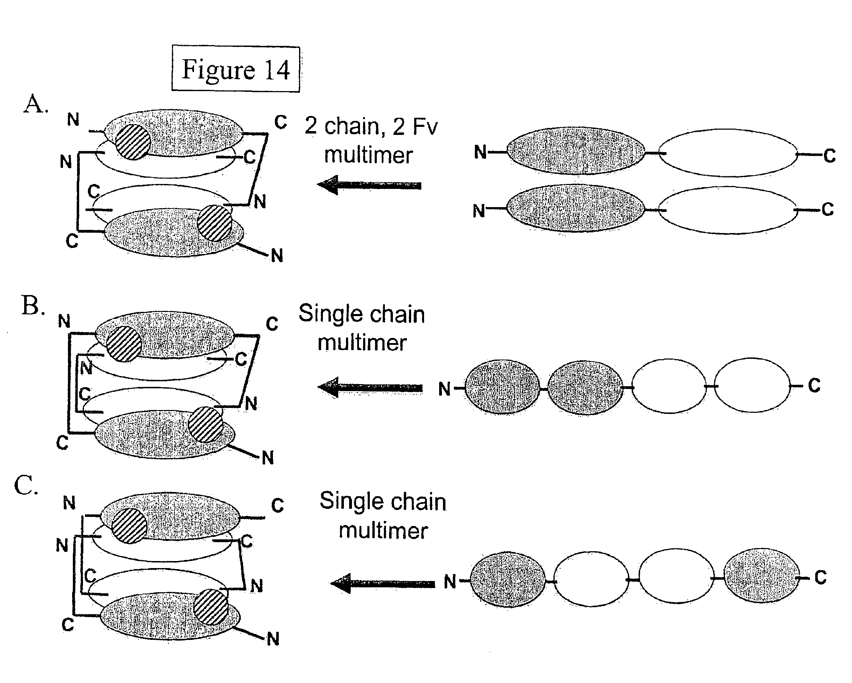

The present disclosure provides multimers of $V_H$ and $V_L$ domains that associate to form multimers of Fvs as depicted in Figure 13 and Figure 14B and C. As used herein, the term "Fv" refers to a non-covalently associated $V_H V_L$ dimer. Such a dimer is depicted, for example, in Figure 13A, where each pair of overlapping dark and white ellipses represents a single Fv. Fv multimers of the present invention do not comprise a light variable domain covalently linked directly to a heavy variable domain from the same Fv. However, Fv multimers of the present invention can comprise a covalent linkage of the light variable domains and heavy variable domains of the same Fv, that are separated by at least one or more domains. For example, exemplary conformations of a multimer are $V_{H1}$-$V_{H2}$-$V_{L1}$-$V_{L2}$, or $V_{H1}$-$V_{L2}$-$V_{L1}$-$V_{H2}$ (where $V_{L\#}$, and $V_{H\#}$ represent the heavy and light variable domains, respectively).

In these and other embodiments, the heavy and light variable domains are aligned such that the corresponding heavy and light variable domains associate to form the corresponding Fv (i.e., $Fv_1 = V_{H1} V_{L1}$, $Fv_2 = V_{H2} V_{L2}$, etc.). Figures 14B and C illustrate such Fv multimers. Those of ordinary skill in the art will readily appreciate that such Fv multimers can comprise additional heavy or light variable domains of an Fv, to form relatively large multimers of, for example, six, eight of more immuno-domains. See, e.g., Figure 13. The Fvs in an Fv multimer of the present invention are not scFvs (i.e., $V_{L1}$, is not covalently linked to $V_{H1}$).

Monomer domains, as described herein, are also readily employed in a immuno-domain-containing heteromultimer (i.e., a multimer that has at least one immuno-domain variant and one monomer domain variant). Thus, multimers of the present invention may have at least one immuno-domain such as a minibody, a single-domain antibody, a single chain variable fragment (ScFv), or a Fab fragment; and at least one monomer domain, such as, for example, an EGF-like domain, a Kringle-domain, a fibronectin type I domain, a fibronectin type II domain, a fibronectin type III domain, a PAN domain, a Gla domain, a SRCR domain, a Kunitz/Bovine pancreatic trypsin Inhibitor domain, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an Immunoglobulin-like domain, a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain, a Laminin-type EGF-like domain, a C2 domain, or variants thereof.

Domains need not be selected before the domains are linked to form multimers. On the other hand, the domains can be selected for the ability to bind to a target molecule before being linked into multimers. Thus, for example, a multimer can comprise two domains that bind to one target molecule and a third domain that binds to a second target molecule.

The selected monomer domains are joined by a linker to form a multimer. For example, a linker is positioned between each separate discrete monomer domain in a multimer. Typically, immuno-domains are also linked to each other or to monomer domains via a linker moiety. Linker moieties that can be readily employed to link immuno-domain variants together are the same as those described for multimers of monomer domain variants. Exemplary linker moieties suitable for joining immuno-domain variants to other domains into multimers are described herein.

Joining the selected monomer domains via a linker can be accomplished using a variety of techniques known in the art. For example, combinatorial assembly of polynucleotides encoding selected monomer domains can be achieved by restriction digestion and re-ligation, by PCR-based, self-priming overlap reactions, or other rcmbinant methods. The linker can be attached to a monomer before the monomer is identified for its ability to bind to a target multimer or after the monomer has been selected for the ability to bind to a target multimer.

The linker can be naturally-occurring, synthetic or a combination of both. For example, the synthetic linker can be a randomized linker, e.g., both in sequence and size. In one aspect, the randomized linker can comprise a fully randomized sequence, or optionally, the randomized linker can be based on natural linker sequences. The linker can comprise, e.g,. a non-polypeptide moiety, a polynucleotide, a polypeptide or the like.

A linker can be rigid, or alternatively, flexible, or a combination of both. Linker flexibility can be a function of the composition of both the linker and the monomer domains that the linker interacts with. The linker joins two selected monomer domain, and maintains the monomer domains as separate discrete monomer domains. The linker can allow the separate discrete monomer domains to cooperate yet maintain separate properties such as multiple separate binding sites for the same ligand in a multimer, or e.g., multiple separate binding sites for different ligands in a multimer. In some cases, a disulfide bridge exists between two linked monomer domains or between a linker and a monomer domain. In some embodiments, the monmer domains and/or linkers comprise metal-binding centers.

Choosing a suitable linker for a specific case where two or more monomer domains (i.e. polypeptide chains) are to be connected may depend on a variety of parameters including, e.g. the nature of the monomer domains, the structure and nature of the target to which the polypeptide multimer should bind and/or the stability of the peptide linker towards proteolysis and oxidation.

The present disclosure provides methods for optimizing the choice of linker once the desired monomer domains/variants have been identified. Generally, libraries of multimers having a composition that is fixed with regard to monomer domain composition, but variable in linker composition and length, can be readily prepared and screened as described above.

Typically, the linker polypeptide may predominantly include amino acid residues selected from the group consisting of Gly, Ser, Ala and Thr. For example, the peptide linker may contain at least 75% (calculated on the basis of the total number of residues present in the peptide linker), such as at least 80%, e.g. at least 85% or at least 90% of amino acid residues selected from the group consisting ofGly, Ser, Ala and Thr. The peptide linker may also consist of Gly, Ser, Ala and/or Thr residues only. The linker polypeptide should have a length, which is adequate to link two monomer domains in such a way that they assume the correct conformation relative to one another so that they retain the desired activity, for example as antagonists of a given receptor.

A suitable length for this purpose is a length of at least one and typically fewer than about 50 amino acid residues, such as 2-25 amino acid residues, 5-20 amino acid residues, 5-15 amino acid residues, 8-12 amino acid residues or 11 residues. Similarly, the polypeptide encoding a linker can range in size, e.g., from about 2 to about 15 amino acids, from about 3 to about 15, from about 4 to about 12, about 10, about 8, or about 6 amino acids. In methods and compositions involving nucleic acids, such as DNA, RNA, or combinations of both, the polynucleotide containing the linker sequence can be, e.g., between about 6 nucleotides and about 45 nucleotides, between about 9 nucleotides and about 45 nucleotides, between about 12 nucleotides and about 36 nucleotides, about 30 nucleotides, about 24 nucleotides, or about 18 nucleotides. Likewise, the amino acid residues selected for inclusion in the linker polypeptide should exhibit properties that do not interfere significantly with the activity or function of the polypeptide multimer. Thus, the peptide linker should on the whole not exhibit a charge which would be inconsistent with the activity or function of the polypeptide multimer, or interfere with internal folding, or form bonds or other interactions with amino acid residues in one or more of the monomer domains which would seriously impede the binding of the polypeptide multimer to the target in question.

In another embodiment of the invention, the peptide linker is selected from a library where the amino acid residues in the peptide linker are randomized for a specific set of monomer domains in a particular polypeptide multimer. A flexible linker could be used to find suitable combinations of monomer domains, which is then optimized using this random library of variable linkers to obtain linkers with optimal length and geometry. The optimal linkers may contain the minimal number of amino acid residues of the right type that participate in the binding to the target and restrict the movement of the monomer domains relative to each other in the polypeptide multimer when not bound to the target.

The use of naturally occurring as well as artificial peptide linkers to connect polypeptides into novel linked fusion polypeptides is well known in the literature (Hallewell et al. (1989), J. Biol. Chem. 264, 5260-5268; Alfthan et al. (1995), Protein Eng. 8, 725-731; Robinson & Sauer (1996), Biochemistry 35, 109-116; Khandekar et al. (1997), J. Biol. Chem. 272, 32190-32197; Fares et al. (1998), Endocrinology 139, 2459-2464; Smallshaw et al. (1999), Protein Eng. 12, 623-630; US 5,856,456).

One example where the use of peptide linkers is widespread is for production of single-chain antibodies where the variable regions of a light chain ($V_L$) and a heavy chain ($V_H$) are joined through an artificial linker, and a large number of publications exist within this particular field. A widely used peptide linker is a 15mer consisting of three repeats of a Gly-Gly-Gly-Gly-Ser (SEQ ID NO: 240) amino acid sequence (($Gly_4Ser)_3$). Other linkers have been used, and phage display technology, as well as, selective infective phage technology has been used to diversify and select appropriate linker sequences (Tang et al. (1996), J. Biol. Chem. 271, 15682-15686; Hennecke et al. (1998), Protein Eng. 11, 405-410). Peptide linkers have been used to connect individual chains in hetero- and homo-dimeric proteins such as the T-cell receptor, the lambda Cro repressor, the P22 phage Arc repressor, IL-12, TSH, FSH, IL-5, and interferon-$\gamma$. Peptide linkers have also been used to create fusion polypeptides. Various linkers have been used and in the case of the Arc repressor phage display has been used to optimize the linker length and composition for increased stability of the single-chain protein (Robinson and Sauer (1998), Proc. Natl. Acad. Sci. USA 95, 5929-5934).

Another type of linker is an intein, i.e. a peptide stretch which is expressed with the single-chain polypeptide, but removed post-translationally by protein splicing. The use of inteins is reviewed by F.S. Gimble in Chemistry and Biology, 1998, Vol 5, No. 10 pp. 251-256.

Still another way of obtaining a suitable linker is by optimizing a simple linker, e.g. ($Gly_4Ser)_n$ (SEQ ID NO: 240), through random mutagenesis.

As mentioned above, it is generally preferred that the peptide linker possess at least some flexibility. Accordingly, in some embodiments, the peptide linker contains 1-25 glycine residues, 5-20 glycine residues, 5-15 glycine residues or 8-12 glycine residues. The peptide linker will typically contain at least 50% glycine residues, such as at least 75% glycine residues. In some embodiments of the invention, the peptide linker comprises glycine residues only.

The peptide linker may, in addition to the glycine residues, comprise other residues, in particular residues selected from the group consisting of Ser, Ala and Thr, in particular Ser. Thus, one example of a specific peptide linker includes a peptide linker having the amino acid sequence $Gly_x$-Xaa-$Gly_y$-Xaa-$Gly_2$ (SEQ ID NO: 203), wherein each Xaa is independently selected from the group consisting Ala, Val, Leu, Ile, Met, Phe, Trp, Pro, Gly, Ser, Thr, Cys, Tyr, Asn, Gln, Lys, Arg, His, Asp and Glu, and wherein x, y and z are each integers in the range from 1-5. In some embodiments, each Xaa is independently selected from the group consisting of Ser, Ala and Thr, in particular Ser. More particularly, the peptide linker has the amino acid sequence Gly-Gly-Gly-Xaa-Gly-Gly-Gly-Xaa-Gly-Gly-Gly (SEQ ID NO: 204), wherein each Xaa is independently selected from the group consisting Ala, Val, Leu, Ile, Met, Phe, Trp, Pro, Gly, Ser, Thr, Cys, Tyr, Asn, Gln, Lys, Arg, His, Asp and Glu. In some embodiments, each Xaa is independently selected from the group consisting of Ser, Ala and Thr, in particular Ser.

In some cases it may be desirable or necessary to provide some rigidity into the peptide linker. This may be accomplished by including proline residues in the amino acid sequence of the peptide linker. Thus, in another embodiment of the invention, the peptide linker comprises at least one proline residue in the amino acid sequence of the peptide linker. For example, the peptide linker has an amino acid sequence, wherein at least 25%, such as at least 50%, e.g. at least 75%, of the amino acid residues are proline residues. In one particular embodiment of the invention, the peptide linker comprises proline residues only.

In some embodiments of the invention, the peptide linker is modified in such a way that an amino acid residue comprising an attachment group for a non-polypeptide moiety is introduced. Examples of such amino acid residues may be a cysteine residue (to which the non-polypeptide moiety is then subsequently attached) or the amino acid sequence may include *an in vivo* N-glycosylation site (thereby attaching a sugar moiety (*in vivo*) to the peptide linker). An additional option is to genetically incorporate non-natural amino acids using evolved tRNAs and tRNA synthetases (*see, e.g.,* U.S. Patent Application Publication 2003/0082575) into the monomer domains or linkers. For example, insertion of keto-tyrosine allows for site-specific coupling to expressed monomer domains or multimers.

In some embodiments of the invention, the peptide linker comprises at least one cysteine residue, such as one cysteine residue. Thus, in some embodiments of the invention the peptide linker comprises amino acid residues selected from the group consisting of Gly, Ser, Ala, Thr and Cys. In some embodiments, such a peptide linker comprises one cysteine residue only.

In a further embodiment, the peptide linker comprises glycine residues and cysteine residue, such as glycine residues and cysteine residues only. Typically, only one cysteine residue will be included per peptide linker. Thus, one example of a specific peptide linker comprising a cysteine residue, includes a peptide linker having the amino acid sequence $Gly_n$-Cys-$Gly_m$ (SEQ ID NO: 205), wherein n and m are each integers from 1-12, e.g., from 3-9, from 4-8, or from 4-7. More particularly, the peptide linker may have the amino acid sequence GGGGG-C-GGGGG (SEQ ID NO: 206).

This approach (i.e. introduction of an amino acid residue comprising an attachment group for a non-polypeptide moiety) may also be used for the more rigid proline-containing linkers. Accordingly, the peptide linker may comprise proline and cysteine residues, such as proline and cysteine residues only. An example of a specific proline-containing peptide linker comprising a cysteine residue, includes a peptide linker having the amino acid sequence $Pro_n$-Cys-$Pro_m$ (SEQ ID NO: 207), wherein n and m are each integers from 1-12, preferably from 3-9, such as from 4-8 or from 4-7. More particularly, the peptide linker may have the amino acid sequence PPPPP-C-PPPPP (SEQ ID NO: 208).

In some embodiments, the purpose of introducing an amino acid residue, such as a cysteine residue, comprising an attachment group for a non-polypeptide moiety is to subsequently attach a non-polypeptide moiety to said residue. For example, non-polypeptide moieties can improve the serum half-life of the polypeptide multimer. Thus, the cysteine residue can be covalently attached to a non-polypeptide moiety. Preferred examples of non-polypeptide moieties include polymer molecules, such as PEG or mPEG, in particular mPEG as well as non-polypeptide therapeutic agents.

The skilled person will acknowledge that amino acid residues other than cysteine may be used for attaching a non-polypeptide to the peptide linker. One particular example of such other residue includes coupling the non-polypeptide moiety to a lysine residue.

Another possibility of introducing a site-specific attachment group for a non-polypeptide moiety in the peptide linker is to introduce an *in vivo* N-glycosylation site, such as one in *vivo* N-glycosylation site, in the peptide linker. For example, an *in vivo* N-glycosylation site may be introduced in a peptide linker comprising amino acid residues selected from the group consisting of Gly, Ser, Ala and Thr. It will be understood that in order to ensure that a sugar moiety is in fact attached to said *in vivo* N-glycosylation site, the nucleotide sequence encoding the polypeptide multimer must be inserted in a glycosylating, eukaryotic expression host.

A specific example of a peptide linker comprising an *in vivo* N-glycosylation site is a peptide linker having the amino acid sequence $Gly_n$-Asn-Xaa-Ser/Thr-$Gly_m$ (SEQ ID NO: 209), preferably $Gly_n$-Asn-Xaa-Thr-$Gly_m$ (SEQ ID NO: 210), wherein Xaa is any amino acid residue except proline, and wherein n and m are each integers in the range from 1-8, preferably in the range from 2-5.

Often, the amino acid sequences of all peptide linkers present in the polypeptide multimer will be identical. Nevertheless, in certain embodiments the amino acid sequences of all peptide linkers present in the polypeptide multimer may be different. The latter is believed to be particular relevant in case the polypeptide multimer is a polypeptide tri-mer or tetra-mer and particularly in such cases where an amino acid residue comprising an attachment group for a non-polypeptide moiety is included in the peptide linker.

Quite often, it will be desirable or necessary to attach only a few, typically only one, non-polypeptide moieties/ moiety (such as mPEG, a sugar moiety or a non-polypeptide therapeutic agent) to the polypeptide multimer in order to achieve the desired effect, such as prolonged serum-half life. Evidently, in case of a polypeptide tri-mer, which will contain two peptide linkers, only one peptide linker is typically required to be modified, e.g. by introduction of a cysteine residue, whereas modification of the other peptide linker will typically not be necessary not. In this case all (both) peptide linkers of the polypeptide multimer (tri-mer) are different.

Accordingly, in a further embodiment of the invention, the amino acid sequences of all peptide linkers present in the polypeptide multimer are identical except for one, two or three peptide linkers, such as except for one or two peptide linkers, in particular except for one peptide linker, which has/have an amino acid sequence comprising an amino acid residue comprising an attachment group for a non-polypeptide moiety. Preferred examples of such amino acid residues include cysteine residues of *in vivo* N-glycosylation sites.

A linker can be a native or synthetic linker sequence. An exemplary native linker includes, e.g., the sequence between the last cysteine of a first LDL receptor A domain and the first cysteine of a second LDL receptor A domain can be used as a linker sequence. Analysis of various A domain linkages reveals that native-linkers range from at least 3 amino acids to fewer than 20 amino acids, e.g., 4, 5, 6, 7, 8, 9, 10, 11, 12, 13, 14, 15, 16, 17, or 18 amino acids long. However, those of skill in the art will recognize that longer or shorter linker sequences can be used. An exemplary A domain linker sequence is depicted in Figure 8. In some embodiments, the linker is a 6-mer of the following sequence $A_1A_2A_3A_4A_5A_6$(EQ ID NO: 244), wherein $A_1$ is selected from the amino acids A, P, T, Q, E and K; $A_2$ and $A_3$ are any amino acid except C, F, Y, W, or M; $A_4$ is selected from the amino acids S, G and R; $A_5$ is selected from the amino acids H, P, and R; and $A_6$ is the amino acid, T.

Methods for generating multimers from monomer domains and/or immuno-domains can include joining the selected domains with at least one linker to generate at least one multimer, e.g., the multimer can comprise at least two of the monomer domains and/or immuno-domains and the linker. The multimer(s) is then screened for an improved avidity or affinity or altered specificity for the desired ligand or mixture of ligands as compared to the selected monomer domains. A composition of the multimer produced by the method is included in the present invention.

In other methods, the selected multimer domains are joined with at least one linker to generate at least two multimers, wherein the two multimers comprise two or more of the selected monomer domains and the linker. The two or more multimers are screened for an improved avidity or affinity or altered specificity for the desired ligand or mixture of ligands as compared to the selected monomer domains. Compositions of two or more multimers produced by the above method are also features of the invention.

Typically, multimers of the present invention are a single discrete polypeptide. Multimers of partial linker-domain-partial linker moieties are an association of multiple polypeptides, each corresponding to a partial linker-domain-partial linker moiety.

When multimers capable of binding relatively large targets are desired, they can be generated by a "walking" selection method. This method is carried out by providing a library of monomer domains and screening the library of monomer domains for affinity to a first target molecule. Once at least one monomer that binds to the target is identified, that monomer is covalently linked to a new library or each remaining member of the original library of monomer domains. This new library of multimers (dimers) is then screened for multimers that bind to the target with an increased affinity, and a multimer that binds to the target with an increased affinity can be identified. The "walking" monomer selection method provides a way to assemble a multimer that is composed of monomers that can act additively or even synergistically with each other given the restraints of linker length. This walking technique is very useful when selecting for and assembling multimers that are able to bind large target proteins with high affinity. The walking method can be repeated to add more monomers thereby resulting in a multimer comprising 2, 3, 4, 5, 6, 7, 8 or more monomers linked together.

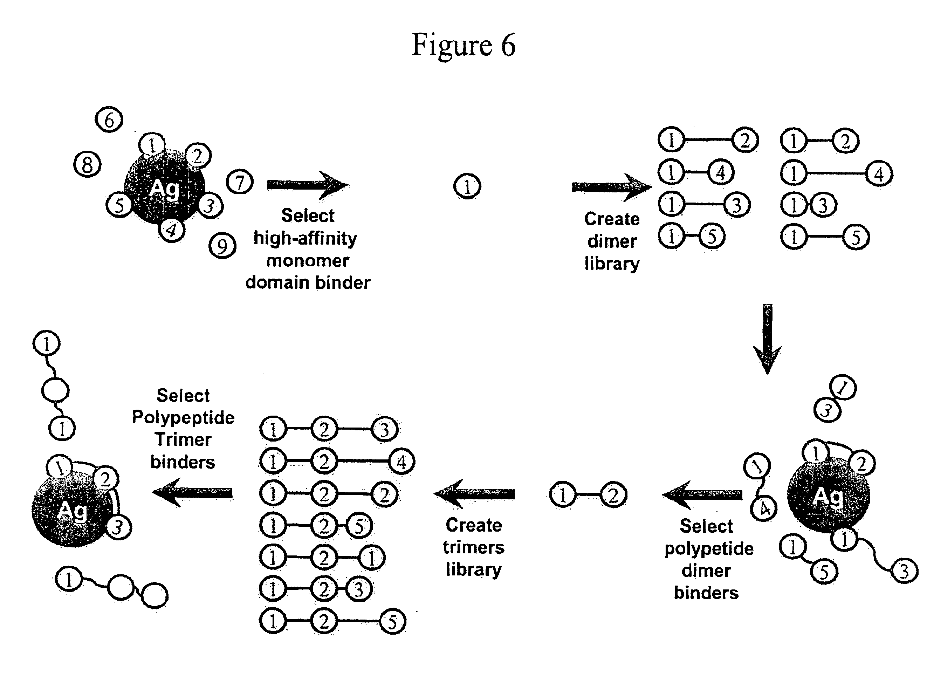

In some embodiments, the selected multimer comprises more than two domains. Such multimers can be generated in a step fashion, e.g., where the addition of each new domain is tested individually and the effect of the domains is tested in a sequential fashion. *See, e.g.,* Figure 6. In an alternate embodiment, domains are linked to form multimers comprising more than two domains and selected for binding without prior knowledge of how smaller multimers, or alternatively, how each domain, bind.

The methods of the present invention also include methods of evolving multimers. The methods can comprise, e.g., any or all of the following steps: providing a plurality of different nucleic acids, where each nucleic acid encoding a monomer domain; translating the plurality of different nucleic acids, which provides a plurality of different monomer domains; screening the plurality of different monomer domains for binding of the desired ligand or mixture of ligands; identifying members of the plurality of different monomer domains that bind the desired ligand or mixture of ligands, which provides selected monomer domains; joining the selected monomer domains with at least one linker to generate at least one multimer, wherein the at least one multimer comprises at least two of the selected monomer domains and the at least one linker; and, screening the at least one multimer for an improved affinity or avidity or altered specificity for the desired ligand or mixture of ligands as compared to the selected monomer domains.

Additional variation can be introduced by inserting linkers of different length and composition between domains. This allows for the selection of optimal linkers between domains. In some embodiments, optimal length and composition of linkers will allow for optimal binding of domains. In some embodiments, the domains with a particular binding affinity (s) are linked via different linkers and optimal linkers are selected in a binding assay. For example, domains are selected for desired binding properties and then formed into a library comprising a variety of linkers. The library can then be screened to identify optimal linkers. Alternatively, multimer libraries can be formed where the effect of domain or linker on target molecule binding is not known.

Methods of the present invention also include generating one or more selected multimers by providing a plurality of monomer domains and/or immuno-domains. The plurality of monomer domains and/or immuno-domains are screened for binding of a desired ligand or mixture of ligands. Members of the plurality of domains that bind the desired ligand or mixture of ligands are identified, thereby providing domains with a desired affinity. The identified domains are joined with at least one linker to generate the multimers, wherein each multimer comprises at least two of the selected domains and the at least one linker; and, the multimers are screened for an improved affinity or avidity or altered specificity for the desired ligand or mixture of ligands as compared to the selected domains, thereby identifying the one or more selected multimers.

Selection of multimers can be accomplished using a variety of techniques including those mentioned above for identifying monomer domains. Other selection methods include, e.g., a selection based on an improved affinity or avidity or altered specificity for the ligand compared to selected monomer domains. For example, a selection can be based on selective binding to specific cell types, or to a set of related cells or protein types (e.g., different virus scrotypes). Optimization of the property selected for, e.g., avidity of a ligand, can then be achieved by recombining the domains, as well as manipulating amino acid sequence of the individual monomer domains or the linker domain or the nucleotide sequence encoding such domains, as mentioned in the present invention.

One method for identifying multimers can be accomplished by displaying the multimers. As with the monomer domains, the multimers are optionally expressed or displayed on a variety of display systems, e.g., phage display, ribosome display, polysome display, nucleotide-linked display (*see, e.g.,* U.S. Patent Nos. 6,281,344; 6,194,550, 6,207,446, 6,214,553, and 6,258,558) and/or cell surface display, as described above. Cell surface displays can include but are not limited to *E. coli,* yeast or mammalian cells. In addition, display libraries of multimers with multiple binding sites can be panned for avidity or affinity or altered specificity for a ligand or for multiple ligands.

Monomers or multimers can be screened for target binding activity in yeast cells using a two-hybrid screening assay. In this type of screen the monomer or multimer library to be screened is cloned into a vector that directs the formation of a fusion protein between each monomer or multimer of the library and a yeast transcriptional activator fragment (i.e., Gal4). Sequences encoding the "target" protein are cloned into a vector that results in the production of a fusion protein between the target and the remainder of the Gal4 protein (the DNA binding domain). A third plasmid contains a reporter gene downstream of the DNA sequence of the Gal4 binding site. A monomer that can bind to the target protein brings with it the Gal4 activation domain, thus reconstituting a functional Gal4 protein. This functional Gal4 protein bound to the binding site upstream of the reporter gene results in the expression of the reporter gene and selection of the monomer or multimer as a target binding protein. (see Chien et.al. (1991) Proc. Natl. Acad. Sci. (USA) 88:9578; Fields S. and Song O. (1989) Nature 340: 245) Using a two-hybrid system for library screening is further described in U.S. Patent No. 5,811,238 (see also Silver S.C. and Hunt S.W. (1993) Mol. Biol. Rep. 17:155; Durfee et al. (1993) Genes Devel. 7:555; Yang et al. (1992) Science 257:680; Luban et al. (1993) Cell 73:1067; Hardy et al. (1992) Genes Devel. 6:801; Bartel et al. (1993) Biotechniques 14:920; and Vojtek et al. (1993) Cell 74:205), Another useful screening system for carrying out the present invention is the *E.coli*/BccP interactive screening system (Germino et al. (1993) Proc. Nat. Acad. Sci. (U.S.A.) 90:993; Guarente L. (1993) Proc. Nat. Acad. Sci. (U.S.A.) 90:1639).

Other variations include the use of multiple binding compounds, such that monomer domains, multimers or libraries of these molecules can be simultaneously screened for a multiplicity of ligands or compounds that have different binding specificity. Multiple predetermined ligands or compounds can be concomitantly screened in a single library, or sequential screening against a number of monomer domains or multimers. In one variation, multiple ligands or compounds, each encoded on a separate bead (or subset of beads), can be mixed and incubated with monomer domains, multimers or libraries of these molecules under suitable binding conditions. The collection of beads, comprising multiple ligands or compounds, can then be used to isolate, by affinity selection, selected monomer domains, selected multimers or library members. Generally, subsequent affinity screening rounds can include the same mixture of beads, subsets thereof, or beads containing only one or two individual ligands or compounds. This approach affords efficient screening, and is compatible with laboratory automation, batch processing, and high throughput screening methods.

In another embodiment, multimers can be simultaneously screened for the ability to bind multiple ligands, wherein each ligand comprises a different label. For example, each ligand can be labeled with a different fluorescent label, contacted simultaneously with a multimer or multimer library. Multimers with the desired affinity are then identified (e.g., by FACS sorting) based on the presence of the labels linked to the desired labels.

Libraries of either monomer domains or multimers (referred in the following discussion for convenience as "affinity agents") can be screened (i.e., panned) simultaneously against multiple ligands in a number of different formats. For example, multiple ligands can be screened in a simple mixture, in an array, displayed on a cell or tissue (e.g., a cell or tissue provides numerous molecules that can be bound by the monomer domains or multimers of the invention), and/or immobilized. The libraries of affinity agents can optionally be displayed on yeast or phage display systems. Similarly, if desired, the ligands (e.g., encoded in a cDNA library) can be displayed in a yeast or phage display system.

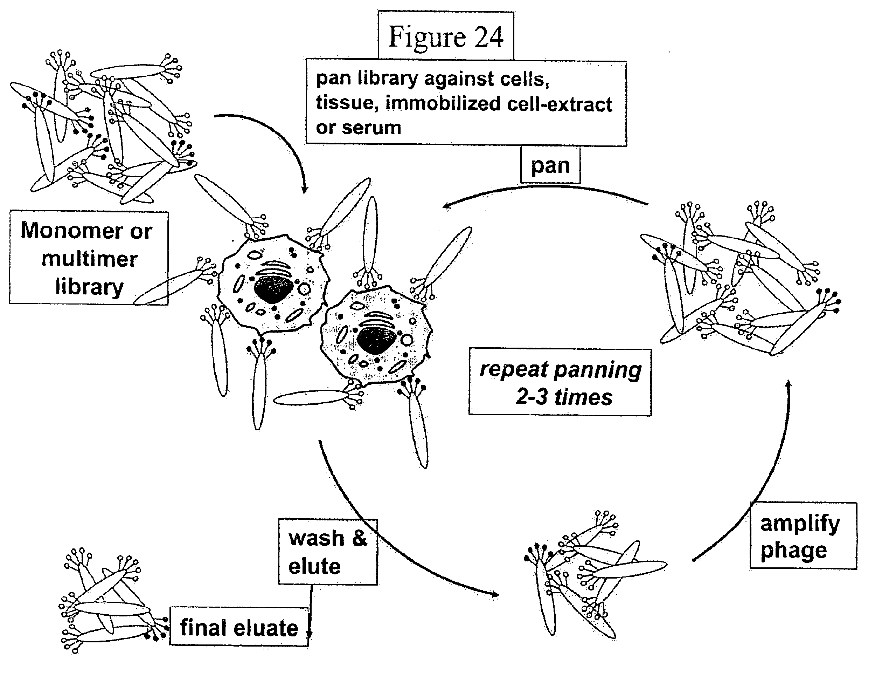

Initially, the affinity agent library is panned against the multiple ligands. Optionally, the resulting "hits" are panned against the ligands one or more times to enrich the resulting population of affinity agents. *See, e.g.,* Figure 24.

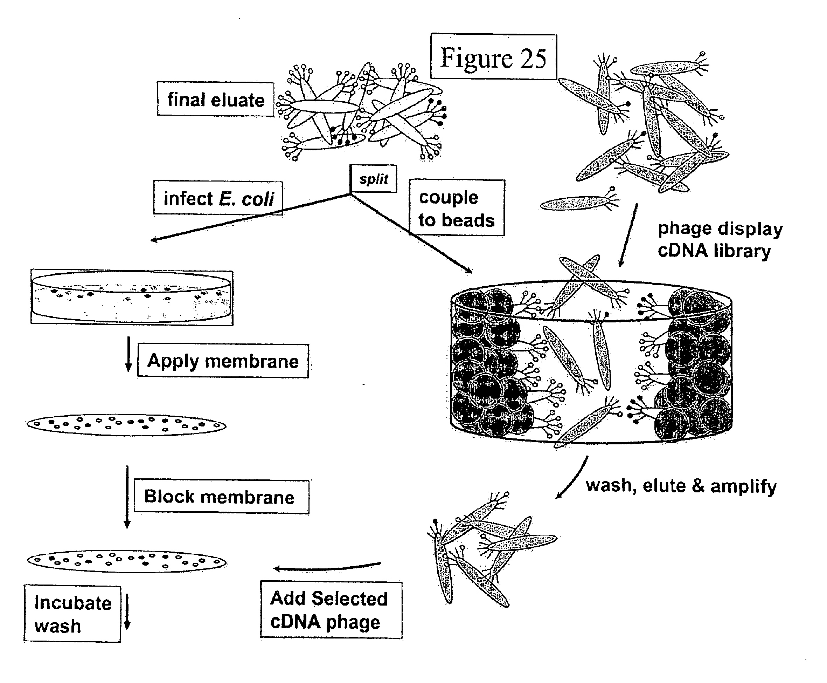

If desired, the identity of the individual affinity agents and/or ligands can be determined. In some embodiments, affinity agents are displayed on phage. Affinity agents identified as binding in the initial screen are divided into a first and second portion. *See, e.g.,* Figure 25. The first portion is infected into bacteria, resulting in either plaques or bacterial colonies, depending on the type of phage used. The expressed phage are immobilized and then probed with ligands displayed in phage selected as described below.

The second portion are coupled to beads or otherwise immobilized and a phage display library containing at least some of the ligands in the original mixture is contacted to the immobilized second portion. Those phage that bind to the second portion are subsequently eluted and contacted to the immobilized phage described in the paragraph above. Phage-phage interactions are detected (e.g., using a monoclonal antibody specific for the ligand-expressing phage) and the resulting phage polynucleotides can be isolated.

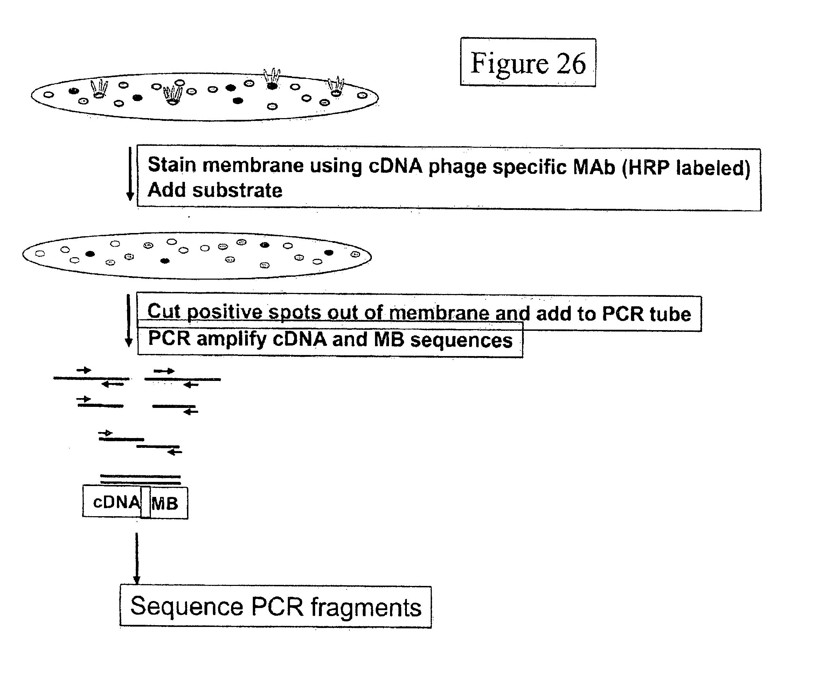

In some embodiments, the identity of a affinity agent-ligand pair is determined. For example, when both the affinity agent and the ligand are displayed on a phage or yeast, the DNA from the pair can be isolated and sequenced. In some embodiments, polynucleotides specific for the ligand and affinity agent are amplified. Amplification primers for each reaction can include 5' sequences that are complementary such that the resulting amplification products are fused, thereby forming a hybrid polynucleotide comprising a polynucleotide encoding at least a portion of the affinity agent and at least a portion of the ligand. The resulting hybrid can be used to probe affinity agent or ligand (e.g., cDNA-encoded) polynucleotide libraries to identify both affinity agent and ligand. *See, e.g.,* Figure 26.

The above-described methods can be readily combined with "walking" to simultaneous generate and identify multiple multimers, each of which bind to a ligand in a mixture of ligands. In these embodiments, a first library of affinity agents (monomer domains, immuno domains or multimers) are panned against multiple ligands and the eluted affinity agents are linked to the first or a second library of affinity agents to form a library of multimeric affinity agents (e.g., comprising 2, 3, 4, 5, 6, 7, 8, 9, or more monomer or immuno domains), which are subsequently panned against the multiple ligands. This method can be repeated to continue to generate larger multimeric affinity agents. Of course, at each stage, the panning is optionally repeated to enrich for significant binders.

The selected multimers of the above methods can be further manipulated, e.g., by recombining or shuffling the selected multimers (recombination can occur between or within multimers or both), mutating the selected multimers, and the like. This results in altered multimers which then can be screened and selected for members that have an enhanced property compared to the selected multimer, thereby producing selected altered multimers.

Linkers, multimers or selected multimers produced by the methods indicated above and below are features of the present invention. Libraries comprising multimers, e.g, a library comprising about 100, 250, 500 or more members produced by the methods of the present invention or selected by the methods of the present invention are provided. In some embodiments, one or more cell comprising members of the libraries, are also included. Libraries of the recombinant polypeptides are also a feature of the present invention, e.g., a library comprising about 100, 250, 500 or more different recombinant polypetides.

Compositions of the present invention can be bound to a matrix of an affinity material, e.g., the recombinant polypeptides. Examples of affinity material include, e.g., beads, a column, a solid support, and/or the like.

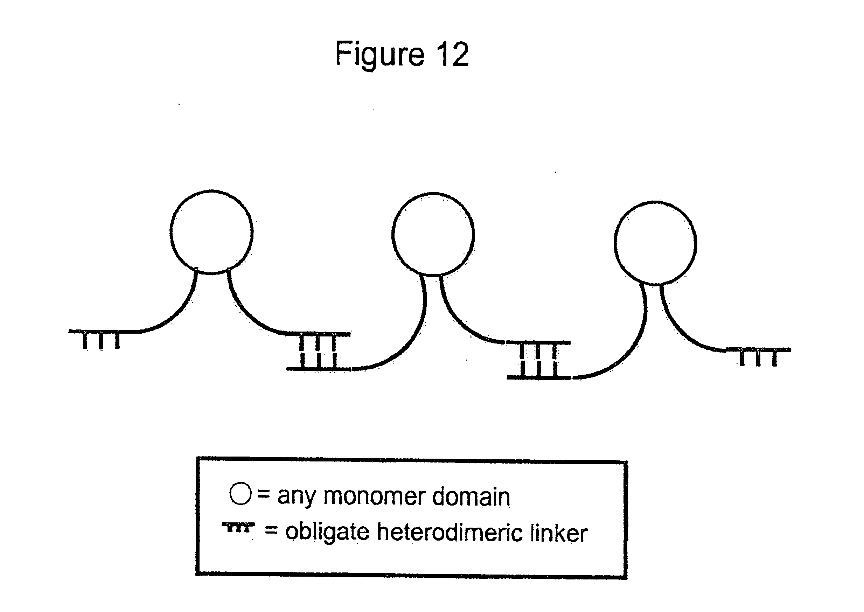

Suitable linkers employed in the practice of the present invention include an obligate heterodimer of partial linker moieties. The term "obligate heterodimer" (also referred to as "affinity peptides") refers herein to a dimer of two partial linker moieties that differ from each other in composition, and which associate with each other in a non-covalent, specific manner to join two domains together. The specific association is such that the two partial linkers associate substantially with each other as compared to associating with other partial linkers. Thus, in contrast to multimers of the present invention that are expressed as a single polypeptide, multimers of domains that are linked together via heterodimers are assembled from discrete partial linker-monomer-partial linker units. Assembly of the heterodimers can be achieved by, for example, mixing. Thus, i f the partial linkers are polypeptide segments, each partial linker-monomer-partial linker unit may be expressed as a discrete peptide prior to multimer assembly. A disulfide bond can be added to covalently lock the peptides together following the correct non-covalent pairing. A multimer containing such obligate heterodimers is depicted in Figure 12. Partial linker moieties that are appropriate for forming obligate heterodimers include, for example, polynucleotides, polypeptides, and the like. For example, when the partial linker is a polypeptide, binding domains are produced individually along with their unique linking peptide (i.e., a partial linker) and later combined to form multimers. *See*, *e.g.,* Madden, M., Aldwin, L., Gallop, M. A., and Stemmer, W. P. C. (1993) Peptide linkers: Unique self-associative high-affinity peptide linkers. Thirteenth American Peptide Symposium, Edmonton, Canada (abstract). The spatial order of the binding domains in the multimer is thus mandated by the heterodimeric binding specificity of each partial linker. Partial linkers can contain terminal amino acid sequences that specifically bind to a defined heterologous amino acid sequence. An example of such an amino acid sequence is the Hydra neuropeptide head activator as described in Bodenmuller et al., The neuropeptide head activator loses its biological activity by dimerization, (1986) EMBO J 5(8):1825-1829. *See, e.g.,* U.S. Patent No. 5,491,074 and WO 94/28173. These partial linkers allow the multimer to be produced first as monomer-partial linker units or partial linker-monomer-partial linker units that are then mixed together and allowed to assemble into the ideal order based on the binding specificities of each partial linker. Alternatively, monomers linked to partial linkers can be contacted to a surface, such as a cell, in which multiple monomers can associate to form higher avidity complexes via partial linkers. In some cases, the association will form via random Brownian motion.

When the partial linker comprises a DNA binding motif, each monomer domain has an upstream and a downstream partial linker (i.e., Lp-domain-Lp, where "Lp" is a representation of a partial linker) that contains a DNA binding protein with exclusively unique DNA binding specificity. These domains can be produced individually and then assembled into a specific multimer by the mixing of the domains with DNA fragments containing the proper nucleotide sequences (i.e., the specific recognition sites for the DNA binding proteins of the partial linkers of the two desired domains) so as to join the domains in the desired order. Additionally, the same domains may be assembled into many different multimers by the addition of DNA sequences containing various combinations of DNA binding protein recognition sites. Further randomization of the combinations of DNA binding protein recognition sites in the DNA fragments can allow the assembly of libraries of multimers. The DNA can be synthesized with backbone analogs to prevent degradation in vivo.

A significant advantage of the present invention is that known ligands, or unknown ligands can be used to select the monomer domains and/or multimers. No prior information regarding ligand structure is required to isolate the monomer domains of interest or the multimers of interest. The monomer domains, immuno-domains and/or multimers identified can have biological activity, which is meant to include at least specific binding affinity for a selected or desired ligand, and, in some instances, will further include the ability to block the binding of other compounds, to stimulate or inhibit metabolic pathways, to act as a signal or messenger, to stimulate or inhibit cellular activity, and the like. Monomer domains can be generated to function as ligands for receptors where the natural ligand for the receptor has not yet been identified (orphan receptors). These orphan ligands can be created to either block or activate the receptor top which they bind.

A single ligand can be used, or optionally a variety of ligands can be used to select the monomer domains, immuno-domains and/or multimers. A monomer domain and/or immuno-domain of the present invention can bind a single ligand or a variety of ligands. A multimer of the present invention can have multiple discrete binding sites for a single ligand, or optionally, can have multiple binding sites for a variety of ligands.

In some embodiments, the multimer comprises monomer domains and/or immuno-domains with specificities for different proteins. The different proteins can be related or unrelated. Examples of related proteins including members of a protein family or different serotypes of a virus. Alternatively, the monomer domains and/or immuno-domains of a multimer can target different molecules in a physiological pathway (e.g., different blood coagulation proteins). In yet other embodiments, monomer domains and/or immuno-domains bind to proteins in unrelated pathways (e.g., two domains bind to blood factors , two other domains and/or immuno-domains bind to inflammation-related proteins and a fifth binds to serum albumin). In another embodiment, a multimer is comprised of monomer domains that bind to different pathogens or contaminants of interest. Such multimers are useful to as a single detection agent capable of detecting for the possibility of any of a number of pathogens or contaminants.

The final conformation of the multimers containing immuno-domains can be a ring structure which would offer enhanced stability and other desired characteristics. These cyclic multimers can be expressed as a single polypeptide chain or may be assembled from multiple discrete polypeptide chains. Cyclic multimers assembled from discrete polypeptide chains are typically an assembly of two polypeptide chains. Figure 13B depicts a cyclic multimer of two polypeptide chains. The formation of cyclic multimer structures can be vastly effected by the spatial arrangement (i.e., distance and order) and dimerization specificity of the individual domains. Parameters such as, for example, linker length, linker composition and order of immuno-domains, can be varied to generate a library of cyclic multimers having diverse structures. Libraries of cyclic multimers can be readily screened in accordance with the invention methods described herein. to identify cyclic multimers that bind to desired target molecules. After the multimers are generated, optionally a cyclization step can be carried out to generate a library of cyclized multimers that can be further screened for desired binding activity.

These cyclic ring structures can be, for example, composed of a multimer of ScFv immuno-domains wherein the immuno-domains are split such that a coiling of the polypeptide multimer chain is required for the immuno-domains to form their proper dimeric structures (e.g., N-terrminus-$V_L1$-$V_L2$-$V_L3$-$V_L4$-$V_L5$-$V_L6$-$V_L7$-$V_L8$-$V_H1$-$V_H2$-$V_H3$-$V_H4$-$V_H5$-$V_H6$-$V_H7$-$V_H8$-C-terminus, or N-terminus-$V_L1$-$V_H2$-$V_L3$-$V_H4$-$V_H1$-$V_L2$-$V_H3$-$V_L4$-C-terminus, and the like). An example of such a cyclic structure is shown in Figure 13A. The ring could also be formed by the mixing of two polypeptide chains wherein each chain contained half of the immuno-domains. For example, one chain contains the $V_L$ domains and the other chain contains the $V_H$ domains such that the correct pairs of $V_L/V_H$ domains are brought together upon the two strands binding. The circularization of the chains can be mandated by changing the frame of the domain order (i.e., polypeptide one: N-terminu$_S$-$V_L1$-$V_L2$-$V_L3$-$V_L4$-$V_L5$-$V_L6$-$V_L7$-$V_L8$-C-terminus and polypeptide two: N-terminus-$V_H4$-$V_H5$-$V_H6$-$V_H7$-$V_H8$-$V_H1$-$V_H2$-$V_H3$-C-terminus) as depicted in Figure 13B.

A single polypeptide chain that forms a tetrameric ring structure could be very stable and have strong binding characteristics. An example of such a ring is shown in figure 13C.

Cyclic multimers can also be formed by encoding or attaching or linking at least one dimerizing domain at or near the N- terminus of a multimer protein and encoding or attaching or linking at least one second dimerizing domain at or near the C-terminus of the multimer protein wherein the first and second dimerization domain have a strong affinity for each other. As used herein, the term "dimerization domain" refers to a protein binding domain (of either immunological or non-immunological origin) that has the ability to bind to another protein binding domain with great strength and specificity such as to form a dimer. Cyclization of the multimer occurs upon binding of the first and the second dimerization domains to each other. Specifically, dimerization between the two domains will cause the multimer to adopt a cyclical structure. The dimerization domain can form a homodimer in that the domain binds to a protein that is identical to itself. The dimerization domain may form a heterodimer in that the domain binds to a protein binding domain that is different from itself. Some uses for such dimerization domains are described in, e.g., U.S. Patent No. 5,491,074 and WO 94/28173.

In some embodiments, the multimers of the invention bind to the same or other multimers to form aggregates. Aggregation can be mediated, for example, by the presence of hydrophobic domains on two monomer domains and/or immuno-domains, resulting in the formation of non-covalent interactions between two monomer domains and/or immuno-domains. Alternatively, aggregation may be facilitated by one or more monomer domains in a multimer having binding specificity for a monomer domain in another multimer. Aggregates can also form due to the presence of affinity peptides on the monomer domains or multimers. Aggregates can contain more target molecule binding domains than a single multimer.

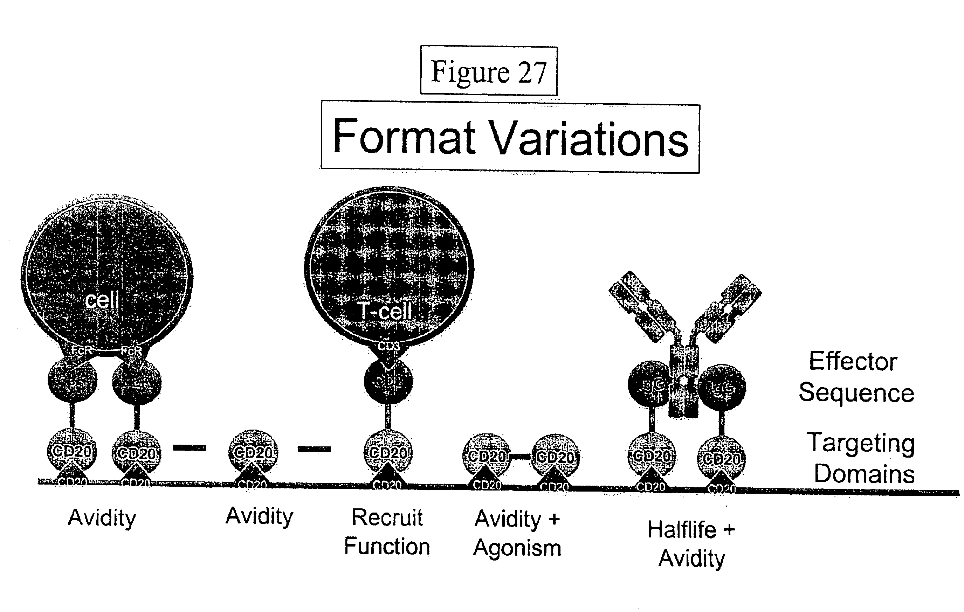

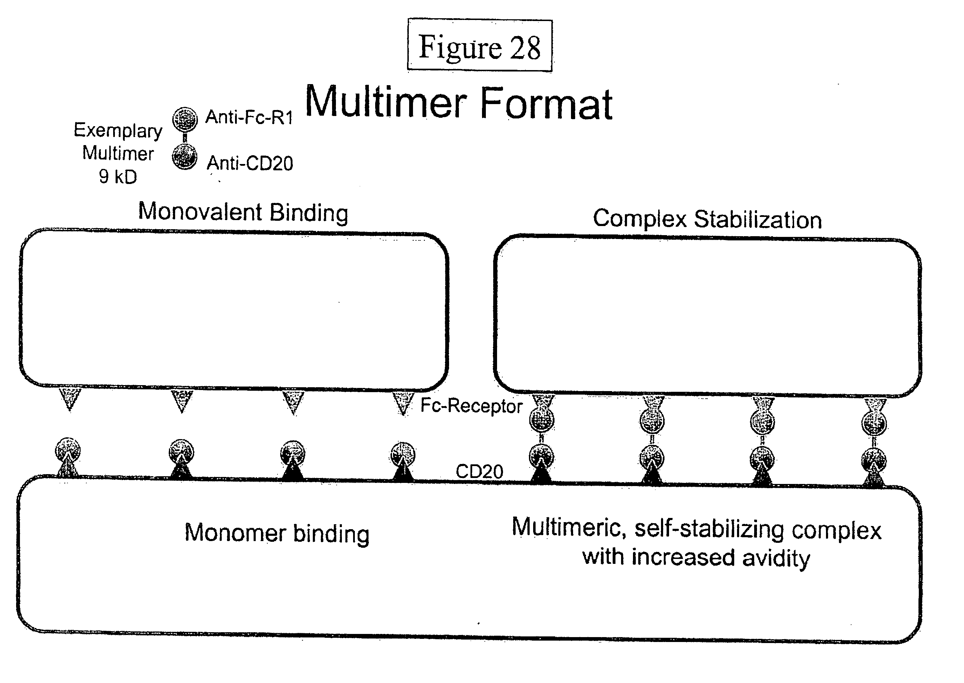

Multimers with affinity for both a cell surface target and a second target, avidity effects can be increased. In some cases, membrane fluidity can be more flexible than protein linkers in optimizing (by self-assembly) the spacing and valency of the interactions. In some cases, multimers will bind to two different targets, each on a different cell or one on a cell and another on a molecule with multiple binding sites. *See. e.g.,* Figures 27 and 28.

### 3. Therapeutic and Prophylactic Treatment Methods

The present invention also includes methods of therapeutically or prophylactically treating a disease or disorder by administering *in vivo* or *ex vivo* one or more nucleic acids or polypeptides of the invention described above (or compositions comprising a pharmaceutically acceptable excipient and one or more such nucleic acids or polypeptides) to a subject, including, e.g., a mammal, including a human, primate, mouse, pig, cow, goat, rabbit, rat, guinea pig, hamster, horse, sheep; or a non-mammalian vertebrate such as a bird (*e.g.*, a chicken or duck), fish, or invertebrate.

In one aspect of the invention, in *ex vivo* methods, one or more cells or a population of cells of interest of the subject (*e.g.*, tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc.*) are obtained or removed from the subject and contacted with an amount of a selected monomer domain and/or multimer of the invention that is effective in prophylactically or therapeutically treating the disease, disorder, or other condition. The contacted cells are then returned or delivered to the subject to the site from which they were obtained or to another site (*e.g.,* including those defined above) of interest in the subject to be treated. If desired, the contacted cells can be grafted onto a tissue, organ, or system site (including all described above) of interest in the subject using standard and well-known grafting techniques or, *e.g.*, delivered to the blood or lymph system using standard delivery or transfusion techniques.

The invention also provides *in vivo* methods in which one or more cells or a population of cells of interest of the subject are contacted directly or indirectly with an amount of a selected monomer domain and/or multimer of the invention effective in prophylactically or therapeutically treating the disease, disorder, or other condition. In direct contact/administration formats, the selected monomer domain and/or multimer is typically administered or transferred directly to the cells to be treated or to the tissue site of interest (*e.g.*, tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc.*) by any of a variety of formats, including topical administration, injection (*e.g.*, by using a needle or syringe), or vaccine or gene gun delivery, pushing into a tissue, organ, or skin site. The selected monomer domain and/or multimer can be delivered, for example, intramuscularly, intradermally, subdermally, subcutaneously, orally, intraperitoneally, intrathecally, intravenously, or placed within a cavity of the body (including, *e.g.*, during surgery), or by inhalation or vaginal or rectal administration.

In *in vivo* indirect contact/administration formats, the selected monomer domain and/or multimer is typically administered or transferred indirectly to the cells to be treated or to the tissue site of interest, including those described above (such as, *e.g.*, skin cells, organ systems, lymphatic system, or blood cell system, *etc.*), by contacting or administering the polypeptide of the invention directly to one or more cells or population of cells from which treatment can be facilitated. For example, tumor cells within the body of the subject can be treated by contacting cells of the blood or lymphatic system, skin, or an organ with a sufficient amount of the selected monomer domain and/or multimer such that delivery of the selected monomer domain and/or multimer to the site of interest (*e.g.*, tissue, organ, or cells of interest or blood or lymphatic system within the body) occurs and effective prophylactic or therapeutic treatment results. Such contact, administration, or transfer is typically made by using one or more of the routes or modes of administration described above.

In another aspect, the disclosure provides *ex vivo* methods in which one or more cells of interest or a population of cells of interest of the subject (*e.g.*, tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc.*) are obtained or removed from the subject and transformed by contacting said one or more cells or population of cells with a polynucleotide construct comprising a nucleic acid sequence of the invention that encodes a biologically active polypeptide of interest (*e.g.*, a selected monomer domain and/or multimer) that is effective in prophylactically or therapeutically treating the disease, disorder, or other condition. The one or more cells or population of cells is contacted with a sufficient amount of the polynucleotide construct and a promoter controlling expression of said nucleic acid sequence such that uptake of the polynucleotide construct (and promoter) into the cell(s) occurs and sufficient expression of the target nucleic acid sequence of the invention results to produce an amount of the biologically active polypeptide, encoding a selected monomer domain and/or multimer, effective to prophylactically or therapeutically treat the disease, disorder, or condition. The polynucleotide construct can include a promoter sequence (*e.g.*, CMV promoter sequence) that controls expression of the nucleic acid sequence of the invention and/or, if desired, one or more additional nucleotide sequences encoding at least one or more of another polypeptide of the invention, a cytokine, adjuvant, or co-stimulatory molecule, or other polypeptide of interest.

Following transfection, the transformed cells are returned, delivered, or transferred to the subject to the tissue site or system from which they were obtained or to another site (*e.g.*, tumor cells, tumor tissue sample, organ cells, blood cells, cells of the skin, lung, heart, muscle, brain, mucosae, liver, intestine, spleen, stomach, lymphatic system, cervix, vagina, prostate, mouth, tongue, *etc.*) to be treated in the subject. If desired, the cells can be grafted onto a tissue, skin, organ, or body system of interest in the subject using standard and well-known grafting techniques or delivered to the blood or lymphatic system using standard delivery or transfusion techniques. Such delivery, administration, or transfer of transformed cells is typically made by using one or more of the routes or modes of administration described above. Expression of the target nucleic acid occurs naturally or can be induced (as described in greater detail below) and an amount of the encoded polypeptide is expressed sufficient and effective to treat the disease or condition at the site or tissue system.

In another aspect, the disclosure provides *in vivo* methods in which one or more cells of interest or a population of cells of the subject (*e.g.*, including those cells and cells systems and subjects described above) are transformed in the body of the subject by contacting the cell(s) or population of cells with (or administering or transferring to the cell(s) or population of cells using one or more of the routes or modes of administration described above) a polynucleotide construct comprising a nucleic acid sequence of the invention that encodes a biologically active polypeptide of interest (*e.g.*, a selected monomer domain and/or multimer) that is effective in prophylactically or therapeutically treating the disease, disorder, or other condition.

The polynucleotide construct can be directly administered or transferred to cell(s) suffering from the disease or disorder (*e.g.*, by direct contact using one or more of the routes or modes of administration described above). Alternatively, the polynucleotide construct can be indirectly administered or transferred to cell(s) suffering from the disease or disorder by first directly contacting non-diseased cell(s) or other diseased cells using one or more of the routes or modes of administration described above with a sufficient amount of the polynucleotide construct comprising the nucleic acid sequence encoding the biologically active polypeptide, and a promoter controlling expression of the nucleic acid sequence, such that uptake of the polynucleotide construct (and promoter) into the cell(s) occurs and sufficient expression of the nucleic acid sequence of the invention results to produce an amount of the biologically active polypeptide effective to prophylactically or therapeutically treat the disease or disorder, and whereby the polynucleotide construct or the resulting expressed polypeptide is transferred naturally or automatically from the initial delivery site, system, tissue or organ of the subject's body to the diseased site, tissue, organ or system of the subject's body (*e.g.*, via the blood or lymphatic system). Expression of the target nucleic acid occurs naturally or can be induced (as described in greater detail below) such that an amount of expressed polypeptide is sufficient and effective to treat the disease or condition at the site or tissue system. The polynucleotide construct can include a promoter sequence (*e.g.*, CMV promoter sequence) that controls expression of the nucleic acid sequence and/or, if desired, one or more additional nucleotide sequences encoding at least one or more of another polypeptide of the invention, a cytokine, adjuvant, or co-stimulatory molecule, or other polypeptide of interest.

In each of the *in vivo* and *ex vivo* treatment methods as described above, a composition comprising an excipient and the polypeptide or nucleic acid of the invention can be administered or delivered. In one aspect, a composition comprising a pharmaceutically acceptable excipient and a polypeptide or nucleic acid of the invention is administered or delivered to the subject as described above in an amount effective to treat the disease or disorder.

In another aspect, in each *in vivo* and *ex vivo* treatment method described above, the amount of polynucleotide administered to the cell(s) or subject can be an amount such that uptake of said polynucleotide into one or more cells of the subject occurs and sufficient expression of said nucleic acid sequence results to produce an amount of a biologically active polypeptide effective to enhance an immune response in the subject, including an immune response induced by an immunogen (*e.g.*, antigen). In another aspect, for each such method, the amount of polypeptide administered to cell (s) or subject can be an amount sufficient to enhance an immune response in the subject, including that induced by an immunogen (*e.g.*, antigen).

In yet another aspect, in an in vivo or in vivo treatment method in which a polynucleotide construct (or composition comprising a polynucleotide construct) is used to deliver a physiologically active polypeptide to a subject, the expression of the polynucleotide construct can be induced by using an inducible on- and off-gene expression system. Examples of such on- and off-gene expression systems include the Tet-On™ Gene Expression System and Tet-Off™ Gene Expression System (*see. e.g.,* Clontech Catalog 2000, pg. 110-111 for a detailed description of each such system), respectively. Other controllable or inducible on- and off-gene expression systems arc known to those of ordinary skill in the art. With such system, expression of the target nucleic of the polynucleotide construct can be regulated in a precise, reversible, and quantitative manner. Gene expression of the target nucleic acid can be induced, for example, after the stable transfected cells containing the polynucleotide construct comprising the target nucleic acid are delivered or transferred to or made to contact the tissue site, organ or system of interest. Such systems are of particular benefit in treatment methods and formats in which it is advantageous to delay or precisely control expression of the target nucleic acid (*e.g.*, to allow time for completion of surgery and/or healing following surgery; to allow time for the polynucleotide construct comprising the target nucleic acid to reach the site, cells, system, or tissue to be treated; to allow time for the graft containing cells transformed with the construct to become incorporated into the tissue or organ onto or into which it has been spliced or attached, *etc.).*

### *4. Additional Multimer Uses*

The potential applications of multimers of the present invention are diverse and include any use where an affinity agent is desired. For example, the invention can be used in the application for creating antagonists, where the selected monomer domains or multimers block the interaction between two proteins. Optionally, the invention can generate agonists. For example, multimers binding two different proteins, e.g., enzyme and substrate, can enhance protein function, including, for example, enzymatic activity and/or substrate conversion.

Other applications include cell targeting. For example, multimers consisting of monomer domains and/or immuno-domains that recognize specific cell surface proteins can bind selectively to certain cell types. Applications involving monomer domains and/or immuno-domains as antiviral agents are also included. For example, multimers binding to different epitopes on the virus particle can be useful as antiviral agents because of the polyvalency. Other applications can include, but are not limited to, protein purification, protein detection, biosensors, ligand-affinity capture experiments and the like. Furthermore, domains or multimers can be synthesized in bulk by conventional means for any suitable use, *e,g*., as a therapeutic or diagnostic agent.

The present invention further provides a method for extending the half-life of a protein of interest in an animal. The protein of interest can be any protein with therapeutic, prophylactic, or otherwise desirable functionality. This method comprises first providing a monomer domain that has been identified as a binding protein that specifically binds to a half-life extender such as a blood-carried molecule or cell, such as serum albumin (e.g., human scrum albumin), IgG, red blood cells, etc.. The half-life extenderbinding monomer is then covalently linked to another monomer domain that has a binding affinity for the protein of interest. These three proteins (half-life extender-binding monomer, protein of interest-binding monomer, and the protein of interest) are then administered to a mammal where they will associate with the half-life extender to form a complex of the four components. This complex formation results in the half-life extender protecting the bound proteins from proteolytic degradation and thereby extending the half-life of the protein (*see, e.g.*, example 3 below). One variation of this use of the invention includes the half-life extender-binding monomer being covalently linked to the protein of interest. The protein of interest may include a monomer domain, a multimer of monomer domains, or a synthetic drug. Alternatively, monomers that bind to either immunoglobulins or erythrocytes could be generated using the above method and could be used for half-life extension.

The half-life extender-binding multimers are typically multimers of at least two domains, chimeric domains, or mutagenized domains. Suitable domains include all of those described herein, that are further screened and selected for binding to a half-life extender. The half-life extender-binding multimers are generated in accordance with the methods for making multimers described herein, using, for example, monomer domains pre-screened for half-life extender -binding activity. For example, some half-life extender-binding LDL receptor class A-domain monomers are described in Example 2 below.

The present invention also provides a method for the suppression of or lowering of an immune response in a mammal. This method comprises first selecting a monomer domain that binds to an immunosuppressive target. Such an "immunosuppressive target" is defined as any protein that when bound by another protein produces an immunosuppressive result in a mammal. The immunosuppressive monomer domain can then be either administered directly or can be covalently linked to another monomer domain or to another protein that will provide the desired targeting of the immunosuppressive monomer. The immunosuppressive multimers are typically multimers of at least two domains, chimeric domains, or mutangenized domains. Suitable domains include all of those described herein and are further screened and selected for binding to an immunosuppressive target. Immunosuppressive multimers are generated in accordance with the methods for making multimers described herein, using, for example, monomer domains pre-screened for CD40L-binding activity. Generation of CD40L-binding LDL receptor class A-domain monomers are described below in Example 4.

In some embodiments, the monomer domains are used for ligand inhibition, ligand clearance or ligand stimulation. Possible ligands in these methods, include, e.g., cytokines or chemokines.

If inhibition of ligand binding to a receptor is desired, a monomer domain is selected that binds to the ligand at a portion of the ligand that contacts the ligand's receptor, or that binds to the receptor at a portion of the receptor that binds contacts the ligand, thereby preventing the ligand-receptor interaction. The monomer domains can optionally be linked to a half-life extender, if desired.

Ligand clearance refers to modulating the half-life of a soluble ligand in bodily fluid. For example, most monomer domains, absent a half-life extender, have a short half-life. Thus, binding of a monomer domain to the ligand will reduce the half-life of the ligand, thereby reducing ligand concentration. The portion of the ligand bound by the monomer domain will generally not matter, though it may be beneficial to bind the ligand at the portion of the ligand that binds to its receptor, thereby further inhibiting the ligand's effect. This method is useful for reducing the concentration of any molecule in the bloodstream.

Alternatively, a multimer comprising a first monomer domain that binds to a half-life extender and a second monomer domain that binds to a portion of the ligand that does not bind to the ligand's receptor can be used to increase the half-life of the ligand.

In another embodiment, a multimer comprising a first monomer domain that binds to the ligand and a second monomer domain that binds to the receptor can be used to increase the effective affinity of the ligand for the receptor.

In another embodiment, multimers comprising at least two monomers that bind to receptors are used to bring two receptors into proximity by both binding the multimer, thereby activating the receptors.

In some embodiments, multimers with two different monomers can be used to employ a target-driven avidity increase. For example, a first monomer can be targeted to a cell surface molecule on a first cell type and a second monomer can be targeted to a surface molecule on a second cell type. By linking the two monomers to forma a multimer and then adding the multimer to a mixture of the two cell types, binding will occur between the cells once an initial binding event occurs between one multimer and two cells, other multimers will also bind both cells.

Further examples of potential uses of the invention include monomer domains, and multimers thereof, that are capable of drug binding (e.g., binding radionucleotides for targeting, pharmaceutical binding for half-life extension of drugs, controlled substance binding for overdose treatment and addiction therapy), immune function modulating (e.g., immunogenicity blocking by binding such receptors as CTLA-4, immunogenicity enhancing by binding such receptors as CD80, or complement activation by Fc type binding), and specialized delivery (e.g., slow release by linker cleavage, electrotransport domains, dimerization domains, or specific binding to: cell entry domains, clearance receptors such as FcR, oral delivery receptors such as plgR for trans-mucosal transport, and blood-brain transfer receptors such as transferrinR).

Additionally, monomers or multimers with different functionality may be combined to form multimers with combined functions. For example, the described HSA-binding monomer and the described CD40L-binding monomer can both be added to another multimer to both lower the immunogenicity and increase the half-life of the multimer.

In further embodiments, monomers or multimers can be linked to a detectable label (e.g., Cy3, Cy5, etc.) or linked to a reporter gene product (e.g., CAT, luciferase, horseradish peroxidase, alkaline phosphotase, GFP, etc.).

In some embodiments, the monomers of the invention are selected for the ability to bind antibodies from specific animals, e.g., goat, rabbit, mouse, etc., for use as a secondary reagent in detection assays.

In some cases, a pair of monomers or multimers are selected to bind to the same target (i.e., for use in sandwich-based assays). To select a matched maxybody pair, two different maxybodies typically are able to bind the target protein simultaneously. One approach to identify such pairs involves the following:

(1) immobilizing the phage mixture that was previously selected to bind the target protein
(2) contacting the target protein to the immobilized phage and washing;
(3) contacting the phage mixture to the bound target and washing; and
(4) eluting the bound phage without eluting the immobilized phage.

In some embodiments, different phage populations with different drug markers are used.

One use of the multimers or monomer domains of the invention is use to replace antibodies or other affinity agents in detection or other affinity-based assays. Thus, in some embodiments, monomer domains or multimers are selected against the ability to bind components other than a target in a mixture. The general approach can include performing the affinity selection under conditions that closely resemble the conditions of the assay, including mimicking the composition of a sample during the assay. Thus, a step of selection could include contacting a monomer domain or multimer to a mixture not including the target ligand and selecting against any monomer domains or multimers that bind to the mixture. Thus, the mixtures (absent the target ligand, which could be depleted using an antibody, monomer domain or multimer) representing the sample in an assay (serum, blood, tissue, cells, urine, semen, etc) can be used as a blocking agent.

### 5. Further Manipulating Monomer Domains and/or multimer Nucleic Acids and Polypeptides

As mentioned above, the polypeptide of the present invention can be altered. Descriptions of a variety of diversity generating procedures for generating modified or altered nucleic acid sequences encoding these polypeptides are described above and below in the following publications and the references cited therein: Soong, N. et al., Moleccdar bleeding of viruses, (2000) Nat Genet 25(4):436-439; Stemmer, et al., Molecular breeding of viruses for targeting and other clinical properties, (1999) Tumor Targeting 4:1-4; Ness et al., DNA Shuffling of subgenornic sequences of subtilisin, (1999) Nature Biotechnology 17:893-896; Chang et al., Evolution of a cytokine using DNA family shuffling, (1999) Nature Biotechnology 17:793-797; Minshull and Stemmer, Protein evolution by molecular breeding, (1999) Current Opinion in Chemical Biology 3:284-290; Christians et al., Directed evolution of thymidine kinase for AZTphosphorylation using DNA family shuffling, (1999) Nature Biotechnology 17:259-264; Crameri et al., DNA shuffling of a family of genes from diverse species accelerates directed evolution, (1998) Nature 391:288-291; Crameri et al., Molecular evolution of an arsenate detoxification pathway hy DNA shuffling, (1997) Nature Biotcchnology 15:436-438; Zhang et al., Directed evolution of an effective fucosidase from a galactosidase by DNA shuffling and screening (1997) Proc. Natl. Acad. Sci. USA 94: 4504-4509; Patten et al., Applications of DNA Shuffling to Pharmaceuticals and Vaccines, (1997) Current Opinion in Biotechnology 8:724-733; Crameri et al., Construction and evolution of antibody-phage libraries by DNA shuffling, (1996) Nature Medicine 2:100-103; Crameri et al., Improved green fluorescent protein by molecular evolution using DNA shuffling, (1996) Nature Biotechnolgy 14:315-319; Gates et al., Affinity selective isolation of ligands from peptide Libraries through display on a lac repressor 'headpiece dimer', (1996) Journal of Molecular Biology 255:373-386; Stemmer, Sexual PCR and Assembly PCR, (1996) In: The Encyclopedia of Molecular Biology. VCH Publishers, New York. pp.447-457; Crameri and Stemmer, Combinatorial multiple cassette mutagenesis creates all the permutations of mutant and wildtype cassettes, (1995) BioTechniques 18:194-195; Stemmer et al., Single-step assembly of a gene and entire plasmid form large numbers of oligodeoxy-ribonucleotides, (1995) Gene, 164:49-53; Stemmer, The Evolution of Molecular Computation, (1995) Science 270: 1510; Stemmer. Searching Sequence Space, (1995) Bio/Technology 13:549-553; Stemmer, Rapid evolution of a protein in vitro by DNA shuffling, (1994) Nature 370:389-391; and Stemmer, DNA shuffling by randorn fragmentation and reassembly: In vitro recombination for molecular evolution, (1994) Proc. Natl. Acad. Sci. USA 91:10747-10751.

Mutational methods of generating diversity include, for example, site-dirccted mutagenesis (Ling et al., Approaches to DNA mutagenesis: an overview, (1997) Anal Biochem. 254(2): 157-178; Dale et al., Oligonucleotide-directed random mutagenesis using the phosphorothioate method, (1996) Methods Mol. Biol. 57:369-374; Smith, In vitro mutagenesis, (1985) Ann. Rev. Genet. 19:423-462; Botstein & Shortle, Strategies and applications of in vitro mutagenesis, (1985) Science 229:1193-1201; Carter, Site-directed mutagenesis, (1986) Biochem. J. 237:1-7; and Kunkel, The efficiency of oligonucleotide directed mutagenesis, (1987) in Nucleic Acids & Molecular Biology (Eckstein, F. and Lilley, D.M.J. eds., Springer Verlag, Berlin)); mutagenesis using uracil containing templates (Kunkel, Rapid and efficient site-specific mutagenesis without phenotypic selection, (1985) Proc. Natl. Acad. Sci. USA 82:488-492; Kunkel et al., Rapid and efficient site-specific mutagesis without phenotypic selection, (1987) Methods in Enzymol. 154, 367-382; and Bass et al., Mutant Trp reepressors with new DNA-binding specificities, (1988) Science 242:240-245); oligonucleotide-directed mutagenesis ((1983) Methods in Enzymal. 100: 468-500; (1987) Methods in Enzymol. 154: 329-350; Zoller & Smith, Oligonucleotide-directed mutagenesis using M13-derived vectors: an efficient and general procedure for the production of point mutations in any DNA fragment, (1982) Nucleic Acids Res. 10:6487-6500; Zoller & Smith, Oligortucleatide-directed mutagenesis of DNA fragments cloned into M13 vectors, (1983) Methods in Enzymol. 100:468-500; and Zoller & Smith, Oligonucleotide-directed mutagenesis: a simple method using two oligonucleotide primers and a single-stranded DNA template, (1987) Methods in Enzymol. 154:329-350); phosphorothioate-modified DNA mutagenesis (Taylor et al., The use of phosphorothioate-modifed DNA in restriction enzyme reactions to prepare nicked DNA, (1985) Nucl. Acids Res. 13: 8749-8764; Taylor et al., The rapid generation of oligonucleotide-directed mutations at high frequency using phosphorothioate-modified DNA, (1985) Nucl. Acids Res. 13: 8765-8787; Nakamaye & Eckstein, Inhibition of restriction endonuclease Nci I cleavage by phosphorothioate groups and its application to oligonucleotide-directed mutagenesis, (1986) Nucl. Acids Res. 14: 9679-9698; Sayers et al., Y-T Exonucleases in phosphorothioate-based oligonucleotide-directed mutagenesis., (1988) Nucl. Acids Res. 16:791-802; and Sayers et al., Strand specific cleavage of phosphorothioate-containing DNA by reaction with restriction endonucleases in the presence of ethidium bromide, (1988) Nucl. Acids Res. 16: 803-814); mutagenesis using gapped duplex DNA (Kramer et al., The gapped duplex DNA approach to oligonucleotide-directed mutation construction, (1984) Nucl. Acids Res. 12: 9441-9456; Kramer & Fritz Oligonucleotide-directed construction of mutations via gapped duplex DNA, (1987) Methods in Enzymol. 154:350-367; Kramer et al., Improved enzymatic in vitro reactions in the gapped duplex DNA approach to oligonucleotide-directed construction of mutations, (1988) Nucl. Acids Res. 16: 7207; and Fritz et al., Oligonucleotide-directed construction of mutations: a gapped duplex DNA procedure without enzymatic reactions in vitro, (1988) Nucl. Acids Res. 16: 6987-6999).

Additional suitable methods include point mismatch repair (Kramer et al., Point Mismatch Repair, (1984) Cell 38:879-887), mutagenesis using repair-deficient host strains (Carter et al., Improved oligonucleotide site-directed mutagenesis using M13 vectors, (1985) Nucl. Acids Res. 13: 4431-4443; and Carter, Improved oligonucleotide-directed mutagenesis using M13 vectors, (1987) Methods in Enzymol. 154: 382-403), deletion mutagenesis (Eghtedarzadeh & Henikoff, Use of oligonucleotides to generate large deletions, (1986) Nucl. Acids Res. 14: 5115), restriction-selection and restriction-purification (Wells et al., Importance of hydrogen-bond formation in stabilizing the transition state of subtilisin, (1986) Phil. Trans. R. Soc. Lond. A 317: 415-423), mutagenesis by total gene synthesis (Nambiar et al., Total synthesis and cloning of a gene coding for the ribonuclease S protein, (1984) Science 223: 1299-1301; Sakamar and Khorana, Total synthesis and expression of a gene for the a-subunit of bovine rod outer segment guanine nucleotide-binding protein (transducin), (1988) Nucl. Acids Res. 14: 6361-6372; Wells et al., Cassette mutagenesis: an efficient merhod for generation of multiple mutations at defined sites, (1985) Gene 34:315-323; and Grundstrom et al., Oligonucleotide-directed mutagenesis by microscale 'shot-gun' gene synthesis, (1985) Nucl. Acids Res. 13: 3305-3316), double-strand break repair (Mandecki, Oligorzucleotide-directed double-strand break repair in plasmids of Escherichia coli: a method for site-specific mutagenesis, (1986) Proc. Natl. Acad. Sci. USA, 83:7177-7181; and Arnold, Protein engineeringfor unusual environments, (1993) Current Opinion in Biotechnology 4:450-455). Additional details on many of the above methods can be found in Methods in Enzymology Volume 154, which also describes useful controls for troubleshooting problems with various mutagenesis methods.

Additional details regarding various diversity generating methods can be found in the following U.S. patents, PCT publications and applications, and EPO publications: U.S. Pat. No. 5,605,793 to Stemmer (February 25, 1997), "Methods for In Vitro Recombination;" U.S. Pat. No. 5,811,238 to Stemmer et al. (September 22, 1998) "Methods for Generating Polynucleotides having Desired Characteristics by Iterative Selection and Recombination;" U.S. Pat. No. 5,830,721 to Stemmer et al. (November 3, 1998), "DNA Mutagenesis by Random Fragmentation and Reassembly;" U.S. Pat. No. 5,834,252 to Stemmer, et al. (November 10, 1998) "End-Complementary Polymerase Reaction;" U.S. Pat. No. 5,837,458 to Minshull, et al. (November 17, 1998), "Methods and Compositions for Cellular and Metabolic Engineering;" WO 95/22625, Stemmer and Crameri, "Mutagenesis by Random Fragmentation and Reassembly;" WO 96/33207 by Stemmer and Lipschutz "End Complementary Polymerase Chain Reaction;" WO 97/20078 by Stemmer and Crameri "Methods for Generating Polynucleotides having Desired Characteristics by Iterative Selection and Recombination;" WO 97/35966 by Minshull and Stemmer, "Methods and Compositions for Cellular and Metabolic Engineering;" WO 99/41402 by Punnonen et al. "Targeting of Genetic Vaccine Vectors;" WO 99/41383 by Punnonen et al. "Antigen Library Immunization;" WO 99/41369 by Punnonen et al. "Genetic Vaccine Vector Engineering;" WO 99/41368 by Punnonen et al. "Optimization of Immunomodulatory Properties of Genetic Vaccines;" EP 752008 by Stemmer and Crameri, "DNA Mutagenesis by Random Fragmentation and Reassembly;" EP 0932670 by Stemmer "Evolving Cellular DNA Uptake by Recursive Sequence Recombination;" WO 99/23107 by Stemmer et al., "Modification of Virus Tropism and Host Range by Viral Genome Shuffling;" WO 99/21979 by Apt et al., "Human Papillomavirus Vectors;" WO 98/31837 by del Cardayre et al. "Evolution of Whole Cells and Organisms by Recursive Sequence Recombination;" WO 98/27230 by Patten and Stemmer, "Methods and Compositions for Polypeptide Engineering;" WO 98/27230 by Stemmer et al., "Methods for Optimization of Gene Therapy by Recursive Sequence Shuffling and Selection," WO 00/00632, "Methods for Generating Highly Diverse Libraries," WO 00/09679, "Methods for Obtaining in Vitro Recombined Polynucleotide Sequence Banks and Resulting Sequences," WO 98/42832 by Arnold et al., "Recombination of Polynucleotide Sequences Using Random or Defined Primers," WO 99/29902 by Arnold et al., "Method for Creating Polynucleotide and Polypeptide Sequences," WO 98/41653 by Vind, "An in Vitro Method for Construction of a DNA Library," WO 98/41622 by Borchert et al., "Method for Constructing a Library Using DNA Shuffling," and WO 98/42727 by Pati and Zarling, "Sequence Alterations using Homologous Recombination;" WO 00/18906 by Patten et al., "Shuffling of Codon-Altered Genes;" WO 00104190 by del Cardayre et al. "Evolution of Whole Cells and Organisms by Recursive Recombination;" WO 00/42561 by Crameri et al., "Oligonucleotide Mediated Nucleic Acid Recombination;" WO 00/42559 by Selifonov and Stemmer "Methods of Populating Data Structures for Use in Evolutionary Simulations;" WO 00/42560 by Selifonov et al., "Methods for Making Character Strings, Polynucleotides & Polypeptides Having Desired Characteristics;" WO 01/23401 by Welch et al., "Use of Codon-Varied Oligonucleotide Synthesis for Synthetic Shuffling;" and PCT/US01/06775 "Single-Stranded Nucleic Acid Template-Mediated Recombination and Nucleic Acid Fragment Isolation" by Affholter.

Another aspect of the present invention includes the cloning and expression of monomer domains, selected monomer domains, multimers and/or selected multimers coding nucleic acids. Thus, multimer domains can be synthesized as a single protein using expression systems well known in the art. In addition to the many texts noted above, general texts which describe molecular biological techniques useful herein, including the use of vectors, promoters and many other topics relevant to expressing nucleic acids such as monomer domains, selected monomer domains, multimers and/or selected multimers, include Berger and Kimmel, Guide to Molecular Cloning Techniques, Methods in Enzymology volume 152 Academic Press, Inc., San Diego, CA (Berger); Sambrook et al., Molecular Cloning - A Laboratory Manual (2nd Ed.), Vol. 1-3, Cold Spring Harbor Laboratory, Cold Spring Harbor, New York, 1989 ("Sambrook") and Current Protocols in Molecular Biology, F.M. Ausubel et al., eds., Current Protocols, a joint venture between Greene Publishing Associates, Inc. and John Wiley & Sons, Inc., (supplemented through 1999) ("Ausubel")). Examples of techniques sufficient to direct persons of skill through in *vitro* amplification methods, useful in identifying, isolating and cloning monomer domains and multimers coding nucleic acids, including the polymerase chain reaction (PCR) the ligase chain reaction (LCR), Q-replicase amplification and other RNA polymerase mediated techniques (e.g., NASBA), are found in Berger, Sambrook, and Ausubel, as well as Mullis et al., (1987) U.S. Patent No. 4,683,202; PCR Protocols A Guide to Methods and Applications (Innis et al. eds) Academic Press Inc. San Diego, CA (1990) (Innis); Arnheim & Levinson (October 1, 1990) C&EN 36-47; The Journal Of NIH Research (1991) 3, 81-94; (Kwoh et al. (1989) Proc. Natl. Acad. Sci. USA 86, 1173; Guatelli et al. (1990) Proc. Natl. Acad. Sci. USA 87, 1874; Lomell et al. (1989) J Clin. Chem 35, 1826; Landegren et al., (1988) Science 241, 1077-1080; Van Brunt (1990) Biotechnology 8, 291-294; Wu and Wallace, (1989) Gene 4, 560; Barringer et al. (1990) Gene 89, 117, and Sooknanan and Malek (1995) Biotechnology 13: 563-564. Improved methods of cloning *in vitro* amplified nucleic acids are described in Wallace et al., U.S. Pat. No. 5,426,039. Improved methods of amplifying large nucleic acids by PCR are summarized in Cheng et al. (1994) Nature 369: 684-685 and the references therein, in which PCR amplicons of up to 40kb are generated. One of skill will appreciate that essentially any RNA can be converted into a double stranded DNA suitable for restriction digestion, PCR expansion and sequencing using reverse transcriptase and a polymerase. *See,* Ausubel, Sambrook and Berger, *all supra.*

The present invention also relates to the introduction of vectors of the invention into host cells, and the production of monomer domains, selected monomer domains immuno-domains, multimers and/or selected multimers of the invention by recombinant techniques. Host cells are genetically engineered (i.e., transduced, transformed or transfected) with the vectors of this invention, which can be, for example, a cloning vector or an expression vector. The vector can be, for example, in the form of a plasmid, a viral particle, a phage, etc. The engineered host cells can be cultured in conventional nutrient media modified as appropriate for activating promoters, selecting transformants, or amplifying the monomer domain, selected monomer domain, multimer and/or selected multimer gene(s) of interest. The culture conditions, such as temperature, pH and the like, are those previously used with the host cell selected for expression, and will be apparent to those skilled in the art and in the references cited herein, including, e.g., Freshney (1994) Culture of Animal Cells, a Manual of Basic Technique, third edition, Wiley- Liss, New York and the references cited therein.

As mentioned above, the polypeptides of the invention can also be produced in non-animal cells such as plants, yeast, fungi, bacteria and the like. Indeed, as noted throughout, phage display is an especially relevant technique for producing such polypeptides. In addition to Sambrook, Berger and Ausubel, details regarding cell culture can be found in Payne et al. (1992) Plant Cell and Tissue Culture in Liquid Systems John Wiley & Sons, Inc. New York, NY; Gamborg and Phillips (eds) (1995) Plant Cell, Tissue and Organ Culture; Fundamental Methods Springer Lab Manual, Springer-Verlag (Berlin Heidelberg New York) and Atlas and Parks (eds) The Handbook of Microbiological Media (1993) CRC Press, Boca Raton, FL.

The present invention also includes alterations of monomer domains, immuno-domains and/or multimers to improve pharmacological properties, to reduce immunogenicity, or to facilitate the transport of the multimer and/or monomer domain into a cell or tissue (e.g., through the blood-brain barrier, or through the skin). These types of alterations include a variety of modifications (e.g., the addition of sugar-groups or glycosylation), the addition of PEG, the addition of protein domains that bind a certain protein (e.g., HSA or other serum protein), the addition of proteins fragments or sequences that signal movement or transport into, out of and through a cell. Additional components can also be added to a multimer and/or monomer domain to manipulate the properties of the multimer and/or monomer domain. A variety of components can also be added including, e.g., a domain that binds a known receptor (e.g., a Fc-region protein domain that binds a Fc receptor), a toxin(s) or part of a toxin, a prodomain that can be optionally cleaved off to activate the multimer or monomer domain, a reporter molecule (e.g., green fluorescent protein), a component that bind a reporter molecule (such as a radionuclide for radiotherapy, biotin or avidin) or a combination of modifications.

### 6. Animal Models

Another aspect of the invention is the development of specific non-human animal models in which to test the immunogenicity of the monomer or multimer domains. The method of producing such non-human animal model comprises: introducing into at least some cells of a recipient non-human animal, vectors comprising genes encoding a plurality of human proteins from the same family of proteins, wherein the genes are each operably linked to a promoter that is functional in at least some of the cells into which the vectors are introduced such that a genetically modified non-human animal is obtained that can express the plurality of human proteins from the same family of proteins.

Suitable non-human animals employed in the practice of the present invention include all vertebrate animals, except humans (e.g., mouse, rat, rabbit, sheep, and the like). Typically, the plurality of members of a family of proteins includes at least two members of that family, and usually at least ten family members. In some embodiments, the plurality includes all known members of the family of proteins. Exemplary genes that can be used include those encoding monomer domains, such as, for example, members of the LDL receptor class A-domain family, the EGF-like domain family, as well as the other domain families described herein.

The non-human animal models of the present invention can be used to screen for immunogenicity of a monomer or multimer domain that is derived from the same family of proteins expressed by the non-human animal model. The present invention includes the non-human animal model made in accordance with the method described above, as well as transgenic non-human animals whose somatic and germ cells contain and express DNA molecules encoding a plurality of human proteins from the same family of proteins (such as the monomer domains described herein), wherein the DNA molecules have been introduced into the transgenic non-human animal at an embryonic stage, and wherein the DNA molecules are each operably linked to a promoter in at least some of the cells in which the DNA molecules have been introduced.

An example of a mouse model useful for screening LDL receptor class A-domain derived binding proteins is described as follows. Gene clusters encoding the wild type human LDL receptor class A-domain monomers are amplified from human cells using PCR. Almost all of the 200 different A-domains can be amplified with only three separate PCR amplification reactions of about 7kb each. These fragments are then used to generate transgenic mice according to the method described above. The transgenic mice will recognize the human A-domains as "sell", thus mimicking the "selfness" of a human with regard to A-domains. Individual A-domain-derived monomers or multimers are tested in these mice by injecting the A-domain-derived monomers or multimers into the mice, then analyzing the immune response (or lack of response) generated. The mice are tested to determine if they have developed a mouse anti-human response (MAHR). Monomers and multimers that do not result in the generation of a MAHR are likely to be non-immunogenic when administered to humans.

Historically, MAHR test in transgenic mice is used to test individual proteins in mice that are transgenic for that single protein. In contrast, the above described method provides a non-human animal model that recognizes an entire family of human proteins as "self," and that can be used to evaluate a huge number of variant proteins that each are capable of vastly varied binding activities and uses.

### 7. Kits

Kits comprising the components needed in the methods (typically in an unmixed form) and kit components (packaging materials, instructions for using the components and/or the methods, one or more containers (reaction tubes, columns, etc.)) for holding the components are a feature of the present invention. Kits of the present invention may contain a multimer library, or a single type of multimer. Kits can also include reagents suitable for promoting target molecule binding, such as buffers or reagents that facilitate detection, including detectably-labeled molecules. Standards for calibrating a ligand binding to a monomer domain or the like, can also be included in the kits of the invention.

The present invention also provides commercially valuable binding assays and kits to practice the assays. In some of the assays of the invention, one or more ligand is employed to detect binding of a monomer domain, immunodomains and/or multimer. Such assays are based on any known method in the art, e.g., flow cytometry, fluorescent microscopy, plasmon resonance, and the like, to detect binding of a ligand(s) to the monomer domain and/or multimer.

Kits based on the assay are also provided. The kits typically include a container, and one or more ligand. The kits optionally comprise directions for performing the assays, additional detection reagents, buffers, or instructions for the use of any of these components, or the like. Alternatively, kits can include cells, vectors, (e.g., expression vectors, secretion vectors comprising a polypeptide of the invention), for the expression of a monomer domain and/or a multimer of the invention.

In a further aspect, the present disclosure provides for the use of any composition, monomer domain, immunodomain, multimers, cell, cell culture, apparatus, apparatus component or kit herein, for the practice of any method or assay herein, and/or for the use of any apparatus or kit to practice any assay or method herein and/or for the use of cells, cell cultures, compositions or other features herein as a therapeutic formulation. The manufacture of all components herein as therapeutic formulations for the treatments described herein is also provided.

## 8. Integrated Systems

The present disclosure provides computers, computer readable media and integrated systems comprising character strings corresponding to monomer domains, selected monomer domains, multimers and/or selected multimers and nucleic acids encoding such polypeptides. These sequences can be manipulated by in silico shuffling methods, or by standard sequence alignment or word processing software.

For example, different types of similarity and considerations of various stringency and character string length can be detected and recognized in the integrated systems herein. For example, many homology determination methods have been designed for comparative analysis of sequences of biopolymers, for spell checking in word processing, and for data retrieval from various databases. With an understanding of double-helix pairwise complement interactions among 4 principal nucleobases in natural polynucleotides, models that simulate annealing of complementary homologous polynucleotide strings can also be used as a foundation of sequence alignment or other operations typically performed on the character strings corresponding to the sequences herein (*e.g.,* word-processing manipulations, construction of figures comprising sequence or subsequence character strings, output tables, etc.). An example of a software package with GOs for calculating sequence similarity is BLAST, which can be adapted to the present invention by inputting character strings corresponding to the sequences herein.

BLAST is described in Altschul et al., (1990) J. Mol. Biol. 215:403-410. Software for performing BLAST analyses is publicly available through the National Center for Biotechnology Information (available on the World Wide Web at ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score  falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) Proc. Natl. Acad. Sci. USA 89:10915).

An additional example of a useful sequence alignment algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, (1987) J. Mol. Evol. 35:351-360. The method used is similar to the method described by Higgins & Sharp, (1989) CADIOS 5:151-153. The program can align, e.g., up to 300 sequences of a maximum length of 5,000 letters. The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments. The program can also be used to plot a dendogram or tree representation of clustering relationships. The program is run by designating specific sequences and their amino acid or nucleotide coordinates for regions of sequence comparison. For example, in order to determine conserved amino acids in a monomer domain family or to compare the sequences of monomer domains in a family, the sequence of the invention, or coding nucleic acids, are aligned to provide structure-function information.

In one aspect, the computer system is used to perform "in silico" sequence recombination or shuffling of character strings corresponding to the monomer domains. A variety of such methods are set forth in "Methods For Making Character Strings, Polynucleotides & Polypeptides Having Desired Characteristics" by Selifonov and Stemmer, filed February 5, 1999 (USSN 60/118854) and "Methods For Making Character Strings, Polynucleotides & Polypeptides Having Desired Characteristics" by Selifonov and Stemmer, filed October 12, 1999 (USSN 09/416,375). In brief, genetic operators are used in genetic algorithms to change given sequences, e.g., by mimicking genetic events such as mutation, recombination, death and the like. Multi-dimensional analysis to optimize sequences can be also be performed in the computer system, e.g., as described in the '375 application.

A digital system can also instruct an oligonucleotide synthesizer to synthesize oligonucleotides, e.g., used for gene reconstruction or recombination, or to order oligonucleotides from commercial sources (e.g., by printing appropriate order forms or by linking to an order form on the Internet).

The digital system can also include output elements for controlling nucleic acid synthesis (e.g., based upon a sequence or an alignment of a recombinant, e.g., shuffled, monomer domain as herein), i.e., an integrated system of the invention optionally includes an oligonucleotide synthesizer or an oligonucleotide synthesis controller. The system can include other operations that occur downstream from an alignment or other operation performed using a character string corresponding to a sequence herein, e.g., as noted above with reference to assays.

EXAMPLES

The following example is offered to illustrate, but not to limit the claimed invention.

## Example 1

This example describes selection of monomer domains and the creation of multimers.

Starting materials for identifying monomer domains and creating multimers from the selected monomer domains and procedures can be derived from any of a variety of human and/or non-human sequences. For example, to produce a selected monomer domain with specific binding for a desired ligand or mixture of ligands, one or more monomer domain gene(s) are selected from a family of monomer domains that bind to a certain ligand. The nucleic acid sequences encoding the one or more monomer domain gene can be obtained by PCR amplification of genomic DNA or cDNA, or optionally, can be produced synthetically using overlapping oligonucleotides.

Most commonly, these sequences are then cloned into a cell surface display format (i.e., bacterial, yeast, or mammalian (COS) cell surface display; phage display) for expression and screening. The recombinant sequences are transfected (transduced or transformed) into the appropriate host cell where they are expressed and displayed on the cell surface. For example, the cells can be stained with a labeled (e.g., fluorescently labeled), desired ligand. The stained cells are sorted by flow cytometry, and the selected monomer domains encoding genes are recovered (e.g., by plasmid isolation, PCR or expansion and cloning) from the positive cells. The process of staining and sorting can be repeated multiple times (e.g., using progressively decreasing concentrations of the desired ligand until a desired level of enrichment is obtained). Alternatively, any screening or detection method known in the art that can be used to identify cells that bind the desired ligand or mixture of ligands can be employed.

The selected monomer domain encoding genes recovered from the desired ligand or mixture of ligands binding cells can be optionally recombined according to any of the methods described herein or in the cited references. The recombinant sequences produced in this round of diversification are then screened by the same or a different method to identify recombinant genes with improved affinity for the desired or target ligand. The diversification and selection process is optionally repeated until a desired affinity is obtained.

The selected monomer domain nucleic acids selected by the methods can be joined together via linker sequence to create multimers, e.g., by the combinatorial assembly of nucleic acid sequences encoding selected monomer domains by DNA ligation, or optionally, PCR-based, self-priming overlap reactions. The nucleic acid sequences encoding the multimers are then cloned into a cell surface display format (i.e., bacterial, yeast, or mammalian (COS) cell surface display; phage display) for expression and screening. The recombinant sequences are transfected (transduced or transformed) into the appropriate host cell where they are expressed and displayed on the cell surface. For example, the cells can be stained with a labeled, e.g., fluorescently labeled, desired ligand or mixture of ligands. The stained cells are sorted by flow cytometry, and the selected multimers encoding genes are recovered (e.g., by PCR or expansion and cloning) from the positive cells. Positive cells include multimers with an improved avidity or affinity or altered specificity to the desired ligand or mixture of ligands compared to the selected monomer domain(s). The process of staining and sorting can be repeated multiple times (e.g., using progressively decreasing concentrations of the desired ligand or mixture of ligands until a desired level of enrichment is obtained). Alternatively, any screening or detection method known in the art that can be used to identify cells that bind the desired ligand or mixture of ligands can be employed.

The selected multimer encoding genes recovered from the desired ligand or mixture of ligands binding cells can be optionally recombined according to any of the methods described herein or in the cited references. The recombinant sequences produced in this round of diversification are then screened by the same or a different method to identify recombinant genes with improved avidity or affinity or altered specificity for the desired or target ligand. The diversification and selection process is optionally repeated until a desired avidity or affinity or altered specificity is obtained.

## Example 2

This example describes the selection of monomer domains that are capable of binding to Human Serum Albumin (HSA).

For the production of phages, *E. coli* DH10B cells (Invitrogen) were transformed with phage vectors encoding a library of LDL receptor class A-domain variants as a fusions to the pIII phage protein. To transform these cells, the electroporation system MicroPulser (Bio-Rad) was used together with cuvettes provided by the same manufacturer. The DNA solution was mixed with 100 $\mu$l of the cell suspension, incubated on ice and transferred into the cuvette (electrode gap 1mm). After pulsing, 2 ml of SOC medium (2 % w/v tryptone, 0.5 % w/v yeast extract, 10 mM NaCl, 10 mM MgSO$_4$, 10 mM MgCl$_2$) were added and the transformation mixture was incubated at 37 C for 1 h. Multiple transformations were combined and diluted in 500 ml 2x YT medium containing 20 $\mu$g/m tetracycline and 2 mM CaCl$_2$. With 10 electroporations using a total of 10 $\mu$g ligated DNA 1.2x10$^8$ independent clones were obtained.

160 ml of the culture, containing the cells which were transformed with the phage vectors encoding the library of the A-domain variant phages, were grown for 24 h at 22 C, 250 rpm and afterwards transferred in sterile centrifuge tubes. The cells were sedimented by centrifugation (15 minutes, 5000 g, 4 °C). The supernatant containing the phage particles was mixed with 1/5 volumes 20 % w/v PEG 8000, 15 % w/v NaCl, and was incubated for several hours at 4 °C. After centrifugation (20 minutes, 10000 g, 4 °C) the precipitated phage particles were dissolved in 2 ml of cold TBS (50 mM Tris, 100 mM NaCl, pH 8.0) containing 2 mM CaCl$_2$. The solution was incubated on ice for 30 minutes and was distributed into two 1.5 ml reaction vessels. After centrifugation to remove undissolved components (5 minutes, 18500 g, 4 °C) the supernatants were transferred to a new reaction vessel. Phage were reprecipitated by adding 1/5 volumes 20 % w/v PEG 8000, 15 % w/v NaCl and incubation for 60 minutes on ice. After centrifugation (30 minutes, 18500 g, 4 °C) and removal of the supernatants, the precipitated phage particles were dissolved in a total of 1 ml TBS containing 2 mM CaCl$_2$. After incubation for 30 minutes on ice the solution was centrifuged as described above. The supernatant containing the phage particles was used directly for the affinity enrichment.

Affinity enrichment of phage was performed using 96 well plates (Maxisorp, NUNC, Denmark). Single wells were coated for 12 h at RT by incubation with 150 $\mu$l of a solution of 100 $\mu$g/ml human serum albumin (HSA, Sigma) in TBS. Binding sites remaining after HSA incubation were saturated by incubation with 250 $\mu$l 2% w/v bovine serum albumin (BSA) in TBST (TBS with 0.1 % v/v Tween 20) for 2 hours at RT. Afterwards, 40 $\mu$l of the phage solution, containing approximately 5x10$^{11}$ phage particles, were mixed with 80 $\mu$l TBST containing 3 % BSA and 2 mM CaCl$_2$ for 1 hour at RT. In order to remove non binding phage particles, the wells were washed 5 times for 1 min using 130 $\mu$l TBST containing 2 mM CaCl$_2$.

Phage bound to the well surface were eluted either by incubation for 15 minutes with 130 $\mu$l 0.1 M glycine/HCl pH 2.2 or in a competitive manner by adding 130 $\mu$l of 500 $\mu$g/ml HSA in TBS. In the first case, the pH of the elution fraction was immediately neutralized after removal from the well by mixing the eluate with 30 $\mu$l 1 M Tris/HCl pH 8.0.

For the amplification of phage, the eluate was used to infect *E. coli* K91BluKan cells (F$^+$). 50 $\mu$l of the eluted phage solution were mixed with 50 $\mu$l of a preparation of cells and incubated for 10 minutes at RT. Afterwards, 20 ml LB medium containing 20 $\mu$g/ml tetracycline were added and the infected cells were grown for 36 h at 22 C, 250 rpm. Afterwards, the cells were sedimented (10 minutes, 5000 g, 4 °C). Phage were recovered from the supernatant by precipitation as described above. For the repeated affinity enrichment of phage particles the same procedure as described in this example was used. After two subsequent rounds of panning against HSA, random colonies were picked and tested for their binding properties against the used target protein.

## Example 3

This example describes the determination of biological activity of monomer domains that are capable of binding to HSA.

In order to show the ability of an HSA binding domain to extend the serum half life of an protein *in vivo,* the following experimental setup was performed. A multimeric A-domain, consisting of an A-domain which was evolved for binding HSA (see Example 2) and a streptavidin binding A-domain was compared to the streptavidin binding A-domain itself. The proteins were injected into mice, which were either loaded or not loaded (as control) with human serum albumin (HSA). Serum levels of a-domain proteins were monitored.

Therefore, an A-domain, which was evolved for binding HSA (see Example 1) was fused on the genetic level with a streptavidin binding A-domain multimer using standard molecular biology methods (see Maniatis et al.). The resulting genetic construct, coding for an A-domain multimer as well as a hexahistidine tag and a HA tag, were used to produce protein in *E. coli.* After refolding and affinity tag mediated purification the proteins were dialysed several times against 150 mM NaCl, 5 mM Tris pH 8.0, 100 $\mu$M CaCl$_2$ and sterile filtered (0.45 $\mu$M).

Two sets of animal experiments were performed. In a first set, 1 ml of each prepared protein solution with a concentration of 2.5 $\mu$M were injected into the tail vein of separate mice and serum samples were taken 2, 5 and 10 minutes after injection. In a second set, the protein solution described before was supplemented with 50 mg/ml human serum albumin. As described above, 1 ml of each solution was injected per animal. In case of the injected streptavidin binding A-domain dimer, serum samples were taken 2, 5 and 10 minutes after injection, while in case of the trimer, serum samples were taken after 10, 30 and 120 minutes. All experiments were performed as duplicates and individual animals were assayed per timepoint.

In order to detect serum levels of A-domains in the serum samples, an enzyme linked immunosorbent assay (ELISA) was performed. Therefore, wells of a maxisorp 96 well microtiter plate (NUNC, Denmark) were coated with each 1 µg anti-His$_6$-antibody in TBS containing 2 mM CaCl$_2$ for 1 h at 4 C. After blocking remaining binding sites with casein (Sigma) solution for 1 h, wells were washed three times with TBS containing 0.1 % Tween and 2 mM CaCl$_2$. Serial concentration dilutions of the serum samples were prepared and incubated in the wells for 2 h in order to capture the α-domain proteins. After washing as before, anti-HA-tag antibody coupled to horse radish peroxidase (HRP) (Roche Diagnostics, 25 µg/ml) was added and incubated for 2 h. After washing as described above, HRP substrate (Pierce) was added and the detection reaction developed according to the instructions of the manufacturer. Light absorption, reflecting the amount of α-domain protein present in the serum samples, was measured at a wavelength of 450 nm. Obtained values were normalized and plotted against a time scale.

Evaluation of the obtained values showed a serum half life for the streptavidin binding A-domain of about 4 minutes without presence of HSA respectively 5.2 minutes when the animal was loaded with HSA. The trimer of A-domains, which contained the HSA binding A-domain, exhibited a serum half life of 6.3 minutes without the presence of HSA but a significantly increased half life of 38 minutes when HSA was present in the animal. This clearly indicates that the HSA binding A-domain can be used as a fusion partner to increase the serum half life of any protein, including protein therapeuticals.

## Example 4

This example describes experiments demonstrating extension of half-life of proteins in blood.

To further demonstrate that blood half-life of proteins can be extended using monomer domains of the invention, individual monomer domain proteins selected against monkey serum albumin, human serum albumin, human IgG, and human red blood cells were added to aliquots of whole, heparinized human or monkey blood.

The following list provides sequences of monomer domains analyzed in this example.

```
IG156      CLSSEFQCCQSSGRCIPLAWVCDGDNDCRDDSDEKSCKPRT
RBCA       CRSSQFQCNDSRICIPGRWRCDGDNDCQDGSDETGCGDSHILPFSTPGPST
RBCB       CPAGEFPCKNGQCLPVTWLCDGVNDCLDGSDEKGCGRPGPGATSAPAA
RBC11      CPPDEFPCKNGQCIPQDWLCDGVNDCLDGSDEKDCGRPGPGATSAPAA
CSA-A8     CGAGQFPCKNGHCLPLNLLCDGVNDCEDNSDEPSELCKALT
```

Blood aliquots containing monomer protein were then added to individual dialysis bags (25,000 MWCO), sealed, and stirred in 4 L of Tris-buffered saline at room temperature overnight.

Anti-6xHis antibody was immobilized by hydrophobic interaction to a 96-well plate (Nunc). Serial dilutions of serum from each blood sample were incubated with the immobilized antibody for 3 hours. Plates were washed to remove unbound protein and probed with α-HA-HRP to detect monomer.

Monomers identified as having long half-lives in dialysis experiments were constructed to contain either an HA, FLAG, E-Tag, or myc epitope tag. Four monomers were pooled, containing one protein for each tag, to make two pools.

One monkey was injected subcutaneously per pool, at a dose of 0.25 mg/kg/monomer in 2.5 mL total volume in saline. Blood samples were drawn at 24, 48, 96, and 120 hours. Anti-6xHis antibody was immobilized by hydrophobic interaction to a 96-well plate (Nunc). Serial dilutions of serum from each blood sample were incubated with the immobilized antibody for 3 hours. Plates were washed to remove unbound protein and separately probed with α-HA-HRP, α-FLAG-HRP, α-ETag-HRP, and α-myc-HRP to detect the monomer.

## Example 5

This example describes the determination of biological activity of monomer domains that are capable of binding to CD40L.

An LDL receptor class A-domain library was screened for monomers capable of binding CD40L using the same screening methods as described above in Example 2, except that recombinant CD40L was used as the target and no competitive elution steps were performed. In order to determine the biological activity of the selected A-domains, proteins were produced in *E. coli* as inclusion bodies or soluble protein. Biological activity of affinity-tag purified A-domain proteins with binding affinity to CD40L was measured by inhibition of rsCD40L stimulated B cell proliferation. Therefore, B cells were stimulated with Interleukin 4 (IL-4, R&D systems, Minneapolis, MN) and recombinant soluble CD40L (rsCD40L, Peprotech, Rocky Hill, NJ), and incorporation of Tritium-labeled thymidine was measured.

B cells were enriched from buffy coats of a healthy donor by gradient centrifugation and further purified by FACS. For a typical assay, B cells were transferred to a 96 well microtiter plate (100000 cells per well), and incubated for 3 days in appropriate tissue culture medium with IL-4 (100 μg/ml), rsCD40L (10 μg/ml) as well as different concentrations of selected α-domain variants. During the final 8 hours of incubation, the cultures were pulsed with 1 μCi/well of $^3$H thymidine (ICN) and the incorporation afterwards measured in a scintillation counter.

Selected A-domains with binding affinity to CD40L were able to inhibit rsCD40L induced B cell stimulation as shown by lowered thymidine incorporation in B cells that were incubated with the A-domains.

## Example 6

This example describes the development of a library of multimers comprised of C2 domains.

A library of DNA sequences encoding monomeric C2 domains is created by assembly PCR as described in Stemmer et al., Gene 164, 49-53 (1995). The oligonucleotides used in this PCR reaction are (SEQ ID NOS: 211-223, respectively, in order of appearance):

```
5'-acactgcaatcgcgccttacggctCCCGGGCGGATCCtcccataagttca
5'-agctaccaaagtgacannknnknnknnknnknnknnknnknnknnkccatacgtcgaattgttca
t
5'-agctaccaaagtgacaaaaggtgcttttggtgatatgttggatactccagatccatacgtcgaattgttca
t
5'-taggaagagaacacgtcattttnnknnknnkattaaccctgtttggaacgagacctttgagt
5'-taggaagagaacacgtcattttaataatgatattaaccctgtttggaacgagacctttgagt
5'-ttggaaatcaccctaatgnnknnknnknnknnknnknnkactctaggtacagcaa
5'-ttggaaatcaccctaatggatgcaaattatgttatggacgaaactctaggtacagcaa
5'-aagaaggaagtcccatttattttcaatcaagttactgaaatggtcttagagatgtccctt
5'-tgtcactttggtagctcttaacacaactacagtgaacttatgggaGGA
5'-acgtgttctcttcctagaatctggagttgtactgatgaacaattcgacgta
5'-attagggtgatttccaaaacattttcttgattaggatctaatataaactcaaaggtctcgtt
5'-atgggacttccttcttttctcccactttcattgaagatacagtaaacgttgctgtacctagagt
5'-gaccgatagcttgccgattgcagtgtGGCCACAGAGGCCTCGAGaacttcaagggacatctctaaga
```

PCR fragments are digested with BamHI and XhoI. Digestion products are separated on 1.5% agarose gel and C2 domain fragments are purified from the gel. The DNA fragments are ligated into the corresponding restriction sites of yeast surface display vector pYD1 (Invitrogen).

The ligation mixture is used for transformation of yeast strain EBY100. Transformants are selected by growing the cells in glucose-containing selective medium (-Trp) at 30°C.

Surface display of the C2 domain library is induced by growing the cells in galactose-containing selective medium at 20°C. Cells are rinsed with PBS and then incubated with fluorescently-labeled target protein and rinsed again in PBS.

Cells are then sorted by FACS and positive cells are regrown in glucose-containing selective medium. The cell culture may be used for a second round of sorting or may be used for isolation of plasmid DNA. Purified plasmid DNA is used as a template to PCR amplify C2 domain encoding DNA sequences.

The oligonucleotides used in this PCR reaction are (SEQ ID NOS: 224-225, respectively, in order of appearance):

```
5'-acactgcaatcgcgccttacggctCAGgtgCTGgtggttcccataagttcactgta
5'-gaccgatagcttgccgattgcagtCAGcacCTGaaccaccaccaccagaaccaccaccaccaacttcaa
 gggacatctcta (linker sequence is underlined).
```

PCR fragments are then digested with AlwNI, digestion products are separated on 1.5% agarose gel and C2 domain fragments are purified from the gel. Subsequently, PCR fragments are multimerized by DNA ligation in the presence of stop fragments. The stop fragments are listed below:

Stop 1 (SEQ ID NO:226):

```
5'-gaattcaacgctactaccattagtagaattgatgccacctttttcagctcgcgcc
ccaaatgaaaaaatggtcaaactaaatctactcgttcgcagaattgggaatcaactgttaca
tggaatgaaacttccagacaccgtactttatgaatatttatgacgattccgaggcgcgcccg
gactacccgtatgatgttccggattatgccccgggatcctcaggtgctg-3' (digested with
EcoRI and AlwNI).
```

Stop2 (SEQ ID NO:227):

```
5'-caggtgctgcactcgaggccactgcggccgcatattaacgtagattttttcctccc
aacgtcctgactggtataatgagccagttcttaaaatcgcataaccagtacatggtgattaa
agttgaaattaaaccgtctcaagagctttgttacgttgatttgggtaatgaagctt-3'
(digested with AlwNI and HindIII).
```

The ligation mixture is then digested with EcoRI and HindIII.

Multimers are separated on 1% agarose gel and DNA fragments corresponding to stop1-C2-C2-stop2 are purified from the gel. Stop1-C2-C2-stop2 fragments are PCR amplified using primers 5' aattcaacgctactaccat-3' (SEQ ID NO:242) and 5'-agcttcattacccaaatcaac-3' (SEQ ID NO:243) and subsequently digested with BamHI and XhoI. Optionally, the polynucleotides encoding the multimers can be put through a further round of affinity screening (e.g., FACS analysis as described above).

Subsequently, high affinity binders are isolated and sequenced. DNA encoding the high binders is cloned into expression vector and replicated in a suitable host. Expressed proteins are purified and characterized.

## Example 7

This example describes the development of a library of trimers comprised of LDL receptor A domains.

A library of DNA sequences encoding monomeric A domains is created by assembly PCR as described in Stemmer et al., Gene 164, 49-53 (1995). The oligonucleotides used in this PCR reaction are (SEQ ID NOS: 228-235, respectively, in order of appearance):

```
5'-CACTATGCATGGACTCAGTGTGTCCGATAAGGGCACACGGTGCCTACCCGTATGATGTTCCGGATTATGCC
CCGGGCAGTA
5'-CGCCGTCGCATMSCMAGYKCNSAGRAATACAWYGGCCGYTWYYGCACBKAAAATTSGYYAGVCNSACAGGTA
CTGCCCGGGGCAT
5'-CGCCGTCGCATMSCMATKCCNSAGRAATACAWYGGCCGYTWYYGCACBKAAAATTSGYYAGVCNSACAGGTA
CTGCCCGGGGCAT
5'-ATGCGACGGCGWWRATGATTGTSVAGATGGTAGCGATGAAVWGRRTTGTVMAVNMVNMVGCCVTACGGGCT
CGGCCTCT
5'-ATGCGACGGCGWWCCGGATTGTSVAGATGGTAGCGATGAAVWGRRTTGTVMAVNMVNMVGCCVTACGGGCT
CGGCCTCT
5'-ATGCGACGGCGWWRATGATTGTSVAGATAACAGCGATGAAVWGRRTTGTVMAVNMVNMVGCCVTACGGGCT
CGGCCTCT
5'-ATGCGACGGCGWWCCGGATTGTSVAGATAACAGCGATGAAVWGRRTTGTVMAVNMVNMVGCCVTACGGGCT
CGGCCTCT
5'-TCCTGGTAGTACTTATCTACTACTATTTGTCTGTGTCTGCTCTGGGTTCCTAACGGTTCGGCCACAGAGGC
CGAGCCCGTA
```

where R=A/G, Y=C/T, M=A/C, K=G/T, S=C/G, W=A/T, B=C/G/T, D=A/G/T, H=A/C/T, V=A/C/G, and N=A/C/G/T.

PCR fragments are digested with XmaI and SfiI. Digestion products are separated on 3% agarose gel and A domain fragments are purified from the gel. The DNA fragments are then ligated into the corresponding restriction sites of phage display vector fuse5-HA, a derivative of fuse5. The ligation mixture is electroporated into electrocompetent *E. coli* cells (F- strain e.g. Top10 or MC1061). Transformed *E. coli* cells are grown overnight in 2xYT medium containing 20 μg/ml tetracycline.

Virions are purified from this culture by PEG-precipitation. Target protein is immobilized on solid surface (e.g. Petri dish or microtiter plate) directly by incubating in 0.1 M NaHCO$_3$ or indirectly via a biotin-streptavidin linkage. Purified virions are added at a typical number of ~1-3 × 10$^{11}$ TU. The petridish or microtiter plate is incubated at 4°C, washed several times with washing buffer (TBS/Tween) and bound phages are eluted by adding glycine-HCl buffer. The eluate is neutralized by adding 1 M Tris-HCl (pH 9.1)

The phages are amplified and subsequently used as input to a second round of affinity selection. ssDNA is extracted from the final eluate using QIAprep M13 kit. ssDNA is used as a template to PCR amplify A domains encoding DNA sequences.

The oligonucleotides used in this PCR reaction are:

5'-aagcctcagcgaccgaa (SEQ ID NO: 236)
5'-agcccaataggaacccat (SEQ ID NO: 237)

PCR fragments are digested with AlwNI and BglI. Digestion products are separated on 3% agarose gel and A domain fragments are purified from the gel. PCR fragments are multimerized by DNA ligation in the presence of the following stop fragments:

Stop (SEQ ID NO: 238):

```
5'-gaattcaacgctactaccattagtagaattgatgccaccttttcagctcgcgccccaaatgaaaaaatggt
caaactaaatctactcgttcgcagaattgggaatcaactgttacatggaatgaaacttccagacaccgtactta
tgaatatttatgacgattccgaggcgcgcccggactacccgtatgatgttccggattatgccccgggcggatcca
gtacctg-3'
```

(digested with EcoRI and ALwNI)

Stop2 (SEQ ID NO: 239):

```
5'-gccctacgggcctcgaggcacctggtgcggccgcatattaacgtagattttcctcccaacgtcctgactg
gtataatgagccagttcttaaaatcgcataaccagtacatggtgattaaagttgaaattaaaccgtctcaagagc
tttgttacgttgatttgggtaatgaagctt-3'
```

(digested with BglI and HindIII)

The ligation mixturee is digested with EcoRI and HindIII.

Multimers are separated on 1% agarose gel and DNA fragments corresponding to stop1-A-A-A-stop2 are purified from the gel. Stop1-A-A-A-stop2 fragments are subsequently PCR amplified using primers 5'-agcttcattacccaaatcaac-3' and 5' aattcaacgctactaccat-3' and subsequently digested with XmaI and SfiI. Selected polynucleotides are then cloned into a phage expression system and tested for affinity for the target protein.

High affinity binders are subsequently isolated and sequenced. DNA encoding the high binders is cloned into expression vector and subsequently expressed in a suitable host. The expressed protein is then purified and characterized.

## Example 8

This example describes the development of CD20-specific LDL receptor-based A domains.

$10^{11}$ phage displaying a library of $10^9$ A-domains were added to $10^6$ Raji or Daudi cells which had been pre-blocked with 5 mg/mL casein. The mixture was incubated at 4°C for 2 hours to allow phage to bind. Cells were washed 5 times with 1 mg/mL casein in TBS. Cells were incubated with 1 mg/mL Rituxan in TBS at 4"C for 2 hours to elute phage specific for CD20. Cells were spun down and the supernatant used to infect E. coli BluKan cells.

Phage were propagated for 2 days at 25°C and purified by standard methods. Panning was repeated three times, alternating selection on either Raji or Daudi cells. Thirty-two clones were picked and incubated with Raji or Daudi cells in the presence or absence of 1 mg/mL Rituxan.

Clones which showed differential binding were sequenced (Table 1) and cloned into expression vectors with SKVILF peptides fused N- and C-terminally. Protein was produced and purified according to standard methods.

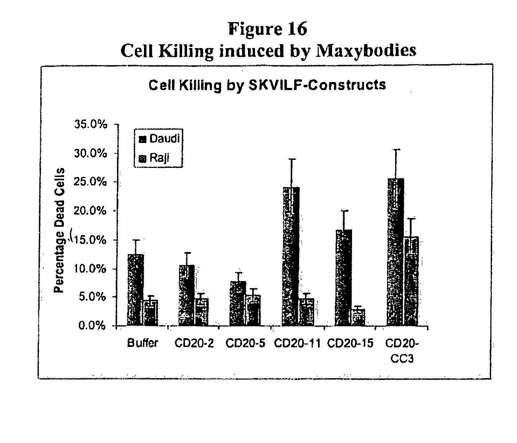

Raji or Daudi cells were incubated in fresh RPMI medium supplemented with 10% FBS in the presence or absence of purified monomers for 6 hours at 37°C. Dead cells were stained with trypan blue and counted visually using a hemocytometer (Figure 16).

Table 1: CD20 binding sequences

| | |
|---|---|
| 2 | **C**LPDEFQ**C**RSTGI**C**IPLAWR**C**DGVND**C**QDDSDETN**C**RATGRT |
| 3 | **C**LPGEFR**C**RGTSI**C**IPPSWV**C**DGVDD**C**GDGSDEALEH**C**GSHILPFSTPGPST |
| 4 | **C**QPNEFP**C**GSTGL**C**VPREWL**C**DGVDH**C**QDGSDEPD**C**GDSHILPFSTPGPST |
| 5 | **C**LPGEFR**C**RGTSI**C**IPPPSWV**C**DGVDD**C**GDGSDEALEH**C**GDSHILPFSTPGPST |

(continued)

| | |
|---|---|
| 6 | **C**RSGEFK**C**HGTRP**C**VPQRWV**C**DDDD**C**VDGSDEKS**C**ETPARR |
| 7 | **C**RSSQF**C**HNTRP**C**IPGRWV**C**DGVND**C**LDGSDEAN**C**RRAARR |
| 8 | **C**LPERFQ**C**AVPGY**C**IPLPGV**C**DGVND**C**QEDSDEPN**C**RAPGLR |
| 9 | **C**RRNEFR**C**KSGH**C**VPQPLV**C**DGVRD**C**EDNSDEPS**C**GRPGPGATSAPAA |
| 10 | **C**RAGEFP**C**KNGQ**C**LPVTWL**C**DGVND**C**LDGSDEKG**C**GRPGAGATSAPAA |
| 11 | **C**PSNEFT**C**FTCKSGH**C**VPQPFV**C**DGVPD**C**NSDETS**C**GRPGPGATSAPAA |
| 14 | **C**RASEFP**C**RGTGT**C**IPRHWL**C**DGEND**C**ADSSDEKD**C**GRPGATSAPAA |
| 15 | **C**PPDEFR**C**KSYKY**C**VPLAFV**C**DGVDD**C**EDGSDEEG**C**GRPGPGATSAPAA |
| 1 | **C**LPDEFQ**C**RSTGI**C**IPLAWR**C**DGVND**C**QDDSDETN**C**RATGRT |
| 6 | **C**PAGEFQ**C**GNGQ**C**IPATWL**C**DGVND**C**LDNSDETG**C**SQDPEFHKV |
| CC3 | **C**PASQFK**C**HNTRT**C**IPRRWV**C**DGVND**C**LDGSDEAN**C**RRAAPT |

## Example 9

This example describes the development of TPO-R-specific LDL receptor-based A domains.

$10^{11}$ phage displaying a library of $10^9$ A-domains were added to recombinant TPO-R, which had been coated to Immunosorp plates (Nunc) and blocked with casein. Phage were incubated with the target for 3 hours at 4°C, washed 3 times with TBS buffer, and eluted with 100 mM Glycine pH 2.2. The eluate was neutralized by addition of 2M Na2HPO$_4$ and used to infect E. coli BluKan cells.

Phage were propagated at 37°C overnight, purified by standard methods, and the selection repeated one time. After the second round of selection on immobilized TPO-R, phage were added to a suspension of TF1 cells, which had been blocked previously with casein. Cells were incubated 2 hours at 4°C, then washed 5 times with TBS.

Phage were eluted by direct addition of E. coli to the cell suspension, followed by propagation of the phage at 23°C for 2 days. The phage were purified by standard methods, and the selection on TF1 cells was repeated one time.

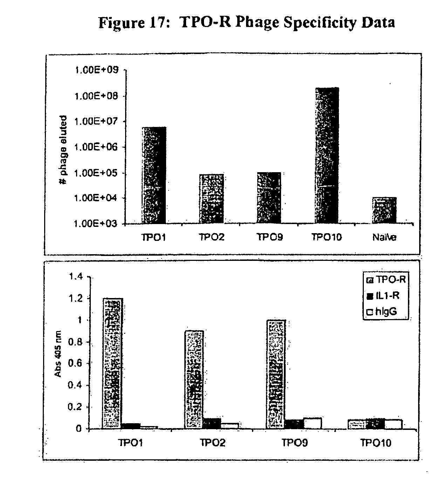

The phage resulting from the second round of selection on TF1 cells was used for one round of selection on immobilized recombinant TPO-R as described above, with the exception that phage were eluted using a solution of 50 mM EDTA and 20 mM DTT. Phage clones were picked and assayed for their ability to bind both recombinant TPO-R and TF1 cells (Figure 17), and sequenced (Table 2).

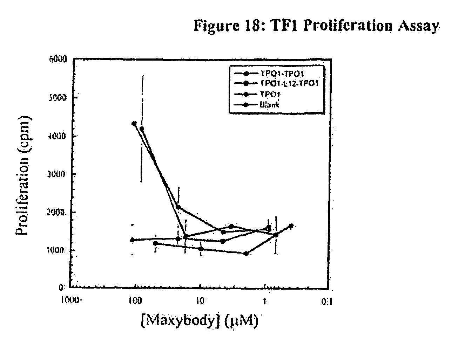

Positive clones were genetically fused to create direct homodimers, with and without insertion of a 12 amino-acid repeated Gly-Gly-Ser linker between the domains, using standard molecular biology techniques, and were cloned into an expression vector. Protein was produced and purified using standard techniques. Protein was assayed for its ability to mimic natural TPO activity in a TF1 cell proliferation assay (Figure 18).

### Table 2: TPO-R Binding Sequences

| | |
|---|---|
| T4690 (TPO1) | **C**HSTGEFR**C**RSSGI**C**VSPTWV**C**DGEND**C**LDGSDEAS**C**TAAGPT |
| T5 (TP02) | **C**PPSEFR**C**NSGQ**C**IPREWR**C**DGDND**C**ADNSDEES**C**SAPASEPPGSLSLQ |
| T2 (TP09) | **C**LPSEFRSSGH**C**IPRRWKR**C**DGEP**C**QDGSDEAN**C**GTSEHTSLQ |
| T1 (TPO10) | **C**QSNEFQ**C**HNYNI**C**LPRPWV**C**DGV**C**DGSDEEG**C**SAPASEPPGSLSLQ |

## Example 10

This example describes the development of IgE-specific LDL receptor-based A domains and multimers.

$10^{11}$ phage displaying a library of $10^9$ A-domains were added to human IgE, which had been immobilized on Immunosorp plates (Nunc) and blocked with casein. Soluble human IgG was added with the phage to a concentration of 5 mg/mL. Plates were incubated at 4°C for 3 hours, then washed 3 times to remove unbound phage. Phage were eluted with a mixture of 50 mM EDTA, 20 mM DTT and used to infect E. coli. Phage were propagated at 25°C for 2 days, and purified by standard methods.

Selection on immobilized IgE was repeated two times. Individual clones were sequenced and assayed for IgE binding affinity. A single clone, which was the major component of the selected library, was chosen for further study (Table 3).

The monomer binding epitope was mapped by measuring the number of phage bound to human IgE immobilized by one of several methods: 1) IgE directly coated to plastic, 2) IgE bound via an antibody that binds IgE at its CE2 domain, 3) IgE bound via an antibody that binds IgE at its CE3 domain, or 4) IgE bound via immobilized recombinant soluble IgE receptor. Differential frequencies of binding of the phage clones show the binding site on IgE that these phage clones recognize.

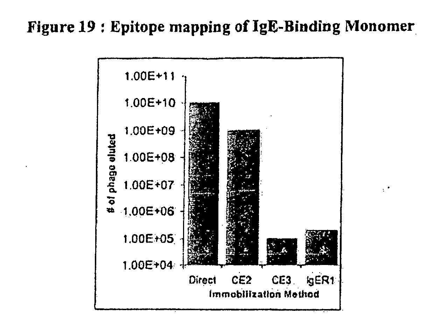

The data show that this monomer binds to CE3 and interferes with normal receptor binding (Figure 19).

A new library was created by ligating a random domain to either the 5' or 3' end of the IgE binding sequence and ligating this construct into the fuse5 phage DNA. Phage were produced and purified by standard methods. Dimer phage were incubated with human IgE, which had been immobilized in 10-fold serial dilutions (from 1 μg to 10 ag per well) to an Immunosorp plate (Nunc) and blocked with casein. Soluble human IgG at 5 mg/mL was added with the phage. Wells were washed 10 times with 100 mM sodium acetate pH 5, and phage were eluted by direct addition of E. coli cells.

The phage titer eluted from each serial dilution was compared to the titer eluted from a blank well, and the lowest dilution which showed enrichment over the blank well was chosen for propagation (typically 1-10 pg per well). Phage were propagated at 25°C for 2 days and purified by standard methods.

Phage were selected on serial dilutions 2 additional times. Individual clones were sequenced (Table 3)

### Table 3 : IgE-Binding Monomer Sequences

IGE-1
CPANEFQCRNSSTCIPRRWLCDGDDDCGDGSDETGCSAPASEPPGSLSLQ

**Walked Dimers**

1
CPANEFQCRNSSTCIPRRWLCDGDDDCGDGSDETGCSAPASEPPGSL
CQPDQFRCSSGRCLSREWLCDGEDDCEDDSDETDCPTRTSLQ

2
CPANEFQCRNSSTCIPRRWLCDGDDDCGDGSDETGCSAPASEPPGSL
CLPSQFPCDSGNCLPLTWLCDGVDDCGDNSDEEDCSAPASEPPGSLSLQ

3
CPANEFQCRNSSTCIPRRWLCDGDDDCGDGSDETGCSAPASEPPGSL
CRANQFPCDNGNCLPQPWRCDGDNDCVDGSDETSCEAPAHTSLQ

4
CPANEFQCRNSSTCIPRRWLCDGDDDCGDGSDETGCSAPASEPPGSL
CAPNEFQCRDNNTCLPEDWRCDGEDDCADNSDEANCTTPGPTSLQ

5
CPANEFQCRNSSTCIPRRWLCDGEDDCEDGSDEASDTCSAPASEPPGSL
CPANEFQCRNSSTCIPRRWLCDGDDDCGDGSDETGCSAPASEPPGSLSLQ

6
CGSGQFPCGSGHCVPLNWVCDGVDDCGDDSDETDCKAHT
CPANEFQCRNSSTCIPRRWLCDGDDDCGDGSDETGCSAPASEPPGSLSLQ

7
CPANEFQCRNSSTCIPRRWLCDGDDDCGDGSDETGCSAPASEPPGSL
CGADQFPCSSGHCIPLPWVCDGEDDCADGSDEADCRGTEPTSLQ

8
CPANEFQCRNSSTCIPRRWLCDGDDDCGDGSDETGCSAPASEPPGSL
CAPSQFRCGNGRCIPRSWRCDGEDDCADDSDEENCSAPASEPPGSLSLQ

9
RVWRRLVGS
CRPNQFTCKSSETCIPAHWRCDGDDDCGDGSDEADCETRT
CPANEFQCRNSSTCIPRRWLCDGDDDCGDGSDETGCSAPASEPPGSLSLQ
10
CPANEFQCRNSSTCIPRRWLCDGDDDCGDGSDETGCSAPASEPPGSL
CQSSQFPCHDYEICLPATLLCDGVDDCLDGSDETNCAKPTSLQ
12
CPANEFQCRNSSTCIPRRWLCDGDDDCGDGSDEPGCSAPASEPPGSL
CPPGEFPCGNGRSVPLTWLCDGVDDCGDNSDETGCETTGRTSLQ
13 (27)
CGSNQFPCENGNCVPLGWGCDGVNDCQDNSDESLATCGRPGPGATSAPAA
CPANEFQCRNSSTCIPRRWLCDGDDDCGDGSDETGCSAPASEPPGSLSLQ
14
CPSGQFPCDNGHCIPRRWLCDGEDDCPDGSDEAQVCQQRT
CPANEFQCRNSSTCIPRRWLCDGDDDCGDGSDETGCSAPASEPPGSLSLQ
15
CPANEFQCRNSSTCIPRRWLCDGDDDCGDGSDETGCSAPASEPPGSLSLQ
ALLCDGVDDCRDGSDESALCEEHT
CPANEFQCRNSSTCIPRRWLCDGDDDCGDGSDETGCSAPASEPPGSLSLQ
16
CPANEFQCRNSSTCIPRRWLCDGDDDCGDGSDEPGCSAPASEPPGSL
CRRAEFTCRNGSCLPVPWLCDAENDCPDGSDEPDCGSPARRSLQ
19
CPANEFQCRNSSTCIPRRWLCDGDDDCGDGSDEPGCSAPASEPPGSL
CPPDQFRCKNGRCIPRHLVCDGDDDCGDDSDEAGCQTRTSLQ
21
CPANEFQCRNSSTCIPRRWLCDGDDDCGDGSDETGCSAPASEPPGSL
CEPGQFQCNNNDTCVSPPWLCDADRDCGRSDERPPHCATPELTSLQ
23
CPAGQFRCENGRCLPPPWRCDGVNDCEDNSDEAGCGDSHILPFSTPGPST
CPANEFQCRNSSTCIPRRWLCDGDDDCGDGSDETGCSAPASEPPGSLSLQ
25
CPANEFQCRNSSTCIPRRWLCDGDDDCGDGSDEPGCSAPASEPPGSL
CLSSQFRCENGQCIPLTWGCDGDDDCQDGSDETNCPTRTSLQ
26
CPANEFQCRNSSTCIPRRWLCDGDDDCVDGSDETGCGSPVPT
CPANEFQCRNSSTCIPRRWLCDGDDDCGDGSDETGCSAPASEPPGSLSLQ
27 (13)
CGSNQFPCENGNCVPLGWGCDGVNDCQDNSDESLATCGRPGPGATSAPAA
CPANEFQCRNSSTCIPRRWLCDGDDDCGDGSDETGCSAPASEPPGSLSLQ
30
CPANEFQCRNSSTCIPRRWLCDGDDDCGDGSDEPGCSAPASEPPGSL
CAASQFRCNNNSRCLPPPLGCDGVDDCGDNSDEADCGRPGPGATSAPAASLQ
31
CPANEFQCRNSSTCIPRRWLCDGDDDCGDGSDETGCSAPASEPPGSL
CPANEFQCRNSSTCIPRRWLCDGEDDCGDGSDETGCSAPASEPPGSLSLQ

## Example 11

This example describes the development of CD28-specific LDL receptor-based A domains and dimers by "walking."

A library of DNA sequences encoding monomeric A domains was created by assembly PCR as described in Stemmer et al., Gene 164:49-53 (1995). The oligonucleotides used in this PCR reaction are:

```
5'-ATTCTCACTCGGCCGACGGTGCCTACCCGT-3'
5'-ACGGTGCCTACCCGTATGATGTTCCGGATTATGCCCCGGGTCTGGAGGCGTCTGGTGGTTCGTGT-3'
5'-CGCCGTCGCAAMSCMASBBCNSTGRAABGCATNTKYYGKWAYYSYKGCATYYAAATTBGBYGRDAGVKTBACACGAACC
    ACCAGA-3'
5'-CGCCGTCGCAAMSCMASBBCNSTGRAABGCAKYKGCCGYTKYYGCATYYAAATTBGBYGRDAGVKTBACACGAACCACCAGA-3'
5'-CGCCGTCGCAAMSCMASBBCNSTGRAABGCATNTKYYGKWAYYSYKGCACBKGAACTSGYYCGVCNSACA
    CGAACCACCAGA-3'
5'-CGCCGTCGCAAMSCMASBBCNSTGRAABGCAKYKGCCGYTKYYGCACBKGAACTSGYYCGVCNSACACGAACCACCAGA-3'
5'-TTGCGACGGCGWWRATGATTGTSNGGACRRCTCGGATGAA-3'
5'-TTGCGACGGCGWWRATGATTGTSSGGACGGCTCGGATGAA-3'
5'-TTGCGACGGCGWWRATGATTGTSRGGACRRCTCGGATGAA-3'
5'-TTGCGACGGCGWWCCGGATTGTSNGGACRRCTCGGATGAA-3'
5'-TTGCGACGGCGWWCCGGATTGTSSGGACGGCTCGGATGAA-3'
5'-TTGCGACGGCGWWCCGGATTGTSRGGACRRCTCGGATGAA-3'
5'-AGGCCTGCAATGACGTABGCKBTKBACAGYYTKYTTCATCCGAGYYGTCC-3'
5'-AGGCCTGCAATGACGTABGTNCGGNSSYTBYACAGYYTKYTTCATCCGAGYYGTCC-3'
5'-AGGCCTGCAATGACACTTTGTGAAATTCCGGATCCTGGCTACAGYYTKYTTCATCCGAGYYGTCC-3'
5'-AGGCCTGCAATGACAGGGAACCCGGCGGTTCAGATGCTGGCGCGCTACAGYYTKYTTCATCCGAGYYGTCC-3'
5'-AGGCCTGCAATGACGCTGCCGGTGCAGAAGTCGCACCTGGGCCCGGACGACCACAGYYTKYTTCATCCGAGYYGTCC-3'
5'-AGGCCTGCAATGACGTGCTCGGACCTGGGGTGCTAAACGGCAGAATATGAGAATCACCACAGYYTKYTTCATCCGAGYYGTCC-3'
5'-AGGCCTGCAATGACGTABGCKBTKBACAMWSCKSCGVTTCATCCGAGCCGTCC-3'
5'-AGGCCTGCAATGACGTABGTNCGGNSSYTBYACAMWSCKSCGVTTCATCCGAGCCGTCC-3'
5'-AGGCCTGCAATGACACTTTGTGAAATTCCGGATCCTGGCTACAMWSCKSCGVTTCATCCGAGCCGTCC-3'
5'-AGGCCTGCAATGACAGGGAACCCGGCGGTTCAGATGCTGGCGCGCTACAMWSCKSCGVTTCATCCGAGCCGTCC-3'
5'-AGGCCTGCAATGACGCTGCCGGTGCAGAAGTCGCACCTGGGCCCGGACGACCACAMWSCKSCGVTTCATCCGAGCCGTCC-3'
5'-AGGCCTGCAATGACGTGCTCGGACCTGGGGTGCTAAACGGCAGAATATGAGAATCACCACAMWSCKSCGVTTCATCCGAGC
    CGTCC-3'
5'-AGGCCTGCAATGACGTABGCKBTKBACAGDKWKCCRRCGVTTCATCCGAGYYGTCC-3'
5'-AGGCCTGCAATGACGTABGTNCGGNSSYTBYACAGDKWKCCRRCGVTTCATCCGAGYYGTCC-3'
5'-AGGCCTGCAATGACACTTTGTGAAATTCCGGATCCTGGCTACAGDKWKCCRRCGVTTCATCCGAGYYGTCC-3'
5'-AGGCCTGCAATGACAGGGAACCCGGCGGTTCAGATGCTGGCGCGCTACAGDKWKCCRRCGVTTCATCCGAGYYGTCC-3'
5'-AGGCCTGCAATGACGCTGCCGGTGCAGAAGTCGCACCTGGGCCCGGACGACCACAGDKWKCCRRCGVTTCATCCGAGYYGTCC-3'
5'-AGGCCTGCAATGACGTGCTCGGACCTGGGGTGCTAAACGGCAGAATATGAGAATCACCACAGDKWKCCRRCGVTTCATCCGAGYYG
    TCC-3'
5'-TGAATTTTCTGTATGAGGTTTTGCTAAACAACTTTCAACAGTTTCGGCCCCAGAGGCCTGCAATGAC-3'
(R=A/G, Y=C/T, M=A/C, K=G/T, S=C/G, W=A/T, B=C/G/T, D=A/G/T, H=A/C/T, V=A/C/G, and
N=A/C/G/T)
```

PCR fragments were digested with XmaI and SfiI. Digestion products were separated on 3% agarose gel and A domain fragments are purified from the gel. The DNA fragments were ligated into the corresponding restriction sites of phage display vector fuse5-HA, a derivative of fuse5 carrying an in-frame HA-epitope. The ligation mixture was electroporated into TransforMax™ EC100™ electrocompetent E. coli cells. Transformed E. coli cells were grown overnight at 37°C in 2xYT medium containing 20 $\mu$g/ml tetracycline and 2 mM CaCl$_2$.

Phage particles were purified from the culture medium by PEG-precipitation. Individual wells of a 96-well microtiter plate (Maxisorp) were coated with target protein (IL-6 or CD28, 1 $\mu$g/well) in 0.1 M NaHCO$_3$. After blocking the wells with TBS buffer containing 10 mg/ml cascin, purified phage was added at a typical number of ~1-3 $\times$ 10$^{11}$. The microtiter plate was incubated at 4°C for 4 hours, washed 5 times with washing buffer (TBS/Tween) and bound phages were eluted by adding glycine-HCl buffer pH 2.2. The eluate was neutralized by adding I M Tris-HCl (pH 9.1). The phage eluate was amplified using E. coli K91BlueKan cells and after purification used as input to a second and a third round of affinity selection (repeating the steps above).

Phage from the final eluate was used directly, without purification, as a template to PCR amplify A domain encoding DNA sequences. The oligonucleotides used in this PCR reaction are:

5'-aagcctcagcgaccgaa

5'-agcccaataggaacccat

The PCR products were purified and subsequently 50% was digested with BpmI and the other 50% with BsrDI.

The digested monomer fragments were 'walked' to dimers by attaching a library of naive A domain fragments using DNA ligation. Naive A domain sequences were obtained by PCR amplification of the initial A domain library (resulting from the PEG purification described above) using the A domain primers described above. The PCR fragments were purified, split into 2 equal amounts and then digested with either BpmI or BsrDI.

Digestion products were separated on a 2% agarose gel and A domain fragments were purified from the gel. The purified fragments were combined into 2 separate pools (naïve/BpmI + selected/BsrDI & naïve/BsrDI + selected/BpmI) and then ligated overnight at 16°C.

The dimeric A domain fragments were PCR amplified (5 cycles), digested with XmaI and SfiI and purified from a 2% agarose gel. Screening steps were repeated as described above except for the washing, which was done more stringently to obtain high-affinity binders. After infection, the K91BlucKan cells were plated on 2xYT agar plates containing 40 $\mu$g/ml tetracycline and grown overnight. Single colonies were picked and grown overnight in 2xYT medium containing

20 $\mu$g/ml tetracycline and 2 mM CaCl$_2$. Phage particles were purified from these cultures.

Binding of the individual phage clones to their target proteins was analyzed by ELISA. Clones yielding the highest ELISA signals were sequenced and subsequently recloned into a protein expression vector. Exemplary sequences are provided below:

>CD28-A1

```
CGPGRFQCESGQCIPATWVCDGENDCVDDSDEKSCATTAPTCLPDQFQCHDYRRCIPLGWVCDGVPDCVDNSDEA
NCEPPT
```

>CD28-A2

```
CGPGRFQCESGQCIPATWVCDGENDCVDDSDEKSCATTAPTCPPDQFTCNSGRCVPLNWLCDGVNDCADSSDEPP
ECQPRT
```

>CD28-A10

```
CGPGRFQCESGQCVPATWVCDGDDDCADGSDEKSCATTAPTCESNQFQCGSGQCLPGTWRCDGVNDCADSSDETG
CGRPGPGATSAPAACGPGRFQCNNGNCVPQTLGCDGDNDCGDSSDEANCSAPASEPPGSL
```

>CD28-A4

```
CGPGRFQCESGQCIPATWVCDGENDCVDDSDEKSCATTAPTCPANQFQCGNGRCIPPAWLCDGVNDCGDGSDESQ
LCAATGPT
```

>CD28-A5

```
CGPGRFQCESGQCIPATWVCDGENDCVDDSDEKSCATTAPTCLPNEFRCSNGQCIPPNWRCDGVDDCRDGSDEAG
CSQDPEFHKV
```

>CD28-A7

```
CGPGRFQCESGQCIPATWVCDGENDCVDDSDEKSCATTAPTCGSGQFRCSNGNCLPLRLGCDGVDDCGDSSDEPL
DPCAATVRT
```

>CD28-A17

```
CGPGRFQCESGQCIPATWVCDGENDCVDDSDEKSCATTAPTCPSGQFKCNSGRCVPPNWLCDGVNDCPDNSDEAN
CPPRT
```

>CD28-A19

```
CGPGRFQCESGQCIPATWVCDGENDCVDDSDEKSCATTAPTCQADEFQCQSSGKCLPVNWVCDGDNDCGDDSDET
NCATTGRT
```

Protein production was induced in the expression vectors with IPTG and purified by metal chelate affinity chromatography. CD28-specific monomers were characterized as follows.

*Biacore*

Two hundred fifty RU CD28 were immobilized by NHS/EDC coupling to a CM5 chip (Biacore). 0.5 and 5 $\mu$M solutions of monomer protein were flowed over the derivatized chip, and the data were analyzed using the standard Biacore software package. *See*, Table 4.

**Table 4**

| CD28 | ka | kd | KD |
|---|---|---|---|
| 4 | 5.3E+03 | 3.9E-03 | 7.4E-07 |

(continued)

| CD28 | ka | kd | KD |
|---|---|---|---|
| 5 | 1.7E+04 | 8.3E-04 | 4.8E-08 |
| 7 | 3.0E+04 | 3.2E-03 | 1.1E-07 |
| 17 | 1.4E+04 | 2.6E-03 | 1.9E-07 |
| 18 | 5.1E+02 | 2.1E-03 | 4.1E-06 |
| 19 | 1.8E+04 | 2.4E-03 | 1.3E-07 |
| 1 | 2.9E+03 | 3.9E-03 | 1.3E-06 |
| 2 | 7.4E+04 | 2.2E-03 | 3.0E-08 |
| 10 | 5.8E+04 | 1.7E-03 | 2.9E-08 |

*ELISA*

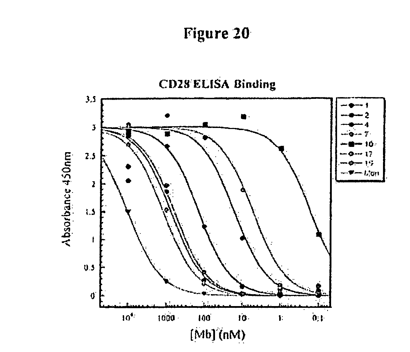

Ten nanograms of CD28 per well was immobilized by hydrophobic interaction to 96-well plates (Nunc). Plates were blocked with 5 mg/mL casein. Serial dilutions of monomer protein were added to each well and incubated for 3 hours. Plates were  washed to remove unbound protein and probed with $\alpha$-HA-HRP to detect monomers. *See*, Figure 20 and Table 5.

**Table 5**

| | Biacore | ELISA |
|---|---|---|
| 4 | 7.4E-07 | 1.4E-07 |
| 5 | 4.8E-08 | 1.0E-06 |
| 7 | 1.1E-07 | 1.2E-06 |
| 17 | 1.9E-07 | 5.4E-09 |
| 18 | 4.1E-06 | 1.0E-05 |
| 19 | 1.3E-07 | 6.3E-07 |
| 1 | 1.3E-06 | 7.8E-07 |
| 2 | 3.0E-08 | 1.6E-08 |
| 10 | 2.9E-08 | 1.7E-10 |

PBMC Assays

Efficacy assays were performed using human and monkey PBMC as described above. CD28 results in PBMC assays:
On human cells:

| CD28 monomer | clone 18 | IC50 = >1,000 nM (low activity) | |
|---|---|---|---|
| CD28 dimer | clone 7 | IC50 = 2 nM = 14 ng/ml | inhibition = 82% |
| CD28 trimer | clone 10 | IC50 = 3 nM = 40 ng/ml | inhibition = 81% |

On monkey cells:

| CD28 dimer | clone 7 | IC50= 2 nM | inhibition = 54% |
|---|---|---|---|
| CD28 trimer | clone 10 | IC50= 7 nM | inhibition = 81 % |

**Example 12**

This example describes the development of IL6-specific LDL receptor-based A domains and dimers.
A library of DNA sequences encoding monomeric A domains was created by assembly PCR as described in Stemmer et al., Gene 164:49-53 (1995). The oligonucleotides used in this PCR reaction are:

```
5'-ATTCTCACTCGGCCGACGGTGCCTACCCGT-3'
5'-ACGGTGCCTACCCGTATGATGTTCCGGATTATGCCCCGGGTCTGGAGGCGTCTGGTGGTTCGTGT-3'
5'-CGCCGTCGCAAMSCMASBBCNSTGRAABGCATNTKYYGKWAYYSYKGCATYYAAATTBGBYGRDAGVKTBACACGAACC
  ACCAGA-3'
5'-CGCCGTCGCAAMSCMASBBCNSTGRAABGCAKYKGCCGYTKYYGCATYYAAATTBGBYGRDAGVKTBACACGAACCACCAGA-3'
5'-CGCCGTCGCAAMSCMASBBCNSTGRAABGCATNTKYYGKWAYYSYKGCACBKGAACTSGYYCGVCNSACA
  CGAACCACCAGA-3'
5'-CGCCGTCGCAAMSCMASBBCNSTGRAABGCAKYKGCCGYTKYYGCACBKGAACTSGYYCGVCNSACACGAACCACCAGA-3'
5'-TTGCGACGGCGWWRATGATTGTSNGGACRRCTCGGATGAA-3'
5'-TTGCGACGGCGWWRATGATTGTSSGGACGGCTCGGATGAA-3'
5'-TTGCGACGGCGWWRATGATTGTSRGGACRRCTCGGATGAA-3'
5'-TTGCGACGGCGWWCCGGATTGTSNGGACRRCTCGGATGAA-3'
5'-TTGCGACGGCGWWCCGGATTGTSSGGACGGCTCGGATGAA-3'

5'-TTGCGACGGCGWWCCGGATTGTSRGGACRRCTCGGATGAA-3'
5'-AGGCCTGCAATGACGTABGCKBTKBACAGYYTKYTTCATCCGAGYYGTCC-3'
5'-AGGCCTGCAATGACGTABGTNCGGNSSYTBYACAGYYTKYTTCATCCGAGYYGTCC-3'
5'-AGGCCTGCAATGACACTTTGTGAAATTCCGGATCCTGGCTACAGYYTKYTTCATCCGAGYYGTCC-3'
5'-AGGCCTGCAATGACAGGGAACCCGGCGGTTCAGATGCTGGCGCGCTACAGYYTKYTTCATCCGAGYYGTCC-3'
5'-AGGCCTGCAATGACGCTGCCGGTGCAGAAGTCGCACCTGGGCCCGGACGACCACAGYYTKYTTCATCCGAGYYGTCC-3'
5'-AGGCCTGCAATGACGTGCTCGGACCTGGGGTGCTAAACGGCAGAATATGAGAATCACCACAGYYTKYTTCATCCGAGYYGTCC-3'
5'-AGGCCTGCAATGACGTABGCKBTKBACAMWSCKSCGVTTCATCCGAGCCGTCC-3'
5'-AGGCCTGCAATGACGTABGTNCGGNSSYTBYACAMWSCKSCGVTTCATCCGAGCCGTCC-3'
5'-AGGCCTGCAATGACACTTTGTGAAATTCCGGATCCTGGCTACAMWSCKSCGVTTCATCCGAGCCGTCC-3'
5'-AGGCCTGCAATGACAGGGAACCCGGCGGTTCAGATGCTGGCGCGCTACAMWSCKSCGVTTCATCCGAGCCGTCC-3'
5'-AGGCCTGCAATGACGCTGCCGGTGCAGAAGTCGCACCTGGGCCCGGACGACCACAMWSCKSCGVTTCATCCGAGCCGTCC-3'
5'-AGGCCTGCAATGACGTGCTCGGACCTGGGGTGCTAAACGGCAGAATATGAGAATCACCACAMWSCKSCGVTTCATCCGAGC
  CGTCC-3'
5'-AGGCCTGCAATGACGTABGCKBTKBACAGDKWKCCRRCGVTTCATCCGAGYYGTCC-3'
5'-AGGCCTGCAATGACGTABGTNCGGNSSYTBYACAGDKWKCCRRCGVTTCATCCGAGYYGTCC-3'
5'-AGGCCTGCAATGACACTTTGTGAAATTCCGGATCCTGGCTACAGDKWKCCRRCGVTTCATCCGAGYYGTCC-3'
5'-AGGCCTGCAATGACAGGGAACCCGGCGGTTCAGATGCTGGCGCGCTACAGDKWKCCRRCGVTTCATCCGAGYYGTCC-3'
5'-AGGCCTGCAATGACGCTGCCGGTGCAGAAGTCGCACCTGGGCCCGGACGACCACAGDKWKCCRRCGVTTCATCCGAGYYGTCC-3'
5'-AGGCCTGCAATGACGTGCTCGGACCTGGGGTGCTAAACGGCAGAATATGAGAATCACCACAGDKWKCCRRCGVTTCATCCGAGYYG
  TCC-3'
5'-TGAATTTTCTGTATGAGGTTTTGCTAAACAACTTTCAACAGTTTCGGCCCCAGAGGCCTGCAATGAC-3'
(R=A/G, Y=C/T, M=A/C, K=G/T, S=C/G, W=A/T, B=C/G/T, D=A/G/T, H=A/C/T, V=A/C/G, and
N=A/C/G/T)
```

PCR fragments were digested with XmaI and SfiI. Digestion products were separated on 3% agarose gel and A domain fragments are purified from the gel. The DNA fragments were ligated into the corresponding restriction sites of phage display vector fuse5-HA, a derivative of fuse5 carrying an in-frame HA-epitope. The ligation mixture was electroporated into TransforMax™ EC100™ electrocompetent *E. coli* cells. Transformed *E. coli* cells were grown overnight at 37°C in 2xYT medium containing 20 $\mu$g/ml tetracycline and 2 mM CaCl$_2$.

Phage particles were purified from the culture medium by PEG-precipitation. Individual wells of a 96-well microtiter plate (Maxisorp) were coated with target protein (IL-6 or CD28, 1 $\mu$g/well) in 0.1 M NaHCO$_3$. After blocking the wells with TBS buffer containing 10 mg/ml casein purified phage was added at a typical number of ~1-3 × 10$^{11}$. The microtiter plate was incubated at 4°C for 4 hours, washed 5 times with washing buffer (TBS/Tween) and bound phages were eluted by adding glycine-HCl buffer pH 2.2. The eluate was neutralized by adding I M Tris-HCl (pH 9.1). The phage eluate was amplified using *E. coli* K91BlueKan cells and after purification used as input to a second and a third round of affinity selection (repeating the steps above).

Phage from the final eluate was used directly, without purification, as a template to PCR amplify A domain encoding DNA sequences. The oligonucleotides used in this PCR reaction are:

5'-aagcctcagcgaccgaa
5'-agcccaataggaacccat

The PCR products were purified and subsequently 50% was digested with BpmI and the other 50% with BsrDI.

## Claims

1. A method for identifying a trimer that binds to a target molecule, the method comprising,
   providing a library of polypeptides, the polypeptides comprising LDL-receptor class A monomer domains, wherein the monomer domains consist of 30-100 amino acids comprising the following sequence:

   $C_aX_{3-14}C_bX_{3-7}C_cX_{4-16}C_dX_{1-2}C_eX_{8-23}C_f$;

wherein C is cysteine, $X_{n-m}$ represents between n and m number of independently selected amino acids; and wherein $C_a$-$C_c$, $C_b$-$C_e$ and $C_d$-$C_f$ form disulfide bonds;

screening the library of polypeptides for affinity to a target molecule;

identifying at least one polypeptide comprising a first LDL-receptor class A monomer domain that specifically binds to a target molecule;

linking the first LDL-receptor class A monomer domain to a plurality of additional LDL-receptor class A monomer domains as defined above to form a library of LDL-receptor class A multimers, the multimers comprising the first monomer domain and one of the plurality of additional monomer domains;

screening the library of multimers for the ability to bind to the target molecule;

identifying a multimer that specifically binds to the target molecule;

linking the identified multimer to a plurality of different monomer domains to form a library of trimers, each trimer comprising the first monomer domain and the second monomer domain of the identified multimer and one of the plurality of different monomer domains;

screening the library of trimers for the ability to bind to the target molecule; and

identifying a trimer that specifically binds to the target molecule..

2. The method of claim 1, wherein the identified trimer has an increased affinity for the target molecule compared to the affinity of the first monomer domain alone for the target molecule.

3. The method of claim 1, wherein the target molecule is a protein.

4. The method of claim 1, wherein the library of polypeptides is encoded by a library of polynucleotides and the library of polynucleotides is expressed to produce the library of polypeptides.

5. The method of claim 4, wherein the library of polypeptides comprise at least 100 different polypeptides, each of the 100 different polypeptides comprising a different monomer domain consisting of between 30-100 amino acids.

## Patentansprüche

1. Verfahren zum Identifizieren eines Trimers, das an ein Zielmolekül bindet, wobei das Verfahren Folgendes umfasst:

Bereitstellen einer Bibliothek von Polypeptiden, wobei die Polypeptide LDL-Rezeptorklasse-A-Monomerdomänen umfassen, wobei die Monomerdomänen aus 30-100 Aminosäuren mit der folgenden Sequenz:

$$C_a X_{3-14} C_b X_{3-7} C_c X_{4-16} C_d X_{1-2} C_e X_{8-23} C_f$$

bestehen,

worin C Cystein bedeutet, $X_{n-m}$ eine Anzahl zwischen n und m unabhängig ausgewählter Aminosäuren bedeutet und worin $C_a$-$C_c$, $C_b$-$C_e$ und $C_d$-$C_f$ Disulfidbrücken bilden;

Durchmustern der Polypeptidbibliothek auf Affinität für ein Zielmolekül;

Identifizieren von mindestens einem Polypeptid umfassend eine erste LDL-Rezeptorklasse-A-Monomerdomäne, die spezifisch an ein Zielmolekül bindet;

Verknüpfen der ersten LDL-Rezeptorklasse-A-Monomerdomäne mit einer Mehrzahl von zusätzlichen LDL-Rezeptorklasse-A-Monomerdomänen wie oben definiert, wodurch eine Bibliothek von LDL-Rezeptorklasse-A-Multimeren gebildet wird, wobei die Multimere die erste Monomerdomäne und eine der Mehrzahl von zusätzlichen Monomerdomänen umfassen;

Durchmustern der Multimerbibliothek auf die Fähigkeit, an das Zielmolekül zu binden;

Identifizieren eines Multimers, das spezifisch an das Zielmolekül bindet;

Verknüpfen des identifizierten Multimers mit einer Mehrzahl von unterschiedlichen Monomerdomänen, wodurch eine Trimerbibliothek gebildet wird, wobei jedes Trimer die erste Monomerdomäne und die zweite Monomerdomäne des identifizierten Multimers und eine der Mehrzahl von unterschiedlichen Monomerdomänen umfasst;

Durchmustern der Trimerbibliothek auf die Fähigkeit, an das Zielmolekül zu binden; und

Identifizieren eines Trimers, das spezifisch an das Zielmolekül bindet.

2. Verfahren nach Anspruch 1, wobei das identifizierte Trimer eine erhöhte Affinität für das Zielmolekül im Vergleich zu der Affinität für das Zielmolekül der ersten Monomerdomäne allein aufweist.

**3.** Verfahren nach Anspruch 1, wobei es sich bei dem Zielmolekül um ein Protein handelt.

**4.** Verfahren nach Anspruch 1, wobei die Polypeptidbibliothek von einer Polynukleotidbibliothek codiert wird und die Polynukleotidbibliothek so exprimiert wird, dass die Polypeptidbibliothek entsteht.

**5.** Verfahren nach Anspruch 4, wobei die Polypeptidbibliothek mindestens 100 unterschiedliche Polypeptide umfasst, wobei jedes der 100 unterschiedlichen Polypeptide eine unterschiedliche Monomerdomäne, die aus zwischen 30-100 Aminosäuren besteht, umfasst.

**Revendications**

**1.** Procédé pour identifier un trimère qui se lie à une molécule cible, le procédé comprenant,
la fourniture d'une banque de polypeptides, les polypeptides comprenant des domaines de monomère de récepteur LDL de classe A, les domaines de monomère étant constitués de 30 à 100 acides aminés comprenant la séquence suivante :

$$C_aX_{3-14}C_bX_{3-7}C_cX_{4-16}C_dX_{1-2}C_eX_{8-23}C_f \; ;$$

où C est la cystéine, $X_{n-m}$ représente un nombre compris entre n et m d'acides aminés indépendamment choisis ; et où $C_a$-$C_c$, $C_b$-$C_e$ et $C_d$-$C_f$ forment des liaisons disulfure ;
le criblage de la banque de polypeptides pour l'affinité pour une molécule cible ;
l'identification d'au moins un polypeptide comprenant un premier domaine de monomère de récepteur LDL de classe A qui se lie spécifiquement à une molécule cible ;
la liaison du premier domaine de monomère de récepteur LDL de classe A à une pluralité de domaines de monomère de récepteur LDL de classe A additionnels tels que définis ci-dessus pour former une banque de multimères de récepteur LDL de classe A, les multimères comprenant le première domaine de monomère et l'un de la pluralité de domaines de monomère additionnels ;
le criblage de la banque de multimères pour la capacité à se lier à la molécule cible ;
l'identification d'un multimère qui se lie spécifiquement à la molécule cible ;
la liaison du multimère identifié à une pluralité de domaines de monomère différents pour former une banque de trimères, chaque trimère comprenant le premier domaine de monomère et le deuxième domaine de monomère du multimère identifié et l'un de la pluralité de domaines de monomère différents ;
le criblage de la banque de trimères pour la capacité à se lier à la molécule cible ; et
l'identification d'un trimère qui se lie spécifiquement à la molécule cible.

**2.** Procédé de la revendication 1, dans lequel le trimère identifié a une affinité augmentée pour la molécule cible comparée à l'affinité du premier domaine de monomère seul pour la molécule cible.

**3.** Procédé de la revendication 1, la molécule cible étant une protéine.

**4.** Procédé de la revendication 1, la banque de polypeptides étant codée par une banque de polynucléotides et la banque de polynucléotides étant exprimée pour produire la banque de polypeptides.

**5.** Procédé de la revendication 4, la banque de polypeptides comprenant au moins 100 polypeptides différents, chacun des 100 polypeptides différents comprenant un domaine de monomère différent comprenant entre 30 et 100 acides aminés.

# Figure 1

EP 2 198 890 B1

## Figure 2
## LDL-receptor class A domain

```
LRP1_HUMAN    C.EPYQFRCKNNR.....CVPGRWQ.CDYDNDCGDNSDEES....C

LRP1_HUMAN    C.LPSQFKCTNTNR....CIPGIFR.CNGQDNCGDGEDERD....C

LDLR_HUMAN    C.SQDEFRCHDGK.....CISRQFV.CDSDRDCLDGSDEAS....C

LRP2_HUMAN    C.SSSAFTCGHGE.....CIPAHWR.CDKRNDCVDGSDEHN....C

LRP2_HUMAN    C.SSSEFQCASGR.....CIPQHWY.CDQETDCFDASDEPAS...C

CORI_HUMAN    CHSQGLVECRNGQ.....CIPSTFQ.CDGDEDCKDGSDEEN....C

MAT_HUMAN     C.PAQTFRCSNGK.....CLSKSQQ.CNGKDDCGDGSDEAS....C

CO8B_HUMAN    C...EGFVCAQTGR....CVNRRLL.CNGDNDCGDQSDEAN....C

MAT_HUMAN     C.TKHTYRCLNGL.....CLSKGNPECDGKEDCSDGSDEKD....C

LDVR_HUMAN    CLGPGKFKCRSGE.....CIDISKV.CNQEQDCRDWSDEPLKE..C

APOER2_HUM    C.PAEKLSCGPTSHK...CVPASWR.CDGEKDCEGGADEAG....C

SORL_HUMAN    CTHFMDFVCKNRQQ....CLFHSMV.CDGIIQCRDGSDEDAAFAGC

ST7_HUMAN     C.AYNQFQCLSRFTKVYTCLPESLK.CDGNIDCLDLGDEID....C


consensus     C.1234F6C12G4.....CI23456.CDG34DC1D3SDE78....C
```

EP 2 198 890 B1

# FIGURE 3

# **A-domains**

## A

~ 40 amino acids

C 1 2 3 4 (H) 6 C 1 2 3 4 C (H) 2 3 4 (H) 6 C (−) 2 3 4 (−) C 1 2 3 4 (−) (−) 7 8 C

## B

# Figure 4

## Ligands recognized by LDL-receptor family

*proteases*
factor IXa
pro-uPA
t-PA
plasminogen
MMP-9

*inhibitors*
$\alpha_2$-macroglobulin
PAI-1
TFPI
pancreatic trypsin inhibitor

*complexes*
protease/
$\alpha_1$-antitrypsin
protein C inhibitor
protease nexin-1
antithrombin
C1-inhibitor
thrombin/heparin cofactor II
cathepsin G/$\alpha_1$-antichymotrypsin

*vitamin-carrier complexes*
vitamin D-bp, vitamin D
retinol-bp, vitamin A
transcobalamin, vitamin B12

*proteins involved in lipoprotein metabolism*
apoB100
apoE
apoJ (clusterin)
apoH ($\beta_2$-glycoprotein I)
Lp(a)
hepatic lipase
lipoprotein lipase
IDL
VLDL
$\beta$-VLDL

*non-human*
pseudomonas exotoxin A
circumsporozoite protein
trichosanthin
ricin A
saporin

*antibiotics*
gentamicin
polymyxin B

*viruses*
HRV2 (Rhino)
HCV (Flavi)
BVDV (Flavi)

*miscellaneous*
albumin
transthyretin
$\beta$-Amyloid precursor protein
RAP
complement C3
lactoferrin
thyroglobulin
thrombospondin
saposin precursor
reelin
insulin
parathyroid hormone (PTH)
aprotinin
$\alpha$-amylase
C1q
$\alpha_1$-microglobulin
$\beta_2$-microglobulin
odorant-binding protein
epidermal growth factor
prolactin
lysozyme
connective tissue growth factor (CTGF)
cytochrome c
seminal vesicle secretory protein II
clara cell secretory protein (CCSP)
cubulin
factor VIII

Figure 5

EP 2 198 890 B1

Figure 6

# Figure 7

1st selection

Phage with monomer hits against a target

Ag

2nd selection

Phage with potential Multimer hits

PCR with stopper seq. primer and cloning sites

Phagemid

1) PCR with primers containing restriction sites allowing for unidirectional ligation
2) Cut and clean

stopper
stopper
linker

Mix with linkers And stoppers

stopper        linker        linker        stopper

Run gel and cut the suitable multimeric form

Increasing [stopper]

EP 2 198 890 B1

## Figure 8

## FIG. 9A

| | 1 | 2 | 3 | 4 | 5 | 6 | 7 | 8 | 9 | 10 | 11 | 12 | 13 | 14 | 15 | 16 | 17 |
|---|---|---|---|---|---|---|---|---|---|---|---|---|---|---|---|---|---|
| A | 0.0 | 7.1 | 12.3 | 3.2 | 1.9 | 1.9 | 7.1 | 0.0 | 15.8 | 1.5 | 0.0 | 1.5 | 0.0 | 1.0 | 3.7 | 7.3 | 9.4 |
| C | 100.0 | 0.0 | 0.6 | 0.0 | 0.0 | 0.0 | 0.0 | 100.0 | 0.0 | 0.0 | 0.0 | 1.5 | 99.5 | 0.0 | 0.0 | 0.0 | 0.0 |
| D | 0.0 | 5.2 | 4.5 | 19.5 | 2.6 | 0.0 | 0.0 | 0.0 | 8.3 | 10.5 | 2.3 | 0.8 | 0.0 | 0.0 | 3.7 | 1.6 | 4.7 |
| E | 0.0 | 4.5 | 9.7 | 2.6 | 37.0 | 0.0 | 3.9 | 0.0 | 3.8 | 0.0 | 0.8 | 9.8 | 0.0 | 0.0 | 1.6 | 7.3 | 7.3 |
| F | 0.0 | 0.6 | 0.0 | 3.9 | 1.9 | 76.0 | 0.6 | 0.0 | 0.8 | 0.0 | 0.0 | 0.8 | 0.0 | 0.5 | 1.6 | 2.1 | 2.6 |
| G | 0.0 | 13.0 | 3.2 | 16.2 | 1.9 | 0.0 | 0.0 | 0.0 | 12.8 | 0.8 | 72.2 | 2.3 | 0.0 | 0.0 | 3.1 | 5.2 | 4.2 |
| H | 0.0 | 1.9 | 1.3 | 5.2 | 3.9 | 1.9 | 3.9 | 0.0 | 3.0 | 0.8 | 4.5 | 9.0 | 0.0 | 0.0 | 4.7 | 3.1 | 5.8 |
| I | 0.0 | 0.0 | 1.3 | 0.0 | 0.0 | 3.9 | 0.6 | 0.0 | 0.0 | 0.0 | 0.0 | 2.3 | 0.0 | 63.9 | 0.0 | 5.2 | 3.7 |
| K | 0.0 | 3.9 | 3.9 | 1.9 | 1.9 | 0.6 | 7.8 | 0.0 | 11.3 | 0.0 | 3.0 | 9.0 | 0.0 | 2.1 | 2.1 | 9.9 | 3.7 |
| L | 0.0 | 8.4 | 4.5 | 0.0 | 1.3 | 3.9 | 3.9 | 0.0 | 1.5 | 0.0 | 1.5 | 4.5 | 0.0 | 11.0 | 5.2 | 12.0 | 3.1 |
| M | 0.0 | 0.6 | 0.6 | 0.0 | 1.3 | 0.6 | 1.9 | 0.0 | 0.0 | 0.0 | 0.0 | 0.8 | 0.0 | 0.0 | 0.5 | 3.1 | 1.6 |
| N | 0.0 | 1.9 | 0.6 | 13.6 | 1.3 | 0.0 | 1.9 | 0.0 | 5.3 | 51.9 | 5.3 | 3.8 | 0.0 | 0.0 | 5.2 | 4.2 | 2.6 |
| P | 0.0 | 10.4 | 33.8 | 1.3 | 0.0 | 0.0 | 9.7 | 0.0 | 4.5 | 0.0 | 0.0 | 0.0 | 0.0 | 0.0 | 45.5 | 7.3 | 2.1 |
| Q | 0.0 | 10.4 | 2.6 | 1.9 | 30.5 | 0.6 | 21.4 | 0.0 | 5.3 | 0.8 | 2.3 | 9.8 | 0.0 | 0.0 | 2.6 | 5.8 | 4.7 |
| R | 0.0 | 7.1 | 3.2 | 3.2 | 1.3 | 0.6 | 18.2 | 0.0 | 7.5 | 0.8 | 4.5 | 32.3 | 0.0 | 0.0 | 1.0 | 9.4 | 11.5 |
| S | 0.0 | 18.8 | 11.7 | 16.2 | 3.9 | 0.6 | 9.7 | 0.0 | 15.0 | 25.6 | 3.0 | 3.0 | 0.0 | 0.0 | 14.7 | 10.5 | 20.4 |
| T | 0.0 | 5.2 | 2.6 | 5.8 | 3.2 | 1.3 | 7.8 | 0.0 | 2.3 | 6.0 | 0.8 | 6.0 | 0.0 | 1.6 | 2.6 | 0.5 | 5.8 |
| V | 0.0 | 0.6 | 1.9 | 0.6 | 0.0 | 0.6 | 1.3 | 0.0 | 2.3 | 0.8 | 0.0 | 3.0 | 0.0 | 17.3 | 0.5 | 3.1 | 2.6 |
| W | 0.0 | 0.0 | 0.6 | 1.9 | 0.0 | 1.9 | 0.0 | 0.0 | 0.0 | 0.0 | 0.0 | 0.0 | 0.0 | 0.0 | 0.5 | 0.0 | 2.6 |
| Y | 0.0 | 0.0 | 0.6 | 2.6 | 5.8 | 5.2 | 0.0 | 0.0 | 0.8 | 0.8 | 0.0 | 0.0 | 0.0 | 0.5 | 2.6 | 1.0 | 1.6 |
| | | | | | | | | | | | | | | | | | |
| | 100.0 | 85.7 | 67.5 | 76.6 | 73.4 | 81.2 | 81.8 | 100.0 | 81.2 | 94.0 | 77.4 | 75.9 | 99.5 | 92.1 | 70.7 | 80.1 | 60.2 |
| | 1 | 9 | 4 | 6 | 3 | 2 | 7 | 1 | 8 | 4 | 2 | 6 | 1 | 3 | 4 | 10 | 6 |
| | 100.0 | 73.4 | 67.5 | 65.6 | 67.5 | 76.0 | 66.2 | 100.0 | 68.4 | 77.4 | 72.2 | 60.9 | 99.5 | 63.9 | 60.2 | 60.2 | 59.2 |
| | 1 | 7 | 4 | 4 | 2 | 1 | 5 | 1 | 6 | 2 | 1 | 4 | 1 | 1 | 2 | 7 | 6 |

EP 2 198 890 B1

## FIG. 9B

| 18 | 19 | 20 | 21 | 22 | 23 | 24 | 25 | 26 | 27 | 28 | 29 | 30 | 31 | 32 | 33 | 34 | 35 |
|---|---|---|---|---|---|---|---|---|---|---|---|---|---|---|---|---|---|
| 1.6 | 2.6 | 0.0 | 0.0 | 1.0 | 2.5 | 5.6 | 0.0 | 0.0 | 6.9 | 0.0 | 4.6 | 4.6 | 0.0 | 0.0 | 16.0 | 3.8 | 0.0 |
| 0.0 | 0.0 | 100.0 | 0.0 | 0.0 | 0.0 | 0.0 | 0.0 | 100.0 | 0.0 | 0.0 | 0.0 | 0.0 | 0.0 | 0.0 | 0.0 | 0.0 | 100.0 |
| 1.0 | 0.0 | 0.0 | 81.2 | 1.5 | 28.4 | 22.3 | 86.3 | 0.0 | 3.8 | 89.3 | 1.5 | 0.0 | 100.0 | 0.0 | 6.9 | 21.4 | 0.0 |
| 1.0 | 1.6 | 0.0 | 0.0 | 2.0 | 15.7 | 4.6 | 3.6 | 0.0 | 9.9 | 0.0 | 2.3 | 6.9 | 0.0 | 100.0 | 16.8 | 0.8 | 0.0 |
| 7.9 | 0.5 | 0.0 | 0.0 | 0.5 | 4.6 | 3.0 | 0.0 | 0.0 | 0.0 | 0.0 | 0.8 | 0.0 | 0.0 | 0.0 | 1.5 | 0.8 | 0.0 |
| 1.0 | 0.0 | 0.0 | 0.0 | 66.0 | 0.5 | 0.0 | 0.0 | 0.0 | 30.5 | 1.5 | 48.9 | 1.5 | 0.0 | 0.0 | 0.8 | 22.9 | 0.0 |
| 0.0 | 2.6 | 0.0 | 0.0 | 3.6 | 4.1 | 1.5 | 3.6 | 0.0 | 0.8 | 0.8 | 2.3 | 0.0 | 0.0 | 0.0 | 2.3 | 6.1 | 0.0 |
| 0.0 | 4.7 | 0.0 | 0.0 | 0.0 | 4.6 | 2.0 | 0.0 | 0.0 | 1.5 | 0.0 | 0.0 | 0.0 | 0.0 | 0.0 | 3.1 | 0.8 | 0.0 |
| 6.8 | 12.6 | 0.0 | 0.0 | 1.0 | 2.5 | 5.1 | 0.0 | 0.0 | 6.1 | 0.0 | 3.8 | 0.0 | 0.0 | 0.0 | 12.2 | 0.8 | 0.0 |
| 14.7 | 15.2 | 0.0 | 0.0 | 1.5 | 2.0 | 3.0 | 0.0 | 0.0 | 6.9 | 0.0 | 1.5 | 0.8 | 0.0 | 0.0 | 9.9 | 0.8 | 0.0 |
| 0.5 | 1.0 | 0.0 | 0.0 | 1.5 | 1.0 | 0.0 | 0.0 | 0.0 | 1.5 | 0.0 | 3.1 | 0.8 | 0.0 | 0.0 | 1.5 | 0.8 | 0.0 |
| 2.1 | 0.0 | 0.0 | 18.8 | 3.6 | 2.0 | 21.3 | 4.1 | 0.0 | 0.0 | 4.6 | 14.5 | 0.0 | 0.0 | 0.0 | 1.5 | 33.6 | 0.0 |
| 0.0 | 0.5 | 0.0 | 0.0 | 0.0 | 0.0 | 10.7 | 0.0 | 0.0 | 9.9 | 0.0 | 0.0 | 0.0 | 0.0 | 0.0 | 0.0 | 0.8 | 0.0 |
| 5.8 | 7.3 | 0.0 | 0.0 | 4.1 | 10.7 | 4.6 | 1.5 | 0.0 | 5.3 | 0.8 | 3.1 | 0.0 | 0.0 | 0.0 | 6.1 | 0.8 | 0.0 |
| 3.7 | 24.1 | 0.0 | 0.0 | 5.6 | 2.5 | 6.1 | 0.0 | 0.0 | 3.8 | 0.0 | 2.3 | 3.1 | 0.0 | 0.0 | 10.7 | 1.5 | 0.0 |
| 1.0 | 0.0 | 0.0 | 0.0 | 3.0 | 2.0 | 2.0 | 0.5 | 0.0 | 7.6 | 1.5 | 4.6 | 80.9 | 0.0 | 0.0 | 0.8 | 4.6 | 0.0 |
| 0.5 | 2.6 | 0.0 | 0.0 | 2.5 | 1.5 | 2.0 | 0.5 | 0.0 | 0.8 | 0.8 | 0.0 | 1.5 | 0.0 | 0.0 | 2.3 | 0.0 | 0.0 |
| 1.0 | 21.5 | 0.0 | 0.0 | 0.5 | 10.2 | 3.6 | 0.0 | 0.0 | 4.6 | 0.0 | 0.0 | 0.0 | 0.0 | 0.0 | 6.1 | 0.0 | 0.0 |
| 46.6 | 2.6 | 0.0 | 0.0 | 0.5 | 2.0 | 1.5 | 0.0 | 0.0 | 0.0 | 0.0 | 3.1 | 0.0 | 0.0 | 0.0 | 0.8 | 0.0 | 0.0 |
| 4.7 | 0.5 | 0.0 | 0.0 | 1.5 | 3.0 | 1.0 | 0.0 | 0.0 | 0.0 | 0.8 | 3.8 | 0.0 | 0.0 | 0.0 | 0.8 | 0.0 | 0.0 |
| | | | | | | | | | | | | | | | | | |
| 81.7 | 80.6 | 100.0 | 100.0 | 71.6 | 65.0 | 71.1 | 86.3 | 100.0 | 83.2 | 89.3 | 63.4 | 87.8 | 100.0 | 100.0 | 84.7 | 84.0 | 100.0 |
| 5 | 5 | 1 | 2 | 2 | 4 | 6 | 1 | 1 | 8 | 1 | 2 | 2 | 1 | 1 | 8 | 4 | 1 |
| 61.3 | 60.7 | 100.0 | 81.2 | 66.0 | 65.0 | 60.4 | 86.3 | 100.0 | 64.1 | 89.3 | 63.4 | 80.9 | 100.0 | 100.0 | 61.1 | 77.9 | 100.0 |
| 2 | 3 | 1 | 1 | 1 | 4 | 4 | 1 | 1 | 5 | 1 | 2 | 1 | 1 | 1 | 5 | 3 | 1 |

EP 2 198 890 B1

# Figure 10

```
                  a            b  c                    de   f  ghi  jk  lm          nop         q
IDD_HUMAN       C........NPGQFACRSGTIQ........CIPLPWQ.CDGWATCEDE......SDEAN......C
LRP3_HUMAN      C........QADEFRCDNGK.........CLPGPWQ.CNTVDECGDG......SDEGN......C
LRP3_HUMAN      C........PGGTFPCSGARSTR.......CLPVERR.CDGLQDCGDG......SDEAG......C
LRP3_HUMAN      C........LPWEQPCGSSSDSDGGSLGDQGCFSEPQR.CDGWWHCASG......RDEQG......C
LRP3_HUMAN      C........PPDQYPCEGGSGL........CYTPADR.CNNQKSCPDG......ADEKN......C
LRP3_HUMAN      C........QPGTFHCGTNL..........CIFETWR.CDGQEDCQDG......SDEHG......C
LRP5_HUMAN      C........SPDQFACATGEID........CIPGAWR.CDGFPECDDQ......SDEEG......C
LRP5_HUMAN      C........SAAQFPCARGQ..........CVDLRLR.CDGEADCQDR......SDEVD......C
LRP5_HUMAN      C........LPNQFRCASGQ..........CVLIKQQ.CDSFPDCIDG......SDELM......C
LRP6_HUMAN      C........SPQQFTCFTGEID........CIPVAWR.CDGFTECEDH......SDELN......C
LRP6_HUMAN      C........SESQFQCASGQ..........CIDGALR.CNGDANCQDK......SDEKN......C
LRP6_HUMAN      C........LIDQFRCANGQ..........CIGKHKK.CDHNVDCSDK......SDELD......C
ST7_HUMAN       C........ACDQFRCGNGK..........CIPEAWK.CNNMDECGDS......SDEEI......C
ST7_HUMAN       C........AYNQFQCLSRFTKVYT.....CLPESLK.CDGNIDCLDL......GDEID......C
ST7_HUMAN       C........LPWEIPCGGNWG.........CYTEQQR.CDGYWHCPNG......RDETN......C
ST7_HUMAN       C........QKEEFPCSRNGV.........CYPRSDR.CNYQNHCPNG......SDEKN......C
ST7_HUMAN       C........QPGNFHCKNNR..........CVFESWV.CDSQDDCGDG......SDEEN......C
CORI_HUMAN      C........GRGENFLCASGI.........CIPGKLQ.CNGYNDCDDW......SDEAH......C
CORI_HUMAN      C........SENLFHCHTGK..........CLNYSLV.CDGYDDCGDL......SDEQN......C
CORI_HUMAN      C........NPTTEHRCGDGR.........CIAMEWV.CDGDHDCVDK......SDEVN......C
CORI_HUMAN      C........HSQGLVECRNGQ.........CIPSTFQ.CDGDEDCKDG......SDEEN......C
CORI_HUMAN      C........SPSHFKCRSGQ..........CVLASRR.CDGQADCDDD......SDEEN......C
CORI_HUMAN      C........KERDLWECPSNKQ........CLKHTVI.CDGFPDCPDY......MDEKN......C
CORI_HUMAN      C........QDDELECANHA..........CVSRDLW.CDGEADCSDS......SDEWD......C
TMS2_HUMAN      C........SNSGIECDSSGT.........CINPSNW.CDGVSHCPGG......EDENR......C
TMS3_HUMAN      C........SGKYRCRSSFK..........CIELIAR.CDGVSDCKDG......EDEYR......C
MAT_HUMAN       C........PGQFTCRTGR...........CIRKELR.CDGWADCTDH......SDELN......C
MAT_HUMAN       C........DAGHQFTCKNKF.........CKPLFWV.CDSVNDCGDN......SDEQG......C
MAT_HUMAN       C........PAQTFRCSNGK..........CLSKSQQ.CNGKDDCGDG......SDEAS......C
MAT_HUMAN       C........TKHTYRCLNGL..........CLSKGNPECDGKEDCSDG......SDEKD......C
ENTK_HUMAN      C........LPGSSPCTDALT.........CIKADLF.CDGEVNCPDG......SDEDNKM....C
ENTK_HUMAN      C........KADHFQCKNGE..........CVPLVNL.CDGHLHCEDG......SDEAD......C
HAI1_HUMAN      C........QPTQFRCSNGC..........CIDSFLE.CDDTPNCPDA......SDEAA......C
CFAI_HUMAN      C.YTQKADSPMDDFFQCVNGK.........YISQMKA.CDGINDCGDQ......SDEL.......C
CFAI_HUMAN      C........QGKGFHCKSGV..........CIPSQYQ.CNGEVDCITG......EDEVG......C
CO6_HUMAN       C........KNKFRCDSGR...........CIARKLE.CNGENDCGDN......SDERD......C
CO7_HUMAN       C........GERFRCFSGQ...........CISKSLV.CNGDSDCDEDS.....ADEDR......C
CO8A_HUMAN      C........GQDFQCKETGR..........CLKRHLV.CNGDQDCLDG......SDEDD......C
CO8B_HUMAN      C........EGPVCAQTGR...........CVNRRLL.CNGDNDCGDQ......SDEAN......C
CO9_HUMAN       C........GNDFQCSTGR...........CIKMRLR.CNGDNDCGDF......SDEDD......C
PERL_HUMAN      C........TEAEFACHSYNE.........CVALEYR.CDRRPDCRDM......SDELN......C
PERL_HUMAN      C........GPQEAACRNGH..........CIPRDYL.CDGQEDCEDG......SDELD......C
PERL_HUMAN      C........EPNEFPCGNGH..........CALKLWR.CDGDFDCEDR......TDEAN......C
PERL_HUMAN      C........GPTQFRCVSTNM.........CIPASFH.CDEESDCPDR......SDEFG......C
SORL_HUMAN      C........LRNQYRCSNGN..........CINSIWW.CDFDNDCGDM......SDERN......C
SORL_HUMAN      C........DLDTQFRCQESGT........CIPLSYK.CDLEDDCGDN......SDESH......C
SORL_HUMAN      C........RSDEYNCSSGM..........CIRSSWV.CDGDNDCRDW......SDEAN......C
SORL_HUMAN      C........EASNFQCRNGH..........CIPQRWA.CDGDTDCQDG......SDEDPVN....C
SORL_HUMAN      C........NGFRCPNGT...........CIPSSKH.CDGLRDCSDG......SDEQH......C
SORL_HUMAN      C........THFMDFVCKNRQQ........CLFHSMV.CDGIIQCRDG......SDEDAAFAG..C
SORL_HUMAN      C........DEFGFQCQNGV..........CISLIWK.CDGMDDCGDY......SDEAN......C
SORL_HUMAN      C........SRYFQFRCENGH.........CIPNRWK.CDRENDCGDW......SDEKD......C
SORL_HUMAN      C........LPNYYRCSSGT..........CVMDTWV.CDGYRDCADG......SDEEA......C
SORL_HUMAN      C........DRFEFECHQPKT.........CIPNWKR.CDGHQDCQDG......RDEAN......C
SORL_HUMAN      C........MSREFQCEDGEA.........CIVLSER.CDGFLDCSDG......SDEKA......C
APOER2_HUM      C........EKDQFQCRHER..........CIPSVWR.CDEDDDCLDH......SDEDD......C
APOER2_HUM      C........ADSDFTCDNGH..........CIHERWK.CDGEEECPDG......SDESEAT....C
APOER2_HUM      C........PAEKLSCGPTSHK........CVPASWR.CDGEKDCEGG......ADEAG......C
APOER2_HUM      C........APHEFQCGNRS..........CLAAVFV.CDGDDDCGDG......SDERG......C
APOER2_HUM      C........GPREFRCGGDGGGA.......CIPERWV.CDRQPDCEDR......SDEAAEL....C
APOER2_HUM      C........ATVSQFACRSGE.........CVHLGWR.CDGDRDCKDK......SDEAD......C
APOER2_HUM      C........RGDEFQCGDGT..........CVLAIKH.CNQEQDCPDG......SDEAG......C
LDLR_HUMAN      C........ERNEFQCQDGK..........CISYKWV.CDGSAECQDG......SDESQET....C
LDLR_HUMAN      C........KSGDFSCGGRVNR........CIPQFWR.CDGQVDCDNG......SDEQG......C
LDLR_HUMAN      C........SQDEFRCHDGK..........CISRQFV.COSDRDCLDG......SDEAS......C
LDLR_HUMAN      C........GPASFQCNSST..........CIPQLWA.CDNDPDCEDG......SDEWPQR....C
LDLR_HUMAN      C........SAFEFHCLSGE..........CIHSSWR.CDGGPDCKDK......SDEEN......C
LDLR_HUMAN      C........RPDEFQCSDGN..........CIHGSRQ.CDREYDCKDM......SDEVG......C
LDLR_HUMAN      C........EGPNKFKCHSGE.........CITLDKV.CNMARDCRDW......SDEPIKE....C
LDVR_HUMAN      C........EPSQFQCTNGR..........CITLLWK.CDGDEDCVDG......SDEKN......C
LDVR_HUMAN      C........AESDFVCNNGQ..........CVPSRWK.CDGDPDCEDG......SDESPEQ....C
```

72

FIG. 10 (CONT)

```
LDVR_HUMAN  C..........RIHEISCGAHSTQ.........CIPVSWR.CDGENDCDSG......EDEEN......C
LDVR_HUMAN  C..........SPDEFTCSSGR..........CISRNFV.CNGQDDCSDG......SDELD......C
LDVR_HUMAN  C..........GAHEFQCSTSS..........CIPISWV.CDDDADCSDQ......SDESLEQ....C
LDVR_HUMAN  C..........PASEIQCGSGE..........CIHKKWR.CDGDPDCKDG......SDEVN......C
LDVR_HUMAN  C..........RPDQFECEDGS..........CIHGSRQ.CNGIRDCVDG......SDEVN......C
LDVR_HUMAN  C..........LGPGKFKCRSGE.........CIDISKV.CNQEQDCRDW......SDEPLKE....C
LRP1_HUMAN  C..........SPKQFACRDQIT.........CISKGWR.CDGERDCPDG......SDEAPEI....C
LRP1_HUMAN  C..........QPNEHNCLGTEL.........CVPMSRL.CNGVQDCMDG......SDEGPH.....C
LRP1_HUMAN  C..........QPGEFACANSR..........CIQERWK.CDGDNDCLDN......SDEAPAL....C
LRP1_HUMAN  C..........PSDRFKCENNR..........CIPNRWL.CDGDNDCGNS......EDESNAT....C
LRP1_HUMAN  C..........PPNQFSCASGR..........CIPISWT.CDLDDDCGDR......SDESAS.....C
LRP1_HUMAN  C..........FPLTQFTCNNGR.........CININWR.CDNDNDCGDN......SDEAG......C
LRP1_HUMAN  C..........SSTQFKCNSGR..........CIPEHWT.CDGDNDCGDY......SDETHAN....C
LRP1_HUMAN  C..........HTDEFQCRLDGL.........CIPLRWR.CDGDTDCMDS......SDEKS......C
LRP1_HUMAN  C..........DPSVKFGCKDSAR........CISKAWV.CDGDNDCEDN......SDEEN......C
LRP1_HUMAN  C..........RPPSHPCANNTSV........CLPPDKL.CDGNDDCGDG......SDEGEL.....C
LRP1_HUMAN  C..........RAQDEFECANGE.........CINFSLT.CDGVPHCKDK......SDEKPSY....C
LRP1_HUMAN  C..........KKTFRQCSNGR..........CVSNMLW.CNGADDCGDG......SDEIP......C
LRP1_HUMAN  C..........GVGEFRCRDGT..........CIGNSSR.CNQFVDCEDA......SDEMN......C
LRP1_HUMAN  CSSYFRLGVKGVLFQPCERTSL.........CYAPSWV.CDGANDCGDY......SDERD......C
LRP1_HUMAN  C..........PLNYFACPSGR..........CIPMSWT.CDKEDDCEHG......EDETH......C
LRP1_HUMAN  C..........SEAQFECQNHR..........CISKQWL.CDGSDDCGDG......SDEAAH.....C
LRP1_HUMAN  C..........GPSSFSCPGTHV.........CVPERWL.CDGDKDCADG......ADESIAAG...C
LRP1_HUMAN  C..........DDREFMCQNRQ..........CIPKHPV.CDHDRDCADG......SDESPE.....C
LRP1_HUMAN  C..........GPSEFRCANGR..........CLSSRQWECDGENDCHDQ......SDEAPKNPH..C
LRP1_HUMAN  C..........NASSQFLCSSGR.........CVAEALL.CNGQDDCGDS......SDERG......C
LRP1_HUMAN  C..........TASQFVCKNDK..........CIPFWWK.CDTEDDCGDH......SDEPPD.....C
LRP1_HUMAN  C..........RPGQFQCSTGI..........CTNPAFI.CDGDNDCQDN......SDEAN......C
LRP1_HUMAN  C..........LPSQFKCTNTNR.........CIPGIFR.CNGQDNCGDG......EDERD......C
LRP1_HUMAN  C..........APNQFQCSITKR.........CIPRVWV.CDRDNDCVDG......SDEPAN.....C
LRP1_HUMAN  C..........GVDEFRCKDSGR.........CIPARWK.CDGEDDCGDG......SDEPKEE....C
LRP1_HUMAN  C..........EPYQFRCKNNR..........CVPGRWQ.CDYDNDCGDN......SDEES......C
LRP1_HUMAN  C..........SESEFSCANGR..........CIAGRWK.CDGDHDCADG......SDEKD......C
LRP1_HUMAN  C..........DMDQFQCKSGH..........CIPLRWR.CDADADCMDG......SDEEA......C
LRP1_HUMAN  C..........PLDEFQCNNTL..........CKPLAWK.CDGEDDCGDN......SDENPEE....C
LRP1_HUMAN  C..........PPNRPFRCKNDRV........CLWIGRQ.CDGTDNCGDG......TDEED......C
LRP1_HUMAN  C..........KDKKEFLCRNQR.........CLSSSLR.CNMFDDCGDG......SDEED......C
LRP2_HUMAN  C..........DSAHFRCGSGH..........CIPADWR.CDGTKDCSDD......ADEIG......C
LRP2_HUMAN  C..........QQGYFKCQSEGQ.........CIPSSWV.CDQDQDCGDG......SDERQD.....C
LRP2_HUMAN  C..........SSHQITCSNGQ..........CIPSEYR.CDHVRDCPDG......ADEND......C
LRP2_HUMAN  C..........EQLTCDNGA............CYNTSQK.CDWKVDCRDS......SDEIN......C
LRP2_HUMAN  C..........LHNEFSCGNGE..........CIPRAYV.CDHDNDCQDG......SDEHA......C
LRP2_HUMAN  C..........GGYQFTCPSGR..........CIYQNWV.CDGEDDCKDN......GDEDG......C
LRP2_HUMAN  C..........SPREWSCPESGR.........CISIYKV.CDGILDCPGR......EDENNTSTGKYC
LRP2_HUMAN  C..........GLFSFPCKNGR..........CVPNYYL.CDGVDDCHDN......SDEQL......C
LRP2_HUMAN  C..........SSSAFTCGHGE..........CIPAHWR.CDKRNDCVDG......SDEHN......C
LRP2_HUMAN  C..........LDTQYTCDNHQ..........CISKNWV.CDTDNDCGDG......SDEKN......C
LRP2_HUMAN  C..........QPSQFNCPNHR..........CIDLSFV.CDGDKDCVDG......SDEVG......C
LRP2_HUMAN  C..........TASQFKCASGDK.........CIGVTNR.CDGVFDCSDN......SDEAG......C
LRP2_HUMAN  C..........HSDEFQCQEDGI.........CIPNFWE.CDGHPDCLYG......SDEHNA.....C
LRP2_HUMAN  C..........PSSYFHCDNGN..........CIHRAWL.CDRDNDCGDM......SDEKD......C
LRP2_HUMAN  C..........PSWQWQCLGHNI.........CVNLSWV.CDGIFDCPNG......TDESPL.....C
LRP2_HUMAN  C..........GASSFTCSNGR..........CISEEWK.CDNDNDCGDG......SDEMESV....C
LRP2_HUMAN  C..........SPTAFTCANGR..........CVQYSYR.CDYYNDCGDG......SDEAG......C
LRP2_HUMAN  C..........NATTEFMCNNRR.........CIPREFI.CNGVDNCHDNNT...SDEKN......C
LRP2_HUMAN  C..........QSGYTKCHNSNI.........CIPRVYL.CDGDNDCGDN......SDENPTY....C
LRP2_HUMAN  C..........SSSEFQCASGR..........CIPQHWV.CDQETDCFDA......SDEPAS.....C
LRP2_HUMAN  C..........LADEFKCDGGR..........CIPSEWI.CDGDNDCGDM......SDEDKRHQ...C
LRP2_HUMAN  C..........SDSEFLCVNDRPPDRR.....CIPQSWV.CDGDVDCTDG......YDENQN.....C
LRP2_HUMAN  C..........SENEFTCCYGL..........CIPKIFR.CDRHNDCGDY......SDERG......C
LRP2_HUMAN  C..........QQNQFTCQNGR..........CISKTFV.CDEDNDCGDG......SDELMHL....C
LRP2_HUMAN  C..........PPHEFKCDNGR..........CIEMMKL.CNHLDDCLDN......SDEKG......C
LRP2_HUMAN  C..........SSTQFLCANNEK.........CIPIWWK.CDGQKDCSDG......SDELAL.....C
LRP2_HUMAN  C..........RLGQFQCSDGN..........CTSPQTL.CNAHQNCPDG......SDEDRLL....C
LRP2_HUMAN  C..........DSNEWQCANKR..........CIPESWQ.CDTFNDCEDN......SDEDSSH....C
LRP2_HUMAN  C..........RPGQFRCANGR..........CIPQAWK.CDVDNDCGDH......SDEPIEE....C
LRP2_HUMAN  C..........DNFTEFSCKTNYR........CIPKWAV.CNGVDDCRDN......SDEQG......C
LRP2_HUMAN  C..........HPVGDFRCKNHH.........CIPLRWQ.CDGQNDCGDN......SDEEN......C
LRP2_HUMAN  C..........TESEFRCVNQQ..........CIPSRWI.CDHYNDCGDN......SDERD......C
LRP2_HUMAN  C..........HPEYFQCTSGH..........CVHSELK.CDGSADCLDA......SDEAD......C
LRP2_HUMAN  C..........QATMFECKNHV..........CIPPYWK.CDGDDCGDG......SDEELHL....C
LRP2_HUMAN  C..........NSPNRFRCDNNR.........CIYSHEV.CNGVDDCGDG......TDETEEH....C
LRP2_HUMAN  C..........TEYEYKCGNGH..........CIPHDNV.CDDADDCGDW......SDELG......C
LR1B_HUMAN  C..........DPGEFLCHDHVT.........CVSQSWL.CDGDPDCPDD......SDESLDT....C
LR1B_HUMAN  C..........PLNHIACLGTNK.........CVHLSQL.CNGVLDCPDG......YDEGVH.....C
LR1B_HUMAN  C..........KAGEFRCKNRH..........CIQARWK.CDGDDDCLDG......SDEDSVN....C
```

73

## FIG. 10 (cont)

```
LR1B_HUMAN    C.........PDDQFKCQNNR..........CIPKRWL.CDGANDCGSN......EDESNQT....C
LR1B_HUMAN    C.........QVDQFSCGNGR..........CIPRAWL.CDREDDCGDQ......TDEMAS.....C
LR1B_HUMAN    C.........EPLTQFVCKSGR..........CISSKWH.CDSDDDCGDG......SDEVG......C
LR1B_HUMAN    C.........FDNQFRCSSGR..........CIPGHWA.CDGDNDCGDF......SDEAQIN....C
LR1B_HUMAN    C.........NGNEFQCHPDGN..........CVPDLWR.CDGEKDCEDG......SDEKG......C
LR1B_HUMAN    C.........DHKTKFSCWSTGR..........CINKAWV.CDGDIDCEDQ......SDEDD......C
LR1B_HUMAN    C.........GPPKHPCANDTSV.........CLQPEKL.CNGKKDCPDG......SDEGYL.....C
LR1B_HUMAN    C.........NAYSEFECGNGE..........CIDYQLT.CDGIPHCKDK......SDEKLLY....C
LR1B_HUMAN    C.........RRGFKPCYNRR..........CIPHGKL.CDGENDCGDN......SDELD......C
LR1B_HUMAN    C.........ATVEFRCADGT..........CIPRSAR.CNQNIDCADA......SDELK......C
LR1B_HUMAN    CTHFYKLGVKTTGPIRCNSTSL.........CVLPTWI.CDGSNDCGDY......SDELK......C
LR1B_HUMAN    C.........EENYFSCPSGR..........CILNTWI.CDGQKDCEDG......RDEFH......C
LR1B_HUMAN    C.........SWNQFACSAQK..........CISKHWI.CDGEDDCGDG......LDESDSI....C
LR1B_HUMAN    C.........AADMFSCQGSRA..........CVPRHWL.CDGERDCPDG......SDELSTAG...C
LR1B_HUMAN    C.........DENAFMCHNKV..........CIPKQFV.CDHDDDCGDG......SDESPQ.....C
LR1B_HUMAN    C.........GTEEFSCADGR..........CLLNTQWQCDGDFDCPDH......SDEAPLNPK..C
LR1B_HUMAN    C.........NSSFFMCKNGR..........CIPSGGL.CDNKDDCGDG......SDERN......C
LR1B_HUMAN    C.........TASQFRCKTDK..........CIPFWWK.CDTVDDCGDG......SDEPDD.....C
LR1B_HUMAN    C.........QPGRFQCGTGL..........CALPAPI.CDGENDCGDN......SDELN......C
LR1B_HUMAN    C.........LSGQFKCTKNQK..........CIPVNLR.CNGQDDCGDE......EDERD......C
LR1B_HUMAN    C.........SPDYFQCKTTKH..........CISKLWV.CDEDPDCADA......SDEAN......C
LR1B_HUMAN    C.........GPHEFQCKNNN..........CIPDHWR.CDSQNDCSDN......SDEEN......C
LR1B_HUMAN    C.........TLKDFLCANGD..........CVSSRFW.CDGDFDCADG......SDERN......C
LR1B_HUMAN    C.........SKDQFRCSNGQ..........CIPAKWK.CDGHEDCKYG......EDEKS......C
LR1B_HUMAN    C.........SSREYICASDG..........CISASLK.CNGEYDCADG......SDEMD......C
LR1B_HUMAN    C.........KEDQFRCNKAH..........CIPIRWL.CDGIHDCVDG......SDEEN......C
LR1B_HUMAN    C.........RADEFLCNNSL..........CKLHFWV.CDGEDDCGDN......SDEAPDM....C
LR1B_HUMAN    C.........PSTRPHRCRNNRI.........CLQSEQM.CNGIDECGDN......SDEDH......C
LR1B_HUMAN    C.........KKDEFACSNKK..........CIPMDLQ.CDRLDDCGDG......SDEQG......C


O75851            C.........AEGEALCQENGH..........CVPHGWL.CDNQDDCGDG......SDEEGE.....C
O75851            C.........GEGQMTCSSGH..........CLPLALL.CDRQDDCGDG......TDEPSYP....C
O75851            C.........PQGLLACADGR..........CLPPALL.CDGHPDCLDA......ADEES......C
O75851            C.........VPGEVSCVDGT..........CLGAIQL.CDGVWDCPDG......ADEGPGH....C
ENSP00000262089
= O75851          C.........GPFEFRCGSGE..........CTPRGWR.CDQEEDCADG......SDERG......C
ENSP00000262089
                  C.........APHHAPCARGPH..........CVSPEQL.CDGVRQCPDG......SDEGPDA....C
O75851            C.........PGLFPCGVAPGL..........CLTPEQL.CDGIPDCPQG......EDELD......C
O75851            C.........PEYTCPNGT..........CIGFQLV.CDGQPDCGRPGQVGPSPEEQG....C
O75851            C.........EPGVGLRCASGE..........CVLRGGP.CDGVLDCEDG......SDEEG......C
ENSP00000262089
                  C.........GPGQTPCEVLG..........CVEQAQV.CDGREDCLDG......SDERH......C
O75851            C.........SPSQLSCGSGE..........CLSAERR.CDLRPDCQDG......SDEDG......C
C18oRP1           C.........KFTCTSGK..........CLYLGSLVCNQQNDCGDN......SDEEN......C
AAH07083/Q9NPFO
                  C.........PPTKFQCRTSGL..........CVPLTWR.CDRDLDCSDG......SDEEE......C
AAH07083/Q9NPFO
                  C.........LAGELRCTLSDD..........CIPLTWR.CDGHPDCPDS......SDELG......C
Q9HBX9            C.........SLGYFPCGNITK..........CLPQLLH.CNGVDDCGNQ......ADEDN......C
Q9BY79/Q96DQ9 C.........AHDEFRCDQLI..........CLLPDSV.CDGFANCADG......SDETN......C
Q9BY79/Q96DQ9 C.........GPSELSCQAGG..........CKGVQWM.CDMWRDCTDG......SDDN......C
BAB55257 =
ENSP00000239367
                  C.........SRYHPFCDDGC..........CIDITLA.CDGVQQCPDG......SDEDF......C
O95518 =          C.........PGEFLCSVNGL..........CVPA....CDGVKDCPNG......LDERN......C
ENSP00000255793
ENSP00000255793
                  C.........RATFQCKEDST..........CISLPKV.CDGQPDCLNG......SDEEQ......C
ENSP00000255793
                  C.........GTFTFQCEDRS..........CVKKPNPQCDGRPDCRDG......SDEEH......C


Q8WXD0     C     QKGYFPCGNLTK     CLPRAFH  CDGKDDCGNG     ADEEN     C
Q8NBJ0     C     STARYHCKNGL      CIDKSFI  CDGQNNCQDN     SDEES     C
Q8NBJ0     C     GPTFFPCASGIH     CIIGRFR  CNGFEDCPDG     SDEEN     C
Q8NBJ0     C     NIPGNFMCSNGR     CIPGAWQ  CDGLPDCFDK     SDEKE     C
MEGF7      C     ALDQFLCWNGR      CIGQRKL  CNGVNDCGDN     SDESPQQN  C
MEGF7      C     EEDEFPCQNGY      CIRSLWH  CDGDNDCGDN     SDEQ      C
MEGF7      C     RSGEFMCDSGL      CINAGWR  CDGDADCDDQ     SDERN     C
MEGF7      C     TAEQFRCHSGR      CVRLSWR  CDGEDDCADN     SDEEN     C
MEGF7      C     SPLDFHCDNGK      CIRRSWV  CDGDNDCEDD     SDEQD     C
MEGF7      C     NLEEFQCAYGR      CILDIYH  CDGDDDCGDW     SDESD     C
MEGF7      C     SDKEFRCSDGS      CIAEHWY  CDGDTDCKDG     SDEEN     C
MEGF7      C     GRSHFTCAVSALGECT CIPAQWQ  CDGDNDCGDH     SDEDG     C
CAD61944   C     LQEEFQCLNHR      CVSAVQR  CDGVDACGDG     SDEAG     C
```

# FIG. 10 (CONT)

| | | | | |
|---|---|---|---|---|
| CAD61944 | C | PPGHFPCGAAGTSGATA | CYLPADR CNYQTFCADG | ADERR | C |
| CAD61944 | C | QPGNFRCRDEK | CVYETWV CDGQPDCADG | SDEWD | C |
| ENSG00000181006 | C | PEITDFLCRDKK | CIASHLL CDYKPDCSDR | SDEAH | C |
| ENSP00000320248 | C | NNRTFKCGNDI | CFRKQNAKCDGTVDCPDG | SDEEG | C |
| ENSP00000277547 | C | PPGHHHCQNKV | CVEPQQL CDGEDNCGDL | SDENPLT | C |
| ENSP00000320022 | C | KQGHLACGDL | CVPPEQL CDFEEQCAGG | EDEQA | C |
| ENSP00000313222 | C | PGNSFSCGNSQ | CVTKVNPECDDQEDCSDG | SDEAH | C |

75

Figure 11

EP 2 198 890 B1

A.

B.

# Figure 11

C.

D.

Light chain is encoded
as a separate protein

| ⬭ = heavy chain |
| ⬬ = light chain |

EP 2 198 890 B1

Figure 12

○ = any monomer domain

〒 = obligate heterodimeric linker

# Figure 13C

4 domain
ring

N

C

100 kD

= heavy domain

= light domain

= antigen binding site

Figure 14

A.

N     C    2 chain, 2 Fv
N      C     multimer
C      N
C      N

N —  — C

N —  — C

B.

N     C    Single chain
N      C     multimer
C      N
C      N

N —  —  —  — C

C.

N     C    Single chain
N      C     multimer
C      N
C      N

N —  —  —  — C

EP 2 198 890 B1

# Figure 15

**Figure 16**
**Cell Killing induced by Maxybodies**

Cell Killing by SKVILF-Constructs

Figure 17: TPO-R Phage Specificity Data

**Figure 18: TF1 Proliferation Assay**

Figure 19 : Epitope mapping of IgE-Binding Monomer

Figure 20

CD28 ELISA Binding

Figure 21

Figure 22

Figure 23

Figure 24

Figure 25

EP 2 198 890 B1

Figure 26

Stain membrane using cDNA phage specific MAb (HRP labeled)
Add substrate

Cut positive spots out of membrane and add to PCR tube
PCR amplify cDNA and MB sequences

cDNA MB

Sequence PCR fragments

EP 2 198 890 B1

Figure 27

Format Variations

Avidity          Avidity          Recruit          Avidity +          Halflife +
                                  Function         Agonism            Avidity

Effector
Sequence

Targeting
Domains

# Figure 28

## Multimer Format

Exemplary Multimer 9 kD

Anti-Fc-R1

Anti-CD20

### Monovalent Binding

### Complex Stabilization

Fc-Receptor

CD20

Monomer binding

Multimeric, self-stabilizing complex with increased avidity

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02088171 A
- WO 0164942 A
- WO 9916873 A
- WO 0060070 A
- WO 0123401 A
- US 9117271 W
- US 9118980 W
- US 9119818 W
- US 9308278 W
- US 6281344 B
- US 6194550 B
- US 6207446 B
- US 6214553 B
- US 6258558 B
- US 5856456 A
- US 20030082575 A
- US 5811238 A
- US 5491074 A
- WO 9428173 A
- US 5605793 A, Stemmer
- US 5830721 A, Stemmer
- US 5834252 A, Stemmer
- US 5837458 A, Minshull
- WO 9522625 A, Stemmer and Crameri
- WO 9633207 A, Stemmer and Lipschutz
- WO 9720078 A, Stemmer and Crameri
- WO 9735966 A, Minshull and Stemmer

- WO 9941402 A, Punnonen
- WO 9941383 A, Punnonen
- WO 9941369 A, Punnonen
- WO 9941368 A, Punnonen
- EP 752008 A, Stemmer and Crameri
- EP 0932670 A, Stemmer
- WO 9923107 A, Stemmer
- WO 9921979 A, Apt
- WO 9831837 A, del Cardayre
- WO 9827230 A, Patten and Stemmer
- WO 0000632 A
- WO 0009679 A
- WO 9842832 A, Arnold
- WO 9929902 A, Arnold
- WO 9841653 A, Vind
- WO 9841622 A, Borchert
- WO 9842727 A, Pati and Zarling
- WO 0018906 A, Patten
- WO 00104190 A, del Cardayre
- WO 0042561 A, Crameri
- WO 0042559 A, Selifonov and Stemmer
- WO 0042560 A, Selifonov
- US 0106775 W
- US 4683202 A, Mullis
- US 5426039 A, Wallace
- US SN60118854 A, Selifonov and Stemmer
- US SN09416375 A, Selifonov and Stemmer

**Non-patent literature cited in the description**

- **HERZ.** Lipoprotein receptors: beacons to neutrons?. *Trends in Neurosciences,* 2001, vol. 24 (4), 193-195
- **GOLDSTEIN ; BROWN.** The Cholesterol Quartet. *Science,* 2001, vol. 292, 1310-1312
- **HUSSAIN et al.** The Mammalian Low-Density Lipoprotein Receptor Family. *Annu. Rev. Nutr.,* 1999, vol. 19, 141-72
- **FRISHMAN, D. ; ARGOS, P.** Knowledge-based secondary structure assignment. *Proteins,* 1995, vol. 23 (4), 566-79
- **JONES, D.T.** Protein secondary structure prediction based on position-specific scoring matrices. *J. Mol. Biol.,* 1999, vol. 292, 195-202
- **MCGUFFIN, L.J. ; BRYSON, K. ; JONES, D.T.** The PSIPRED protein structure prediction server. *Bioinformatics,* 2000, vol. 16, 404-405

- **PESSI et al.** A designed metal-binding protein with a novel fold. *Nature,* 1993, vol. 362, 367-369
- **HAMERS-CASTERMAN, C. et al.** Naturally occurring antibodies devoid of light chains. *Nature,* 1993, vol. 363, 446-448
- **DUMOULIN et al.** Single-domain antibody fragments with high conformational stability. *Protein Science,* 2002, vol. 11, 500-515
- **BIRD et al.** Single-chain antigen-binding proteins. *Science,* 1988, vol. 242 (4877), 423-426
- **HUSTON et al.** Protein engineering of antibody binding sites: recovery of specific activity in an anti-digoxin single-chain Fv analogue produced in Escherichia coli. *Proc Natl Acad Sci U S A,* 1988, vol. 85 (16), 5879-83
- **PLUCKTHUN ; SKERRA.** Expression of functional antibody Fv and Fab fragments in Escherichia col. *Methods Enzymol,* 1989, vol. 178, 497-515

- **HEGYI, H ; BORK, P.** On the classification and evolution of protein modules. *J. Protein Chem.,* 1997, vol. 16 (5), 545-551
- **BARON et al.** Protein modules. *Trends Biochem. Sci.,* 1991, vol. 16 (1), 13-7
- **PONTING et al.** Evolution of domain families. *Adv. Protein Chem.,* 2000, vol. 54, 185-244
- **DOOLITTLE.** The multiplicity of domains in proteins. *Annu. Rev. Biochem,* 1995, vol. 64, 287-314
- **DOOLITTE ; BORK.** Evolutionarily mobile modules in proteins. *Scientific American,* 1993, vol. 269 (4), 50-6
- **BORK.** Shuffled domains in extracellular proteins. *FEBS letters,* 1991, vol. 286 (1-2), 47-54
- **SCHULTZ et al.** SMART: a web-based tool for the study of genetically mobile domains. *Nucleic Acid Res.,* 2000, vol. 28 (1), 231-34
- **SHULTZ.** SMART: a web-based tool for the study of genetically mobile domains. *Nucleic Acids Research,* 2000, vol. 28 (1), 231-234
- **PEARL.** Assigning genomic sequences to CATH. *Nucleic Acids Research,* 2000, vol. 28 (1), 277-282
- **HOWELL ; HERTZ.** The LDL receptor gene family: signaling functions during development. *Current Opinion in Neurobiology,* 2001, vol. 11, 74-81
- **KRIEGER.** The ''best'' of cholesterols, the ''worst '' of cholesterols: A tale of two receptors. *PNAS,* 1998, vol. 95, 4077-4080
- **MOESTRUP ; VERROUST.** Megalin-and Cubilin-Mediated Endocytosis of Protein-Bound Vitamins, Lipids, and Hormones in Polarized Epithelia. *Ann. Rev. Nutr.,* 2001, vol. 21, 407-28
- **RIZO ; SÜDHOF.** *J. Biol. Chem,* 1998, vol. 273, 15879-15882
- **CHO.** *J. Biol. Chem.,* 2001, vol. 276, 32407-32410
- **RIZO ; SÜDHOF.** *J. Biol. Chem.,* 1998, vol. 273, 15879-15882
- **YAMAZAKI et al.** Elements of neutral Adhesion Molecules and a Yeast Vacuolar Protein Sorting Receptor are Present in a Novel Mammalian Low Density Lipoprotein Receptor Family Member. *Journal of Biological Chemistry,* 1996, vol. 271 (40), 24761-24768
- **NAKAYAMA ET AL. , IDENTIFICATION OF HIGH-MOLECULAR-WEIGHT PROTEINS WITH MULTIPLE EGF-LIKE MOTIFS BY MOTIF-TRAP SCREENING ET AL.** Nakayama et al., Identification of High-Molecular-Weight Proteins with Multiple EGF-like Motifs by Motif-Trap Screening. *Genomics,* 1998, vol. 51, 27-34
- **LIU et al.** Genomic Organization of New Candidate Tumor Suppressor Gene, LRPIB. *Genomics,* 2000, vol. 69, 271-274
- **LIU et al.** The Putative Tumor Suppressor LRP1B, a Novel Member of the Low Density Lipoprotein (LDL) Receptor Family, Exhibits Both Overlapping and Distinct Properties with the LDL Receptor-related Protein. *Journal of Biological Chemistry,* 2001, vol. 276 (31), 28889-28896
- **ISHII et al.** cDNA of a New Low-Density Lipoprotein Receptor-Related Protein and Mapping of its Gene (LRP3) to Chromosome Bands 19q12-q13.2. *Genomics,* 1998, vol. 51, 132-135
- **ORLANDO et al.** Identification of the second cluster of ligand-binding repeats in megalin as a site for receptor-ligand interactions. *PNAS USA,* 1997, vol. 94, 2368-2373
- **JEON ; SHIPLEY.** Vesicle-reconstituted Low Density Lipoprotein Receptor. *Journal of Biological Chemistry,* 2000, vol. 275 (39), 30458-30464
- **SIMMONS et al.** Human Low Density Lipoprotein Receptor Fragment. *Journal of Biological Chemistry,* 1997, vol. 272 (41), 25531-25536
- **FASS et al.** Molecular Basis of familial hypercholesterolaemia from structure of LDL receptor module. *Nature,* 1997, vol. 388, 691-93
- **DALY et al.** Three-dimensional structure of a cysteine-rich repeat from the low-density lipoprotein receptor. *PNAS USA,* 1995, vol. 92, 6334-6338
- **NORTH ; BLACKLOW.** Structural Independence of Ligand-Binding Modules Five and Six of the LDL Receptor. *Biochemistry,* 1999, vol. 38, 3926-3935
- **NORTH ; BLACKLOW.** Solution Structure of the Sixth LDL-A module of the LDL Receptor. *Biochemistry,* 2000, vol. 39, 25640-2571
- **NORTH ; BLACKLOW.** Evidence that Familial Hypercholeslerolemia Mutations of the LDL Receptor Cause Limited Local Misfolding in an LDL-A Module Pair. *Biochemistry,* 2000, vol. 39, 13127-13135
- **BEGLOVA et al.** Backbone Dynamics of a Module Pair from the Ligand-Binding Domain of the LDL Receptor. *Biochemistry,* 2001, vol. 40, 2808-2815
- **BIERI et al.** Folding, Calcium binding, and Structural Characterization of a Concatemer of the Fist and Second Ligand-Binding Modules of the Low-Density Lipoprotein Receptor. *Biochemistry,* 1998, vol. 37, 10994-11002
- **JEON et al.** Implications for familial hypercholesterolemia from the structure of the LDL receptor YWTD-EGF domain pair. *Nature Structural Biology,* 2001, vol. 8 (6), 499-504
- **KURNIAWAN et al.** NMR structure of a concatemer of the first and second ligand-binding modules of the human low-density lipoprotein receptor. *Protein Science,* 2000, vol. 9, 1282-1293

- **ESSER et al.** Mutational Analysis of the Ligand Binding Domain of the Low Density Lipoprotein Receptor. *Journal of Biological Chemistry*, 1988, vol. 263 (26), 13282-13290
- **RUSSELL et al.** Different Combinations of Cysteine-rich Repeats Mediate Binding of Low Density Lipoprotein Receptor to Two Different Proteins. *Journal of Biological Chemistry*, 1989, vol. 264 (36), 21682-21688
- **DAVIS et al.** Acid-dependent ligand dissociation and recycling of LDL receptor meditated by growth factor homology region. *Nature*, 1987, vol. 326, 760-765
- **RONG et al.** Conversion of a human low-density lipoprotein receptor ligand binding repeat to a virus receptor: Identification of residues important for ligand specificity. *PNAS USA*, 1998, vol. 95, 8467-8472
- **AGNELLO et al.** Hepatitis C virus and other Flaviviridae viruses enter cells via low density lipoprotein receptor. *PNAS*, 1999, vol. 96 (22), 12766-1277 1
- **ESSER ; RUSSELL.** Transport-deficient Mutations in the Low Density lipoprotein receptor. *Journal of Biological Chemistry*, 1988, vol. 263 (26), 13276-1328 1
- **DAVIS et al.** The Low Density Lipoprotein Receptor. *Journal of Biological Chemistry*, 1987, vol. 262 (9), 4075-4082
- **PEACOCK et al.** Human Low Density Lipoprotein Receptor Expressed in Xenopus Oocytes. *Journal of Biological Chemistry*, 1988, vol. 263 (16), 7838-7845
- **SAVONEN et al.** The Carboxyl-terminal Domain of Receptor-associated Protein Facilitates Proper Folding and Trafficking of the Very Low Density Lipoprotein Receptor by Interaction with the Three Amino-terminal Ligand-binding Repeats of the Receptor. *Journal of Biological Chemistry*, 1999, vol. 274 (36), 25877-25882
- **HEWAT et al.** The cellular receptor to human rhinovirus 2 binds around the 5-fold axis and not in the canyon: a structural view. *EMBO Journal*, 2000, vol. 19 (23), 6317-6325
- **OKUN et al.** VLDL Receptor Fragments of Different Lengths Bind to Human Rhinovirus HRV2 with Different Stoichiometry. *Journal of Biological Chemistry*, 2001, vol. 276 (2), 1057-1062
- **RETTENBERGER et al.** Ligand Binding Properties of the Very Low Density Lipoprotein Receptor. *Journal of biological Chemistry*, 1999, vol. 274 (13), 8973-8980
- **MIKHAILENKO et al.** Functional Domains of the very low density lipoprotein receptor: molecular analysis of ligand binding and acid-dependent ligand dissociation mechanisms. *Journal of Cell Science*, 1999, vol. 112, 3269-3281
- **BRANDES et al.** Alternative Splicing in the Ligand Binding Domain of Mouse ApoE Receptor-2 Produces Receptor Variants Binding Reelin but not alpa2-macroglobulin. *Journal of Biological Chemistry*, 2001, vol. 276 (25), 22160-22169
- **KIM et al.** Exon/Intron Organization, Chromosome Localization, Alternative Splicing, and Transcription Units of the Human Apolipoprotein E Receptor 2 Gene. *Journal of Biological Chemistry*, 1997, vol. 272 (13), 8498-8504
- **OBERMOELLER-MCCORMICK et al.** Dissection of receptor folding and ligand-binding property with functional minireceptors of LDL receptor-related protein. *Journal of Cell Science*, 2001, vol. 114 (5), 899-908
- **HOM et al.** Molecular Analysis of Ligand Binding of the Second Cluster of Complement-type Repeats of the Low Density Lipoprotein Receptor-related Protein. *Journal of Biological Chemistry*, 1997, vol. 272 (21), 13608-13613
- **NEELS et al.** The Second and Fourth Cluster of Class A Cysteine-rich Repeats of the Low Density Lipoprotein Receptor-related Protein Share Ligand-binding Properties. *Journal of Biological Chemistry*, 1999, vol. 274 (44), 31305-31311
- **OBERMOELLER et al.** Differential Functions of the Triplicated Repeats Suggest Two Independent Roles for the Receptor-Associated Protein as a Molecular Chaperone. *Journal of Biological Chemistry*, 1997, vol. 272 (16), 10761-10768
- **ANDERSEN et al.** Identification of the Minimal Functional Unit in the Low Density Lipoprorein Receptor-related Protein for Binding the Receptor-associated Protein (RAP). *Journal of Biological Chemistry*, 2000, vol. 275 (28), 21017-21024
- **ANDERSEN et al.** Specific Binding of alpha-Macroglobulin to Complement-Type Repeat CR4 of the Low-Density Lipoprotein Receptor-Related Protein. *Biochemistry*, 2000, vol. 39, 10627-10633
- **VASH et al.** Three Complement-Type Repeats of the Low-Density Lipoprotein Receptor-Related Protein Define a Common Binding Site for RAP, PAI-1, and Lactoferrin. *Blood*, 1998, vol. 92 (9), 3277-3285
- **DOLMER et al.** NMR Solution Structure of Complement-like Repeat CR3 from the L ow Density Lipoprotein Receptor-related Protein. *Journal of Biological Chemistry*, 2000, vol. 275 (5), 3264-3269
- **HUANG et al.** NMR Solution Structure of Complement-like Repeat CR8 from the Low Density Lipoprotein Receptor-related Protein. *Journal of Biological Chemistry*, 1999, vol. 274 (20), 14130-14136
- **LIU et al.** Uptake of HIV-1 Tat protein mediated by low-density lipoprotein receptor-related protein disrupts the neuronal metabolic balance of the receptor ligands. *Nature Medicine*, 2000, vol. 6 (12), 1380-1387

- **FITZGERALD et al.** Pseudomonas Exotoxin-mediated Selection Yields Cells with Altered Expression of Low-Density Lipoprotein Receptor-related Protein. *Journal of Cell Biology,* 1995, vol. 129, 1533-41
- **WILLNOW ; HERZ.** Genetic deficiency in low density lipoprotein receptor-related protein confers cellular resistance to Pseudomonas exotoxin A. *Journal of Cell Science,* 1994, vol. 107, 719-726
- **TROMMSDORF et al.** Interaction of Cytosolic Adaptor Proteins with Neuronal Apolipoprotein E Receptors and the Amyloid Precursor Protein. *Journal of Biological Chemistry,* 1998, vol. 273 (5), 33556-33560
- **STOCKINGER et al.** The Low Density Lipoprotein Receptor Gene Family. *Journal of Biological Chemistry,* 1998, vol. 273 (48), 32213-32221
- **OBERMOELLER et al.** Ca+2 and Receptor-associated Protein are independently required for proper folding and disulfide bond formation of the low density lipoprotein receptor-related protein. *Journal of Biological Chemistry,* 1998, vol. 273 (35), 22374-22381
- **SATO et al.** 39-kDa receptor-associated protein (RAP) facilitates secretion and ligand binding of extracellular region of very-low-density-lipoprotein receptor: implications for a distinct pathway from low-density-lipoprotein receptor. *Biochem. J.,* 1999, vol. 341, 377-383
- **AVROMOGLU et al.** Functional Expression of the Chicken Low Density Lipoprotein Receptor-related Protein in a mutant Chinese Hamster Ovary Cell Line Restores Toxicity of Pseudomonas Exotoxin A and Degradation of alpha2-Macroglobulin. *Journal of Biological Chemistry,* 1998, vol. 273 (11), 6057-6065
- **KINGSLEY ; KRIEGER.** Receptor-mediated endocytosis of low density lipoprotein: Somatic cell mutants define multiple genes required for expression of surface-receptor activity. *PNAS USA,* 1984, vol. 81, 5454-5458
- **LI et al.** Differential Functions of Members of the Low Density Lipoprotein Receptor Family Suggests by their distinct endocystosis rates. *Journal of Biological Chemistry,* 2001, vol. 276 (21), 18000-18006
- **SPRINGER.** An Extracellular beta-Propeller Module Predicted in Lipoprotein and Scavenger Receptors, Tyrosine Kinases, Epidermal Growth Factor Precursor, and Extracellular Matrix Components. *J. Mol. Biol.,* 1998, vol. 283, 837-862
- **MAMMEN et al.** *Angew Chem Int. Ed.,* 1998, vol. 37, 2754-2794
- **MULLER et al.** *Anal. Biochem.,* 1998, vol. 261, 149-158
- **HALLEWELL et al.** *J. Biol. Chem.,* 1989, vol. 264, 5260-5268

- **ALFTHAN et al.** *Protein Eng.,* 1995, vol. 8, 725-731
- **ROBINSON ; SAUER.** *Biochemistry,* 1996, vol. 35, 109-116
- **KHANDEKAR et al.** *J. Biol. Chem.,* 1997, vol. 272, 32190-32197
- **FARES et al.** *Endocrinology,* 1998, vol. 139, 2459-2464
- **SMALLSHAW et al.** *Protein Eng.,* 1999, vol. 12, 623-630
- **TANG et al.** *J. Biol. Chem.,* 1996, vol. 271, 15682-15686
- **HENNECKE et al.** *Protein Eng.,* 1998, vol. 11, 405-410
- **ROBINSON ; SAUER.** *Proc. Natl. Acad. Sci. USA,* 1998, vol. 95, 5929-5934
- **F.S. GIMBLE.** *Chemistry and Biology,* 1998, vol. 5 (10), 251-256
- **CHIEN.** *Proc. Natl. Acad. Sci. (USA,* 1991, vol. 88, 9578
- **FIELDS S. ; SONG O.** *Nature,* 1989, vol. 340, 245
- **SILVER S.C. ; HUNT S.W.** *Mol. Biol. Rep.,* 1993, vol. 17, 155
- **DURFEE et al.** *Genes Devel.,* 1993, vol. 7, 555
- **YANG et al.** *Science,* 1992, vol. 257, 680
- **LUBAN et al.** *Cell,* 1993, vol. 73, 1067
- **HARDY et al.** *Genes Devel.,* 1992, vol. 6, 801
- **BARTEL et al.** *Biotechniques,* 1993, vol. 14, 920
- **VOJTEK et al.** *Cell,* 1993, vol. 74, 205
- **GERMINO et al.** *Proc. Nat. Acad. Sci. (U.S.A.,* 1993, vol. 90, 993
- **GUARENTE L.** *Proc. Nat. Acad. Sci. (U.S.A.,* 1993, vol. 90, 1639
- **MADDEN, M. ; ALDWIN, L. ; GALLOP, M. A. ; STEMMER, W. P. C.** Peptide linkers: Unique self-associative high-affinity peptide linkers. *Thirteenth American Peptide Symposium,* 1993
- **BODENMULLER et al.** The neuropeptide head activator loses its biological activity by dimerization. *EMBO J,* 1986, vol. 5 (8), 1825-1829
- **SOONG, N. et al.** Moleccdar bleeding of viruses. *Nat Genet,* 2000, vol. 25 (4), 436-439
- **STEMMER et al.** Molecular breeding of viruses for targeting and other clinical properties. *Tumor Targeting,* vol. 4, 1-4
- **NESS et al.** DNA Shuffling of subgenornic sequences of subtilisin. *Nature Biotechnology,* 1999, vol. 17, 893-896
- **CHANG et al.** Evolution of a cytokine using DNA family shuffling. *Nature Biotechnology,* 1999, vol. 17, 793-797
- **MINSHULL ; STEMMER.** Protein evolution by molecular breeding. *Current Opinion in Chemical Biology,* 1999, vol. 3, 284-290

- **CHRISTIANS et al.** Directed evolution of thymidine kinase for AZTphosphorylation using DNA family shuffling. *Nature Biotechnology,* 1999, vol. 17, 259-264
- **CRAMERI et al.** DNA shuffling of a family of genes from diverse species accelerates directed evolution. *Nature,* 1998, vol. 391, 288-291
- **CRAMERI et al.** Molecular evolution of an arsenate detoxification pathway hy DNA shuffling. *Nature Biotcchnology,* 1997, vol. 15, 436-438
- **ZHANG et al.** Directed evolution of an effective fucosidase from a galactosidase by DNA shuffling and screening. *Proc. Natl. Acad. Sci. USA,* 1997, vol. 94, 4504-4509
- **PATTEN et al.** Applications of DNA Shuffling to Pharmaceuticals and Vaccines. *Current Opinion in Biotechnology,* 1997, vol. 8, 724-733
- **CRAMERI et al.** Construction and evolution of antibody-phage libraries by DNA shuffling. *Nature Medicine,* 1996, vol. 2, 100-103
- **CRAMERI et al.** Improved green fluorescent protein by molecular evolution using DNA shuffling. *Nature Biotechnolgy,* 1996, vol. 14, 315-319
- **GATES et al.** Affinity selective isolation of ligands from peptide Libraries through display on a lac repressor 'headpiece dimer'. *Journal of Molecular Biology,* 1996, vol. 255, 373-386
- Sexual PCR and Assembly PCR. **STEMMER.** The Encyclopedia of Molecular Biology. VCH Publishers, 1996, 447-457
- **CRAMERI ; STEMMER.** Combinatorial multiple cassette mutagenesis creates all the permutations of mutant and wildtype cassettes. *BioTechniques,* 1995, vol. 18, 194-195
- **STEMMER et al.** Single-step assembly of a gene and entire plasmid form large numbers of oligodeoxy-ribonucleotides. *Gene,* 1995, vol. 164, 49-53
- **STEMMER.** The Evolution of Molecular Computation. *Science,* 1995, vol. 270, 1510
- **STEMMER.** Searching Sequence Space. *Bio/Technology,* 1995, vol. 13, 549-553
- **STEMMER.** Rapid evolution of a protein in vitro by DNA shuffling. *Nature,* 1994, vol. 370, 389-391
- **STEMMER.** DNA shuffling by randorn fragmentation and reassembly: In vitro recombination for molecular evolution. *Proc. Natl. Acad. Sci. USA,* 1994, vol. 91, 10747-10751
- **LING et al.** Approaches to DNA mutagenesis: an overview. *Anal Biochem.,* 1997, vol. 254 (2), 157-178
- **DALE et al.** Oligonucleotide-directed random mutagenesis using the phosphorothioate method. *Methods Mol. Biol.,* 1996, vol. 57, 369-374
- **SMITH.** In vitro mutagenesis. *Ann. Rev. Genet.,* 1985, vol. 19, 423-462
- **BOTSTEIN ; SHORTLE.** Strategies and applications of in vitro mutagenesis. *Science,* 1985, vol. 229, 1193-1201
- **CARTER.** Site-directed mutagenesis. *Biochem. J.,* 1986, vol. 237, 1-7
- The efficiency of oligonucleotide directed mutagenesis. **KUNKEL.** Nucleic Acids & Molecular Biology. Springer Verlag, 1987
- **KUNKEL.** Rapid and efficient site-specific mutagenesis without phenotypic selection. *Proc. Natl. Acad. Sci. USA,* 1985, vol. 82, 488-492
- **KUNKEL et al.** Rapid and efficient site-specific mutagesis without phenotypic selection. *Methods in Enzymol.,* 1987, vol. 154, 367-382
- **BASS et al.** Mutant Trp reepressors with new DNA-binding specificities. *Science,* 1988, vol. 242, 240-245
- *Methods in Enzymal.,* 1983, vol. 100, 468-500
- *Methods in Enzymol.,* 1987, vol. 154, 329-350
- **ZOLLER ; SMITH.** Oligonucleotide-directed mutagenesis using M13-derived vectors: an efficient and general procedure for the production of point mutations in any DNA fragment. *Nucleic Acids Res.,* 1982, vol. 10, 6487-6500
- **ZOLLER ; SMITH.** Oligortucleatide-directed mutagenesis of DNA fragments cloned into M13 vectors. *Methods in Enzymol.,* 1983, vol. 100, 468-500
- **ZOLLER ; SMITH.** Oligonucleotide-directed mutagenesis: a simple method using two oligonucleotide primers and a single-stranded DNA template. *Methods in Enzymol.,* 1987, vol. 154, 329-350
- **TAYLOR et al.** The use of phosphorothioate-modifed DNA in restriction enzyme reactions to prepare nicked DNA. *Nucl. Acids Res.,* 1985, vol. 13, 8749-8764
- **TAYLOR et al.** The rapid generation of oligonucleotide-directed mutations at high frequency using phosphorothioate-modified DNA. *Nucl. Acids Res.,* 1985, vol. 13, 8765-8787
- **NAKAMAYE ; ECKSTEIN.** Inhibition of restriction endonuclease Nci I cleavage by phosphorothioate groups and its application to oligonucleotide-directed mutagenesis. *Nucl. Acids Res.,* 1986, vol. 14, 9679-9698
- **SAYERS et al.** Y-T Exonucleases in phosphorothioate-based oligonucleotide-directed mutagenesis. *Nucl. Acids Res.,* 1988, vol. 16, 791-802
- **SAYERS et al.** Strand specific cleavage of phosphorothioate-containing DNA by reaction with restriction endonucleases in the presence of ethidium bromide. *Nucl. Acids Res.,* 1988, vol. 16, 803-814
- **KRAMER et al.** The gapped duplex DNA approach to oligonucleotide-directed mutation construction. *Nucl. Acids Res.,* 1984, vol. 12, 9441-9456
- **KRAMER ; FRITZ.** Oligonucleotide-directed construction of mutations via gapped duplex DNA. *Methods in Enzymol.,* 1987, vol. 154, 350-367

- **KRAMER et al.** Improved enzymatic in vitro reactions in the gapped duplex DNA approach to oligonucleotide-directed construction of mutations. *Nucl. Acids Res.,* 1988, vol. 16, 7207
- **FRITZ et al.** Oligonucleotide-directed construction of mutations: a gapped duplex DNA procedure without enzymatic reactions in vitro. *Nucl. Acids Res.,* 1988, vol. 16, 6987-6999
- **KRAMER et al.** Point Mismatch Repair. *Cell,* 1984, vol. 38, 879-887
- **CARTER et al.** Improved oligonucleotide site-directed mutagenesis using M13 vectors. *Nucl. Acids Res.,* 1985, vol. 13, 4431-4443
- **CARTER.** Improved oligonucleotide-directed mutagenesis using M13 vectors. *Methods in Enzymol.,* 1987, vol. 154, 382-403
- **EGHTEDARZADEH ; HENIKOFF.** Use of oligonucleotides to generate large deletions. *Nucl. Acids Res.,* 1986, vol. 14, 5115
- **WELLS et al.** Importance of hydrogen-bond formation in stabilizing the transition state of subtilisin. *Phil. Trans. R. Soc. Lond. A,* 1986, vol. 317, 415-423
- **NAMBIAR et al.** Total synthesis and cloning of a gene coding for the ribonuclease S protein. *Science,* vol. 223, 1299-1301
- **SAKAMAR ; KHORANA.** Total synthesis and expression of a gene for the a-subunit of bovine rod outer segment guanine nucleotide-binding protein (transducin). *Nucl. Acids Res.,* vol. 14, 6361-6372
- **WELLS et al.** Cassette mutagenesis: an efficient merhod for generation of multiple mutations at defined sites. *Gene,* vol. 34, 315-323
- **GRUNDSTROM et al.** Oligonucleotide-directed mutagenesis by microscale 'shot-gun' gene synthesis. *Nucl. Acids Res.,* 1985, vol. 13, 3305-3316
- **MANDECKI.** Oligorzucleotide-directed double-strand break repair in plasmids of Escherichia coli: a method for site-specific mutagenesis. *Proc. Natl. Acad. Sci. USA,* 1986, vol. 83, 7177-7181
- **ARNOLD.** Protein engineeringfor unusual environments. *Current Opinion in Biotechnology,* 1993, vol. 4, 450-455
- *Methods in Enzymology,* vol. 154
- Guide to Molecular Cloning Techniques. **BERGER ; KIMMEL.** Methods in Enzymology. Academic Press, Inc, vol. 152
- **SAMBROOK et al.** Molecular Cloning - A Laboratory Manual. Cold Spring Harbor Laboratory, 1989, vol. 1
- Current Protocols in Molecular Biology. Current Protocols, 1999
- PCR Protocols A Guide to Methods and Applications. Academic Press Inc, 1990
- **ARNHEIM ; LEVINSON.** C&EN 36-47. *The Journal Of NIH Research,* 01 October 1990, vol. 3, 81-94
- **KWOH et al.** *Proc. Natl. Acad. Sci. USA,* 1989, vol. 86, 1173
- **GUATELLI et al.** *Proc. Natl. Acad. Sci. USA,* 1990, vol. 87, 1874
- **LOMELL et al.** *J Clin. Chem,* 1989, vol. 35, 1826
- **LANDEGREN et al.** *Science,* 1988, vol. 241, 1077-1080
- **VAN BRUNT.** *Biotechnology,* 1990, vol. 8, 291-294
- **WU ; WALLACE.** *Gene,* 1989, vol. 4, 560
- **BARRINGER et al.** *Gene,* 1990, vol. 89, 117
- **SOOKNANAN ; MALEK.** *Biotechnology,* 1995, vol. 13, 563-564
- **CHENG et al.** *Nature,* 1994, vol. 369, 684-685
- **FRESHNEY.** Culture of Animal Cells, a Manual of Basic Technique. Wiley- Liss, 1994
- **PAYNE et al.** Plant Cell and Tissue Culture in Liquid Systems. John Wiley & Sons, Inc, 1992
- Plant Cell, Tissue and Organ Culture. Fundamental Methods Springer Lab Manual. Springer-Verlag, 1995
- The Handbook of Microbiological Media. CRC Press, 1993
- **ALTSCHUL et al.** *J. Mol. Biol.,* 1990, vol. 215, 403-410
- **HENIKOFF ; HENIKOFF.** *Proc. Natl. Acad. Sci. USA,* 1989, vol. 89, 10915
- **FENG ; DOOLITTLE.** *J. Mol. Evol.,* 1987, vol. 35, 351-360
- **HIGGINS ; SHARP.** CADIOS. 1989, vol. 5, 151-153
- **STEMMER et al.** *Gene,* 1995, vol. 164, 49-53